# EUROPEAN PATENT APPLICATION

(11) **EP 4 786 495 A1**
(43) Date of publication of application: **05.08.2026**
(21) Application number: 24871005.5
(22) Date of filing: 27.09.2024
(51) Int. Cl.: C07K 16/28, A61K 39/395, A61K 39/00

(54) **BDCA2 MONOCLONAL ANTIBODY, FUSION PROTEIN COMPOSED OF SAME AND TACI, AND METHOD AND USE THEREFOR**

(30) Priority: 28.09.2023 WO PCT/CN2023/122885; 24.05.2024 WO PCT/CN2024/095279
(71) Applicant: Nanjing Leads Biolabs Co., Ltd., Jiangbei New Area Nanjing Jiangsu 210031 (CN)
(72) Inventor: WANG, Baohui, Nanjing, Jiangsu 210019 (CN); SUN, Jianming, Nanjing, Jiangsu 210019 (CN); LI, Fengxia, Nanjing, Jiangsu 210019 (CN); HUANG, Xiao, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/121877
(87) International publication number: WO 2025/067469

(57) **Abstract**

Provided are a monoclonal antibody specifically binding to BDCA2, and a bispecific fusion protein of BDCA2 and TACI constructed on the basis of the BDCA2 monoclonal antibody. Also provided are an immunoconjugate comprising the antibody, a bispecific molecule and a pharmaceutical composition, and diagnostic and therapeutic methods using the anti-BDCA2 antibody and BDCA2/TACI bispecific fusion protein.

## Description

### TECHNICAL FIELD

The present disclosure belongs to the field of molecular biology and biological products. Specifically, the present disclosure relates to a monoclonal antibody or a fragment thereof, a bispecific fusion protein comprising the monoclonal antibody or the fragment thereof, and a preparation method therefor and use thereof.

### BACKGROUND

Systemic lupus erythematosus (SLE) is a chronic, systemic autoimmune disease that affects multiple organs throughout the body, including the skin, joints, central nervous system, and kidney, through immune complexes, autoantibodies, cellular immunity, and inflammatory responses (Kaul A, Gordon C, Crow MK, Touma Z, Urowitz MB, van Vollenhoven R, Ruiz-Irastorza G, Hughes G. Systemic lupus erythematosus. Nat Rev Dis Primers. 2016 Jun 16; 2:16039). Plasmacytoid dendritic cells (pDCs) are a special type of dendritic cells (DCs), which can sense nucleic acid substances through TLR7 or TLR9 and produce a large amount of type I interferons (IFNs), causing the innate immune response of the body. (Saadeh D, Kurban M, Abbas O. Update on the role of plasmacytoid dendritic cells in inflammatory/autoimmune skin diseases. Exp Dermatol. 2016 Jun; 25(6):415-21). In addition to pDCs, B cells also play an important role in the progression of SLE. B cells can not only secrete autoantibodies, but also can serve as antigen presenting cells to influence the function of T cells, thereby secreting a series of precursor inflammatory factors (Chan, VF., Tsang, HL., Tam, RY. et al. B-cell-targeted therapies in systemic lupus erythematosus. Cell Mol Immunol 10, 133-142 (2013)). Therefore, targeting pDCs and/or B cells is an effective approach for treating SLE.

Blood DC antigen 2 (BDCA2) is a C-type lectin receptor specifically expressed on the surface of human pDCs. By binding to the pDCs membrane surface receptor FcεRIγ, BDCA2 activates the ITAM-regulated signaling pathway, leading to downstream protein phosphorylation, and ultimately inhibiting CpG from inducing pDCs to produce type I interferons (Gilliet M, Cao W, Liu YJ. Plasmacytoid dendritic cells: sensing nucleic acids in viral infection and autoimmune diseases. Nat Rev Immunol. 2008 Aug; 8(8):594-606). Therefore, targeting BDCA2 to further activate the downstream signaling pathway of BDCA2 and inhibit the production of type I interferons is an effective approach for treating SLE.

Transmembrane activator and calcium-modulating cyclophilin ligand interactor (TACI) is a B cell surface receptor. Its extracellular region comprises two cysteine-rich regions and it can bind to B cell activating factor of the tumor necrosis factor family (BAFF) and a proliferation-inducing ligand (APRIL). BAFF is a type II transmembrane protein. It belongs to the TNF ligand superfamily and is produced mainly by myeloid cells, such as monocytes, macrophages, neutrophils, and DCs. BAFF exists mainly in two forms, i.e., membrane-bound and soluble forms, and its receptors include TACI, B cell maturation antigen (BCMA), and BAFF receptor (BAFF-R). APRIL is also a type II transmembrane protein. It belongs to the TNF ligand superfamily and is mainly produced by myeloid cells. Different from BAFF, APRIL is only secreted. APRIL shares two receptors with BAFF, i.e., BCMA and TACI. After binding to BAFF and APRIL, TACI can induce the class transformation of IgG and IgA of B cells and promote the differentiation and survival of plasma cells (Zhang Y, Li J, Zhang YM, Zhang XM, Tao J. Effect of TACI signaling on humoral immunity and autoimmune diseases. J Immunol Res. 2015;2015:247426).

The present disclosure aims to construct a bifunctional molecule targeting both BDCA2 and BAFF/APRIL, so that in one aspect, pDCs are eliminated, a downstream signaling pathway of BDCA2 is activated, and the expression of type I interferon is inhibited, and in another aspect, the binding of BAFF/APRIL to its receptor is blocked, and the overactivation of B cells is inhibited. This may result in better therapeutic effects on SLE.

### SUMMARY

The present disclosure provides a novel anti-BDCA2 antibody or an antigen-binding fragment thereof, which has the advantages of high affinity, high specificity, etc., for human and cynomolgus monkey BDCA2. The present disclosure further provides a novel bispecific fusion protein targeting both BDCA2 and BAFF/APRIL. Also provided are a polynucleotide encoding the antibody or the bispecific fusion protein or a fragment thereof, a vector comprising the polynucleotide, a host cell comprising the polynucleotide or the vector, a method for treating a disease associated with BDCA2 and/or BAFF/APRIL using the antibody or the bispecific fusion protein, and use of the antibody or the bispecific fusion protein in the treatment, prevention, and/or diagnosis of a disease associated with BDCA2 and/or BAFF/APRIL in an individual. The anti-BDCA2 antibody, the BDCA2×BAFF/APRIL bispecific fusion protein, or an antigen/target-binding fragment thereof provided herein can be used as an independent therapy or in combination with other therapies and/or other agents to treat related diseases such as inflammatory disorders/diseases and/or autoimmune disorders/diseases.

In one aspect, the present disclosure provides an anti-BDCA2 antibody or an antigen-binding fragment thereof, wherein the anti-BDCA2 antibody or the antigen-binding fragment thereof comprises a heavy chain variable region and/or a light chain variable region, wherein the heavy chain variable region VH comprises 3 CDRs: HCDR1, HCDR2, and HCDR3, and the light chain variable region VL comprises 3 CDRs: LCDR1, LCDR2, and LCDR3, wherein
(i) the HCDR1, the HCDR2, and the HCDR3 are three complementarity determining regions HCDR1, HCDR2, and HCDR3 of the VH set forth in SEQ ID NO: 1; and the LCDR1, the LCDR2, and the LCDR3 are three complementarity determining regions LCDR1, LCDR2, and LCDR3 of the VL set forth in SEQ ID NO: 2;
(ii) the HCDR1, the HCDR2, and the HCDR3 are three complementarity determining regions HCDR1, HCDR2, and HCDR3 of the VH set forth in SEQ ID NO: 3; and the LCDR1, the LCDR2, and the LCDR3 are three complementarity determining regions LCDR1, LCDR2, and LCDR3 of the VL set forth in SEQ ID NO: 4; or
(iii) the HCDR1, the HCDR2, and the HCDR3 are three complementarity determining regions HCDR1, HCDR2, and HCDR3 of the VH set forth in SEQ ID NO: 13; and the LCDR1, the LCDR2, and the LCDR3 are three complementarity determining regions LCDR1, LCDR2, and LCDR3 of the VL set forth in SEQ ID NO: 15; or
(iv) the HCDR1, the HCDR2, and the HCDR3 are three complementarity determining regions HCDR1, HCDR2, and HCDR3 of the VH set forth in SEQ ID NO: 14; and the LCDR1, the LCDR2, and the LCDR3 are three complementarity determining regions LCDR1, LCDR2, and LCDR3 of the VL set forth in SEQ ID NO: 15; or
(v) relative to the sequence in any one of (i)-(iv), a sequence comprises at least one and no more than 5, 4, 3, 2, or 1 amino acid modification (preferably amino acid substitution, and preferably conservative substitution) in the three HCDRs or the three LCDRs; for example, the amino acid modification is a mutation enhancing the stability of the antibody;
preferably, the CDRs are determined by the Kabat numbering scheme.

In some embodiments, the anti-BDCA2 antibody or the antigen-binding fragment thereof of the present disclosure comprises:
(i) HCDR1 set forth in SEQ ID NO: 33, HCDR2 set forth in SEQ ID NO: 34, HCDR3 set forth in SEQ ID NO: 35, LCDR1 set forth in SEQ ID NO: 36, LCDR2 set forth in SEQ ID NO: 37, and LCDR3 set forth in SEQ ID NO: 38; or
(ii) HCDR1 set forth in SEQ ID NO: 39, HCDR2 set forth in SEQ ID NO: 40 or 49, HCDR3 set forth in SEQ ID NO: 41, LCDR1 set forth in SEQ ID NO: 42 or 48, LCDR2 set forth in SEQ ID NO: 43, and LCDR3 set forth in SEQ ID NO: 44; or
(iii) relative to the sequence in any one of (i) or (ii), a sequence comprising at least one and no more than 5, 4, 3, 2, or 1 amino acid modification (preferably amino acid substitution, and preferably conservative substitution) in the three HCDRs or the three LCDRs; for example, the amino acid modification is a mutation enhancing the stability of the antibody.

In some embodiments of the antibody or the antigen-binding fragment thereof of the present disclosure, the heavy chain variable region VH
(i) comprises or consists of an amino acid sequence having at least 90%, 91%, 92%, 93%, 94%, 95%, 96%, 97%, 98%, or 99% identity to the amino acid sequence of SEQ ID NO: 1, 3, 13, or 14; or
(ii) comprises or consists of the amino acid sequence of SEQ ID NO: 1, 3, 13, or 14.

In some embodiments of the antibody or the antigen-binding fragment thereof of the present disclosure, the light chain variable region VL
(i) comprises or consists of an amino acid sequence having at least 90%, 91%, 92%, 93%, 94%, 95%, 96%, 97%, 98%, or 99% identity to the amino acid sequence of SEQ ID NO: 2, 4, or 15; or
(ii) comprises or consists of the amino acid sequence of SEQ ID NO: 2, 4, or 15.

In some embodiments of the antibody or the antigen-binding fragment thereof of the present disclosure,
(i) the heavy chain variable region comprises or consists of the amino acid sequence set forth in SEQ ID NO: 1 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 2 or an amino acid sequence having at least 90%, 91%, 92%, 93%, 94%, 95%, 96%, 97%, 98%, or 99% identity to the amino acid sequence; or
(ii) the heavy chain variable region comprises or consists of the amino acid sequence set forth in SEQ ID NO: 3 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 4 or an amino acid sequence having at least 90%, 91%, 92%, 93%, 94%, 95%, 96%, 97%, 98%, or 99% identity to the amino acid sequence; or
(iii) the heavy chain variable region comprises or consists of the amino acid sequence set forth in SEQ ID NO: 13 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15 or an amino acid sequence having at least 90%, 91%, 92%, 93%, 94%, 95%, 96%, 97%, 98%, or 99% identity to the amino acid sequence; or
(iv) the heavy chain variable region comprises or consists of the amino acid sequence set forth in SEQ ID NO: 14 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15 or an amino acid sequence having at least 90%, 91%, 92%, 93%, 94%, 95%, 96%, 97%, 98%, or 99% identity to the amino acid sequence.

In some embodiments of the antibody or the antigen-binding fragment thereof of the present disclosure, the VH and the VL respectively comprise or consist of the amino acid sequences set forth in:
(i). SEQ ID NO: 1 and SEQ ID NO: 2;
(ii). SEQ ID NO: 3 and SEQ ID NO: 4;
(iii). SEQ ID NO: 13 and SEQ ID NO: 15;
(iv). SEQ ID NO: 14 and SEQ ID NO: 15.

In some embodiments, the antibody or the antigen-binding fragment thereof of the present disclosure further comprises Fc or a heavy chain constant region CH. For example, the Fc region is an Fc region of IgG1, IgG2, IgG3, or IgG4, preferably an Fc region of IgG1; or the antibody heavy chain constant region CH is a heavy chain constant region of IgG1, IgG2, IgG3, or IgG4, preferably a heavy chain constant region of IgG1. In some more specific embodiments, the heavy chain constant region CH (i) comprises or consists of an amino acid sequence having at least 85%, 90%, 91%, 92%, 93%, 94%, 95%, 96%, 97%, 98%, or 99% identity to the amino acid sequence of SEQ ID NO: 5; or (ii) comprises or consists of the amino acid sequence of SEQ ID NO: 5.

In some embodiments, the antibody or the antigen-binding fragment thereof of the present disclosure comprises an antibody heavy chain constant region featuring enhanced ADCC and/or prolonged half-life and/or increased affinity for FcRn, e.g., having an altered glycosylation type, e.g., being low-fucosylated or defucosylated or comprising increased proportion of bisecting GlcNac structure, and/or e.g., carrying an amino acid substitution combination of M252Y/S254T/T256E according to the EU numbering. In a specific embodiment, the heavy chain constant region comprises or consists of the amino acid sequence of SEQ ID NO: 54.

In some embodiments, the antibody or the antigen-binding fragment thereof of the present disclosure further comprises a light chain constant region. For example, the light chain constant region is a lambda or kappa light chain constant region. In some more specific embodiments, the light chain constant region (i) comprises or consists of an amino acid sequence having at least 85%, 90%, 91%, 92%, 93%, 94%, 95%, 96%, 97%, 98%, or 99% identity to the amino acid sequence of SEQ ID NO: 6; or (ii) comprises or consists of the amino acid sequence of SEQ ID NO: 6.

In some embodiments, the antibody or the antigen-binding fragment thereof of the present disclosure comprises a heavy chain, wherein the heavy chain comprises or consists of the amino acid sequence of SEQ ID NO: 9, 11, 16, or 17, or an amino acid sequence having at least 85%, 90%, 91%, 92%, 93%, 94%, 95%, 96%, 97%, 98%, or 99% identity to the amino acid sequence.

In some embodiments, the antibody or the antigen-binding fragment thereof of the present disclosure comprises a light chain, wherein the light chain comprises or consists of the amino acid sequence of SEQ ID NO: 10, 12, or 18, or an amino acid sequence having at least 85%, 90%, 91%, 92%, 93%, 94%, 95%, 96%, 97%, 98%, or 99% identity to the amino acid sequence.

In some embodiments, the antibody or the antigen-binding fragment thereof of the present disclosure comprises a heavy chain and a light chain, wherein
(i) the heavy chain comprises or consists of the amino acid sequence of SEQ ID NO: 9 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 10 or an amino acid sequence having at least 85%, 90%, 91%, 92%, 93%, 94%, 95%, 96%, 97%, 98%, or 99% identity to the amino acid sequence;
(ii) the heavy chain comprises or consists of the amino acid sequence of SEQ ID NO: 11 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 12 or an amino acid sequence having at least 85%, 90%, 91%, 92%, 93%, 94%, 95%, 96%, 97%, 98%, or 99% identity to the amino acid sequence;
(iii) the heavy chain comprises or consists of the amino acid sequence of SEQ ID NO: 16 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 18 or an amino acid sequence having at least 85%, 90%, 91%, 92%, 93%, 94%, 95%, 96%, 97%, 98%, or 99% identity to the amino acid sequence; or
(iv) the heavy chain comprises or consists of the amino acid sequence of SEQ ID NO: 17 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 18 or an amino acid sequence having at least 85%, 90%, 91%, 92%, 93%, 94%, 95%, 96%, 97%, 98%, or 99% identity to the amino acid sequence.

In some more specific embodiments, the heavy chain and the light chain respectively comprise or consist of amino acid sequences set forth in the following SEQ ID NOs:
(i). SEQ ID NO: 9 and SEQ ID NO: 10;
(ii). SEQ ID NO: 11 and SEQ ID NO: 12;
(iii).SEQ ID NO: 16 and SEQ ID NO: 18;
(iv). SEQ ID NO: 17 and SEQ ID NO: 18.

In some embodiments, the constant region of the antibody of the present disclosure is defucosylated or low-fucosylated.

In some embodiments, the antibody or the antigen-binding fragment thereof of the present disclosure is a humanized antibody or a chimeric antibody. In some embodiments, the antibody or the antigen-binding fragment thereof of the present disclosure is a monoclonal antibody.

In some embodiments, the antigen-binding fragment of the present disclosure is selected from: a Fab, a Fab', a Fab'-SH, an Fv, a single-chain antibody (e.g., scFv), an (Fab')₂, a single-domain antibody (e.g., VHH), a domain antibody (dAb), and a linear antibody.

In yet another aspect of the present disclosure, provided is a bispecific fusion protein specifically binding to human BDCA2 and human BAFF and APRIL, which comprises (i) a first target-binding region specifically binding to BDCA2, and (ii) a second target-binding region specifically binding to BAFF and/or APRIL.

In some embodiments of the bispecific fusion protein molecule of the present disclosure, the first target-binding region and the second target-binding region are linked via a linker, or are directly linked without a linker. In some more specific embodiments, the linker may be any universal flexible sequence known in the art or available in the future, typically a short flexible amino acid sequence. In some specific embodiments, the linker comprises 10-30 amino acids in length. In some embodiments, the linker is (GGGGS)n, (GSSSS)n, (GGGSG)n, (GGSGG)n, (GSGGG)n, (SGGGG)n, (GGTGS)n, (GTSPGG)n, (GNGGGS)n, (GGG)n, (DGGGS)n, (TGEKP)n, (GGRR)n, (EGKSSGSGSESKVD)n, (KESGSVSSEQLAQFRSLD)n, (GGRRGGGS)n, (LRQRDGERP)n, (LRQKDGGGSERP)n, (GSTSGSGKPGSGEGSTKG)n, and/or G₄S-GGSGG-G₄S-SGGGG, etc., wherein n is an integer equal to or greater than 1; for example, n is an integer of 1, 2, 3, 4, 5, 6, 7, 8, or 9. In a specific embodiment, the linker is (G₄S)n or (GS₄)n, wherein n is an integer equal to or greater than 1; for example, n is an integer of 1, 2, 3, 4, 5, 6, 7, 8, or 9. In some specific embodiments, the linker is selected from GS₄, G₄S, (GS₄)₂, (G₄S)₂, (GS₄)₃, (G₄S)₃, (GS₄)₄, and (G₄S)₄.

In some embodiments of the bispecific fusion protein molecule of the present disclosure, the first target-binding region is an anti-BDCA2 antibody or an antigen-binding fragment thereof or is derived from the anti-BDCA2 antibody or the antigen-binding fragment thereof; preferably, (i) the anti-BDCA2 antibody or the antigen-binding fragment thereof is the anti-BDCA2 antibody or the antigen-binding fragment thereof of the present disclosure; or (ii) the anti-BDCA2 antibody or the antigen-binding fragment thereof exhibits exactly the same binding specificity as the anti-BDCA2 antibody or the antigen-binding fragment thereof of the present disclosure, e.g., binding affinity, avidity, binding epitope, effector function, etc.

In some embodiments of the bispecific fusion protein molecule of the present disclosure, the second target-binding region comprises a BAFF/APRIL receptor or a functional fragment thereof specifically binding to BAFF and APRIL. Preferably, the BAFF/APRIL receptor is a TACI molecule; for example, the TACI molecule (i) comprises or consists of an amino acid sequence having at least 85%, 90%, 91%, 92%, 93%, 94%, 95%, 96%, 97%, 98%, or 99% identity to the amino acid sequence of SEQ ID NO: 20; (ii) comprises or consists of the amino acid sequence of SEQ ID NO: 20; or (iii) comprises or consists of the amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 20.

In some more specific embodiments, the functional fragment of the BAFF/APRIL receptor comprises a CRD2 derived from TACI, e.g., a CRD2 of wild-type TACI, or a variant CRD2 comprising a modification relative to the wild-type CRD2. In some more specific embodiments, the CRD2 of the TACI molecule (i) comprises or consists of an amino acid sequence having at least 85%, 90%, 91%, 92%, 93%, 94%, 95%, 96%, 97%, 98%, or 99% identity to the amino acid sequence of SEQ ID NO: 21; (ii) comprises or consists of the amino acid sequence of SEQ ID NO: 21; (iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 21.

In some embodiments, the fusion protein molecule of the present disclosure comprises:
(i) a chain A: a light chain of an anti-BDCA2 antibody;
(ii) a chain B: a heavy chain of an anti-BDCA2 antibody having TACI or a functional fragment thereof linked at the N-terminus or C-terminus, the linking being performed with or without a linker. In some more specific embodiments, the fusion protein molecule consists of 2 chains A and 2 chains B; preferably, the fusion protein molecule consists of 2 identical chains A and 2 identical chains B, i.e., the fusion protein molecule is a homodimer.

In some more specific embodiments, the chain B comprises, from N-terminus to C-terminus, the following elements in sequence:
(i) an anti-BDCA2 heavy chain, a linker, and TACI; or
(ii) TACI, a linker, and an anti-BDCA2 heavy chain; or
(iii) an anti-BDCA2 heavy chain, a linker, TACI, and a polyserine residue; or
(iv) TACI, a polyserine residue sequence, a linker, and an anti-BDCA2 heavy chain;

wherein the TACI is, e.g., a full-length TACI molecule or a fragment thereof;
the linker is preferably GS₄, G₄S, (GS₄)₂, (G₄S)₂, (GS₄)₃, (G₄S)₃, (GS₄)₄, or (G₄S)₄; the polyserine residue is SSSS, SSSSS, SSSSSS, SSSSSSS, SSSSSSSS, SSSSSSSSS, or SSSSSSSSSS,
and optionally, the linker may be absent.

In some more specific embodiments, the chain B comprises, from N-terminus to C-terminus, the following elements in sequence:
(i) an anti-BDCA2 heavy chain, a linker, and a CRD2 domain; or
(ii) a CRD2 domain, a linker, and an anti-BDCA2 heavy chain; or
(iii) an anti-BDCA2 heavy chain, a linker, a CRD2 domain, and a polyserine residue; or
(iv) a CRD2 domain, a polyserine residue sequence, a linker, and an anti-BDCA2 heavy chain;

the linker is preferably GS₄, G₄S, (GS₄)₂, (G₄S)₂, (GS₄)₃, (G₄S)₃, (GS₄)₄, or (G₄S)₄; the polyserine residue is SSSS, SSSSS, SSSSSS, SSSSSSS, SSSSSSSS, SSSSSSSSS, or SSSSSSSSSS,
and optionally, the linker may be absent.

In some more specific embodiments, the chain A comprises or consists of the amino acid sequence set forth in SEQ ID NO: 18 or an amino acid sequence having at least 85%, 90%, 91%, 92%, 93%, 94%, 95%, 96%, 97%, 98%, or 99% identity to the amino acid sequence. In some more specific embodiments, the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence.

In some more specific embodiments, the chain A comprises or consists of the amino acid sequence set forth in SEQ ID NO: 18 or an amino acid sequence having at least 85%, 90%, 91%, 92%, 93%, 94%, 95%, 96%, 97%, 98%, or 99% identity to the amino acid sequence; and the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence.

In some more specific embodiments, the chain A comprises or consists of the amino acid sequence set forth in SEQ ID NO: 18, and the chain B comprises or consists of the amino acid sequence set forth in any one of SEQ ID NOs: 22-32, 50-51, and 53.

In some embodiments, the fusion protein molecule of the present disclosure comprises (e.g., the chain B comprises) an antibody heavy chain constant region featuring enhanced ADCC and/or prolonged half-life and/or increased affinity for FcRn, e.g., having an altered glycosylation type, e.g., being low-fucosylated or defucosylated or comprising increased proportion of bisecting GlcNac structure, and/or e.g., carrying an amino acid substitution combination of M252Y/S254T/T256E according to the EU numbering. In a more specific embodiment, the heavy chain constant region comprises or consists of the amino acid sequence of SEQ ID NO: 54.

In some more specific embodiments, the fusion protein molecule of the present disclosure comprises a defucosylated or low-fucosylated antibody heavy chain constant region. In one embodiment, the anti-BDCA2 antibody or the fragment thereof or the BDCA2×BAFF/APRIL bispecific fusion protein or the target-binding fragment thereof of the present disclosure is capable of inhibiting the overactivation of plasmacytoid dendritic cells and/or eliminating plasmacytoid dendritic cells, inhibiting IFNα release, inhibiting B cell proliferation, and treating or ameliorating inflammatory disorders/diseases and/or autoimmune disorders/diseases, such as systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, ulcerative colitis, neuromyelitis optica, neuromyelitis optica spectrum disorder, primary Sjögren's syndrome, and myasthenia gravis.

In one embodiment, the anti-BDCA2 antibody or the fragment thereof or the BDCA2×BAFF/APRIL bispecific fusion protein or the target-binding fragment thereof of the present disclosure is capable of treating a tumor, e.g., a hematopoietic tumor.

In a third aspect, the present disclosure further provides a polynucleotide (nucleic acid) encoding the anti-BDCA2 antibody, the BDCA2×BAFF/APRIL bispecific fusion protein, or the antigen/target-binding fragment thereof of the present disclosure, and a vector, preferably an expression vector, comprising the polynucleotide.

In a fourth aspect, the present disclosure provides a host cell comprising the polynucleotide or the vector of the present disclosure. The host cell may be a prokaryotic cell and a eukaryotic cell commonly used in the art.

In a fifth aspect, the present disclosure provides a method for producing the anti-BDCA2 antibody or the BDCA2×BAFF/APRIL bispecific fusion protein of the present disclosure, which comprises: (i) culturing the host cell of the present disclosure under conditions suitable for expression of the antibody or the fusion protein of the present disclosure, and optionally, (ii) isolating the antibody or the fusion protein of the present disclosure.

In a sixth aspect, the present disclosure provides a kit, a pharmaceutical composition, a combination product, or an article of manufacture comprising the anti-BDCA2 antibody or the BDCA2×BAFF/APRIL bispecific fusion protein of the present disclosure. In one embodiment, the pharmaceutical composition, the combination product, or the article of manufacture provided herein further comprises an additional therapeutic agent and optionally a pharmaceutical supplementary material; preferably, the additional therapeutic agent is selected from an anti-inflammatory agent, a cytotoxic agent, an immunosuppressant, an additional antibody, a small molecule drug, and the like. In a seventh aspect, the present disclosure provides use of the anti-BDCA2 antibody or the BDCA2×BAFF/APRIL bispecific fusion protein, the kit, the pharmaceutical composition, the combination product, or the article of manufacture of the present disclosure in the treatment, prevention, and/or diagnosis of a disease associated with BDCA2 and/or a disease associated with BAFF/APRIL. In one embodiment, the disease associated with BDCA2 is, for example, systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, and ulcerative colitis. In one embodiment, the disease associated with BAFF/APRIL is, for example, systemic lupus erythematosus, neuromyelitis optica, neuromyelitis optica spectrum disorder, rheumatoid arthritis, IgA nephropathy, autoimmune glomerulonephritis, primary Sjögren's syndrome, multiple sclerosis (e.g., relapsing-remitting type), and myasthenia gravis (e.g., generalized type).

In one embodiment, the present disclosure provides the anti-BDCA2 antibody or the BDCA2×BAFF/APRIL bispecific fusion protein, or the corresponding polynucleotide, vector, host cell, kit, pharmaceutical composition, combination product, or article of manufacture for use in one or more of the following *in vivo* or *in vitro*:
- binding to human BDCA2 and human BAFF/APRIL; or
- activating human BDCA2 signaling pathway; or
- blocking BAFF/APRIL signaling pathway; or
- inhibiting expression of type I interferon; or
- exhibiting effector functions against BDCA2, such as ADCC, ADCP, and CDC;
- exhibiting a neutralizing effect against BAFF/APRIL molecules;
- killing cells expressing human BDCA2 protein; or
- eliminating pDC cells *in vivo*
- inhibiting local or systemic inflammatory responses and/or immune responses.

In one embodiment, the present disclosure provides use of the anti-BDCA2 antibody or the BDCA2×BAFF/APRIL bispecific fusion protein, or the corresponding polynucleotide, vector, host cell, kit, pharmaceutical composition, combination product, or article of manufacture in the manufacture of a medicament for the treatment, prevention, and/or diagnosis of systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, ulcerative colitis, neuromyelitis optica, neuromyelitis optica spectrum disorder, primary Sjögren's syndrome, and/or myasthenia gravis.

In an eighth aspect, the present disclosure provides a method for inhibiting the overactivation of plasmacytoid dendritic cells and/or eliminating plasmacytoid dendritic cells, a method for inhibiting IFNα release, a method for inhibiting B cell proliferation, or a method for treating or ameliorating an inflammatory disorder/disease and/or an autoimmune disorder/disease, which comprises administering to a patient in need thereof a therapeutically effective amount of the anti-BDCA2 antibody or the BDCA2×BAFF/APRIL bispecific fusion protein of the present disclosure, or the pharmaceutical composition, the combination product, or the article of manufacture of the present disclosure.

### BRIEF DESCRIPTION OF THE DRAWINGS

FIG. 1 shows the structure of a bispecific fusion protein.
FIG. 2 shows the binding of various anti-BDCA2 chimeric antibodies to a cell line expressing human BDCA2 protein.
FIG. 3 shows the killing ability of various anti-BDCA2 chimeric antibodies on a cell line expressing human BDCA2 protein via ADCC.
FIG. 4 shows the inhibitory effect of various anti-BDCA2 chimeric antibodies on TLR9 agonist-induced stimulation of PBMCs, as reflected by the change in the amount of IFN-α produced.
FIG. 5 shows the killing of cells expressing human BDCA2 protein by anti-BDCA2 humanized antibodies via ADCC.
FIG. 6 shows the inhibitory effect of anti-BDCA2 humanized antibodies on TLR9 agonist-induced stimulation of PBMCs, as reflected by the change in the amount of IFN-α produced.
FIG. 7 shows the effect of anti-BDCA2 humanized antibodies in eliminating pDCs in a huHSC-NCG mouse model. The number and percentage of pDCs in the peripheral blood and the number and percentage of pDCs in the spleen of the treated mice are shown in panels a, b, c, and d, respectively.
FIG. 8 shows the results of a sandwich ELSA assay for bispecific fusion proteins.
FIG. 9 shows the inhibition of B cell proliferation by bispecific fusion proteins and bispecific fusion proteins with different linker peptides.
FIG. 10 shows the killing of cells expressing human BDCA2 protein by bispecific fusion proteins and bispecific fusion proteins with different linker peptides.
FIG. 11 shows the inhibitory effect of bispecific fusion proteins and bispecific fusion proteins with different linker peptides on TLR9 agonist-induced stimulation of PBMCs, as reflected by the change in the amount of IFN-α produced.
FIG. 12 shows the effect of bispecific fusion proteins in eliminating pDCs in a huHSC-NCG mouse model. The number and percentage of pDCs in the peripheral blood and the number and percentage of pDCs in the spleen of the treated mice are shown in panels 12a, 12b, 12c, and 12d, respectively.
FIG. 13 shows the effect of bispecific fusion proteins in a KLH-induced delayed-type hypersensitivity (DTH) BALB/c mouse model.
FIG. 14 shows the inhibition of B cell proliferation by the bispecific fusion protein having amino acid substitutions M252Y/S254T/T256E (YTE mutations) in the Fc region.
FIG. 15 shows the killing of cells expressing human BDCA2 protein by the bispecific fusion protein having YTE mutations in the Fc region.
FIG. 16 shows the inhibitory effect of the bispecific fusion protein having YTE mutations in the Fc region on TLR9 agonist-induced stimulation of PBMCs, as reflected by the change in the amount of IFN-α produced.
FIG. 17 shows the effect of the bispecific fusion protein having YTE mutations in the Fc region in eliminating pDCs in a huHSC-NCG mouse model. The number and percentage of pDCs in the peripheral blood and the number and percentage of pDCs in the spleen of the treated mice are shown in panels 17a, 17b, 17c, and 17d, respectively.
FIG. 18 shows the effect of the bispecific fusion protein having YTE mutations in the Fc region in a KLH-induced delayed-type hypersensitivity (DTH) B-hFcRn mouse model and the measurement of antibody concentrations in mouse serum.
FIG. 19 shows the efficacy of the bispecific fusion protein in an MOG₃₅₋₅₅-induced C57BL/6J experimental allergic encephalomyelitis (EAE) model.
FIG. 20 shows the PK assay of the bispecific fusion protein having YTE mutations in the Fc region in B-hFcRn mice.
FIG. 21 shows the PK assay of the bispecific fusion protein having YTE mutations in the Fc region in cynomolgus monkeys.

### DETAILED DESCRIPTION

### I. Definitions

Unless otherwise defined, all technical and scientific terms used herein have the same meaning as commonly understood by those of ordinary skill in the art to which the present disclosure belongs. All publications, patent applications, patents, and other references mentioned herein are incorporated by reference in their entireties. In addition, the materials, methods, and examples described herein are illustrative only and are not intended to be limiting. Other features, objectives, and advantages of the present disclosure will be apparent from this description and drawings and from the appended claims.

In order to illustrate this description, the following definitions will be used, and wherever appropriate, terms used in the singular form may also include the plural form, and vice versa. It should be understood that the terms used herein are for the purpose of describing specific embodiments only, and are not intended to be limiting.

The term "about" used in combination with a numerical value is intended to encompass the numerical values in a range from a lower limit less than the specified numerical value by 10% to an upper limit greater than the specified numerical value by 10%.

As used herein, the term "and/or" refers to any one of the options or any two or more of the options. As used herein, the term "comprise" or "include" is intended to mean that the elements, integers, or steps are included, but not to the exclusion of any other elements, integers, or steps. Herein, the term "comprise" or "include", unless otherwise specified, also encompasses the situation where the entirety consists of the described elements, integers, or steps. For example, when referring to an antibody variable region "comprising" a specific sequence, it is also intended to encompass an antibody variable region consisting of the specific sequence.

The term "BDCA2" used herein refers to a type II C-type lectin BDCA2 specifically expressed on pDCs. It consists of a single extracellular carbohydrate recognition domain at the C-terminus, a transmembrane region from an asparagine residue at position 45 to an isoleucine residue at position 213, and a short cytoplasmic tail at the N-terminus (without the signal motif). BDCA2 sends intracellular signals through a related transmembrane adapter FcεRIγ. Antibody-mediated binding of BDCA2 results in the recruitment of spleen tyrosine kinase (SYK) to the phosphorylated immunoreceptor tyrosine-based activation motif (ITAM) of FcεRIγ. The activation of Syk leads to the activation of the B cell linker (BLNK), Bruton's tyrosine kinase (BTK), and phospholipase Cγ2 (PLCγ2), resulting in the mobilization of Ca²⁺. The activation of BDCA2 will enable it to bind to a cell surface FcεRIγ receptor for internalization, thereby activating downstream signals through the ITAM of the FcεRIγ receptor, inhibiting the expression of type I interferon induced by TLR7 and TLR9, and simultaneously down-regulating the expression of the FcεRIγ receptor from the surface of pDCs. The BDCA2 antibody of the present disclosure binds to BDCA2 and causes its internalization, thereby producing therapeutic effects on a range of diseases, such as systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, and ulcerative colitis; in addition, the BDCA2 antibody of the present disclosure also exhibits therapeutic effects on some hematopoietic tumors, particularly BDCA2-positive blood system tumors, such as blastic plasmacytoid dendritic cell neoplasm. As used herein, the term "BDCA2-mediated disease" refers to a disease in which BDCA2 is involved in the development, progression, delay of disease, or the like, e.g., involving activation (e.g., abnormal activation or overactivation) of BDCA2, and the involvement of BDCA2 directly or indirectly produces at least one of the following effects: the onset/development of the disease; increased/deepened pathological changes of the disease; an enlarged site/extent of disease impact; aggravated body damage caused by the disease; an increased pain from the disease; disease insensitivity/resistance to treatment measures; and a reduced tendency to self-heal and a poorer prognosis of the disease. In some embodiments of the present disclosure, the BDCA2-mediated disease is an inflammatory disease, e.g., the inflammatory disease described above; in some embodiments of the present disclosure, the BDCA2-mediated disease is a tumor, e.g., the tumor described above. The term "BDCA2" includes variants, subtypes, and species homologs of human BDCA2 or BDCA2 of other species, and analogs having at least one common epitope with BDCA2, unless otherwise stated. The term includes unprocessed full-length BDCA2 and BDCA2 in any form resulting from intracellular processing. The term encompasses "full-length" unprocessed BDCA2 and BDCA2 in any form resulting from intracellular processing or any fragment thereof, such as a splice variant or an allelic variant. In one embodiment, BDCA2 refers to full-length BDCA2 from humans or cynomolgus monkeys, or fragments thereof (such as mature fragments thereof lacking a signal peptide).

"TACI", short for "transmembrane activator and CAML-interactor", is one of the common receptors for BAFF (B cell activating factor, or BLyS, TNSF13B, THANK, TALL-1, and zTNF4) and APRIL (proliferation-inducing ligand) (other receptors include BCMA, BAFF-R, and the like). Their ligand-receptor interactions can activate downstream pathways, promote the development and proliferation of B lymphocytes, and increase the expression level of various immunoglobulins in serum. Mature T lymphocytes also express TACI on the surface, and thus cellular immunity is also regulated by this mechanism. Overexpression of BAFF and APRIL is one of the causes of many autoimmune diseases, and inhibiting BAFF and APRIL is an effective approach for treating autoimmune diseases, such as systemic lupus erythematosus, neuromyelitis optica, neuromyelitis optica spectrum disorder, rheumatoid arthritis, IgA nephropathy, primary Sjögren's syndrome, relapsing-remitting multiple sclerosis, and generalized myasthenia gravis. Meanwhile, since BAFF and APRIL have promoting effects on certain hematopoietic tumors, inhibiting BAFF and APRIL can also be used for the treatment of such hematopoietic tumors. The term "disease associated with BAFF/APRIL" refers to any disorder that is caused by or exacerbated by abnormal expression (e.g., increased expression) or abnormal activity of BAFF and/or APRIL or otherwise associated with BAFF/APRIL. In one embodiment, the disease associated with BAFF/APRIL is an inflammatory disease, e.g., the inflammatory disease described above; in some embodiments of the present disclosure, the disease associated with BAFF/APRIL is a tumor, e.g., the tumor described above.

Due to the high affinity of TACI for BAFF and APRIL, soluble TACI (e.g., the extracellular portion of TACI) can be used as a blocking molecule to bind to BAFF or APRIL and thereby block the binding of BAFF or APRIL to cell membrane receptors. The extracellular region of TACI comprises two cysteine-rich domains (CRDs), but further studies have found that the N-terminal CRD is not involved in ligand binding. Therefore, the second CRD, CRD2 (also referred to herein as D2), is considered to be a critical part determining the specific binding of TACI to BAFF/APRIL. In the context of the present disclosure, unless otherwise specified, the term "TACI" may encompass any functional variant, truncation or variant thereof, and fragment or variant thereof of a natural TACI molecule, for example, an extracellular region, a CRD1-2 domain, a CRD2 domain, an N-terminal truncation of the CRD2 domain, a C-terminal truncation of the CRD2 domain, a fragment consisting of amino acid residues at positions 30-118, and the like, of a natural TACI molecule or a functional variant thereof.

The term "immune response" refers to the action of, for example, lymphocytes, antigen-presenting cells, phagocytes, granulocytes, and soluble macromolecules produced by the above cells or liver (including antibodies, cytokines, and complements) that result in selective damage to, destruction of, or elimination from the human body of invading pathogens, cells or tissues infected with pathogens, cancerous cells, or, in the cases of autoimmunity or pathological inflammation, normal human cells or tissues.

The term "human plasmacytoid dendritic cells (pDCs)" is a specialized population of bone marrow-derived cells that secrete type I interferons (IFNs) in response to toll-like receptor (TLR) ligands. pDCs are a key link of cellular immunity, involving the induction and initiation of immune responses and antigen tolerance. pDCs are different from ordinary DCs in that, for example, they acquire antigens by receptor-mediated endocytosis, etc. The type I interferons produced by pDCs activated by pattern recognition receptors can also present antigens, thereby associating innate and acquired immune responses. At the same time, the overactivation of pDCs may also negatively affect the immune response process, for example, possibly leading to autoimmune diseases.

The term "signal transduction pathway" or "signal transduction activity" refers to a biochemical causal relationship generally initiated by an interaction between proteins (such as binding of a growth factor to a receptor) and resulting in the transmission of a signal from one portion of a cell to another portion of the cell. In general, the transmission includes specific phosphorylation of one or more tyrosine, serine, or threonine residues on one or more proteins in a series of reactions causing signal transduction. The penultimate process typically involves a nuclear event, resulting in a change in gene expression.

The terms "activity" and "bioactivity", and the terms "biological property" and "biological characteristic", are used interchangeably herein and include, but are not limited to, epitope/antigen affinity and specificity, the ability to neutralize or antagonize BDCA2 activity *in vivo* or *in vitro*, IC₅₀, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain a high expression level of the protein in mammalian cells. The aforementioned properties or characteristics are observed, determined, or assessed using techniques well known in the art, including but not limited to ELISA, FACS, or BIACORE plasma resonance analysis, unlimited *in vitro* or *in vivo* neutralization assays, receptor binding, cytokine or growth factor production and/or secretion, signal transduction, and immunohistochemistry of tissue sections of different sources (including humans, primates, or any other source).

As used herein, the term "isolated protein" or "isolated polypeptide" is a protein or polypeptide that, by virtue of its origin or source of derivation, is isolated from naturally associated components that accompany it in its native state, is substantially free of other proteins from the same species, is expressed by a cell from a different species, or does not occur in nature. A polypeptide that is chemically synthesized or synthesized in a cell system different from the cell from which it naturally originates may be "isolated" from its naturally associated components. A protein may also be rendered substantially free of naturally associated components by isolation, using protein purification techniques well known in the art.

The term "specific binding" or "specifically binds," when involving the interaction of an antibody, a binding protein, or a peptide with a second chemical substance, means that the interaction is dependent upon the presence of a particular structure (e.g., an antigenic determinant or epitope) on the second chemical substance; for example, an antibody recognizes and binds to a specific protein structure, but not to proteins in general. If an antibody is specific for an epitope "A", the presence of a molecule containing the epitope A (or free, unlabeled A) will reduce the amount of labeled A bound to the antibody in a reaction containing labeled "A" and the antibody. Consistent with the present disclosure, a specifically binding protein binds to a corresponding antigen with a K_{D} of 10 nM or less, e.g., 1 nM or less. The term "K_{D}" refers to the equilibrium dissociation constant (the inverse of the equilibrium association constant) and is used herein according to definitions provided in the art. The K_{D} value of the binding of an antibody or a binding protein to a corresponding antigen may be determined by well-known methods, including but not limited to, fluorescence titration, competitive ELISA, calorimetric methods (such as isothermal titration calorimetry (ITC)), flow cytometric titration analysis (FACS titration), bio-layer interferometry (BLI), and surface plasmon resonance (BIAcore). The terms "antigen-binding site" and "antigen-binding specificity" as used herein are used interchangeably and refer to a region where an antibody actually binds to an antigen.

The term "antibody" refers to any form of antibody having a desired biological activity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "isolated antibody" refers to a binding compound in a purified state, and in this context, it means that the molecule is substantially free of other biomolecules such as nucleic acids, proteins, lipids, sugars, or other substances such as cellular debris and growth medium. The term "isolate(d)" does not mean the complete absence of such substances or the absence of water, buffers, or salts, unless they are present in amounts that will significantly interfere with the experimental or therapeutic use of the binding compounds described herein.

The term "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "full-length antibody" refers to an immunoglobulin molecule comprising at least four peptide chains when present naturally, including two heavy (H) chains and two light (L) chains linked to each other by disulfide bonds. Each heavy chain consists of a heavy chain variable region (abbreviated herein as VH) and a heavy chain constant region (abbreviated herein as CH). The heavy chain constant region consists of 3 domains, CH1, CH2, and CH3. Each light chain consists of a light chain variable region (abbreviated herein as VL) and a light chain constant region. The light chain constant region consists of one domain, CL. The VH and VL regions can be further divided into complementarity determining regions (CDRs) with high variability and more conservative regions called framework regions (FRs) that are interspersed with the CDRs. Each VH or VL region consists of 3 CDRs and 4 FRs arranged in the following order from the amino terminus to the carboxyl terminus: FR1, CDR1, FR2, CDR2, FR3, CDR3, FR4. The variable regions of the heavy and light chains comprise binding domains that interact with antigens. The constant regions of an antibody can mediate the binding of immunoglobulins to host tissues or factors, including various cells of the immune system (e.g., effector cells) and the first component (Clq) of classical complement system. The term "antigen-binding fragment" of an antibody ("parent antibody") includes a fragment or a derivative of an antibody, typically including at least one fragment of an antigen-binding region or variable region (e.g., one or more CDRs) of a parent antibody, which retains at least some of the binding specificity of the parent antibody. Examples of the binding fragment of an antibody include, but are not limited to, Fab, Fab', F(ab')₂, and Fv fragments well-known in the art; a diabody; a linear antibody; a single-chain antibody molecule, e.g., sc-Fv; and a nanobody and a multispecific antibody formed by antibody fragments. In some preferred embodiments of the present disclosure, the antigen-binding fragment of the present disclosure is selected from Fab, Fab', F(ab')₂, and Fv fragments; a diabody; a linear antibody; a single-chain antibody molecule, such as sc-Fv; and a nanobody and a multispecific antibody formed by antibody fragments. A binding fragment or a derivative generally retains at least 10% of its antigen binding activity when the antigen binding activity is expressed on a molar concentration basis. Preferably, the binding fragment or the derivative retains at least 20%, 50%, 70%, 80%, 90%, 95%, or 100% or more of the antigen binding affinity of the parent antibody. It is also contemplated that the antigen-binding fragment of the antibody may comprise conservative or non-conservative amino acid substitutions that do not significantly alter its bioactivity (referred to as "conservative variants" or "function-conserved variants" of the antibody). The term "binding compound" refers to both an antibody and a binding fragment thereof.

The term "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

The term "single-domain antibody" generally refers to an antibody in which a single variable domain(e.g., a heavy chain variable domain (VH) or a light chain variable domain(VL), a heavy chain variable domain derived from a Camelidae heavy-chain antibody, and a VH-like single domain derived from fish IgNAR (v-NAR)) can impart antigen binding. That is, the single variable domain requires no interaction with another variable domain to recognize a target antigen. Examples of single-domain antibodies include single-domain antibodies derived from Camelidae (Llama and camel) and cartilaginous fishes (e.g., nurse sharks) (WO 2005/035572). The single-domain antibody derived from Camelidae is also referred to as VHH or "nanobody" in the present application. The term "VHH" or "VHH domain" is used herein to refer to a heavy chain variable domain derived from a heavy-chain antibody lacking a light chain, i.e., it consists of only one heavy chain variable region and comprises only one chain, FR4-CDR3-FR3-CDR2-FR2-CDR1-FR1, from C-terminus to N-terminus, also known as a single variable domain (sVD). Unlike the conventional VH of a four-chain immunoglobulin, the VHH domain does not need to pair with a light chain variable domain to form an antigen-binding site. A single-domain antibody is the minimal unit known to bind to a target antigen. In some cases, for therapeutic applications of VHH, it is desirable to reduce its immunogenicity. Preferably, in some embodiments, the present disclosure provides an antibody or a bispecific fusion protein comprising a humanized VHH domain.

The term "Fc region" is used to define the C-terminal region of an immunoglobulin heavy chain, which may be produced by digesting an intact antibody with papain. The Fc region may be a natural sequence Fc region or a variant Fc region. The Fc region of an immunoglobulin generally comprises two constant domains, i.e., a CH2 domain and a CH3 domain, and optionally comprises a CH4 domain, for example in the case of the Fc regions of IgM and IgE antibodies. The Fc region of IgG, IgA, and IgD antibodies comprises a hinge region, a CH2 domain, and a CH3 domain. In contrast, the Fc region of IgM and IgE antibodies lacks the hinge region, but comprises a CH2 domain, a CH3 domain, and a CH4 domain. In certain embodiments, a human IgG heavy chain Fc region extends from Cys226 or Pro230 to the carbonyl terminus of the heavy chain. However, the C-terminal lysine (Lys447) of the Fc region may or may not be present. Variant Fc regions having substitutions of amino acid residues in the Fc portion to alter antibody effector functions are known in the art (see, e.g., Winter et al., U.S. Pat. Nos. 5,648,260 and 5,624,821). The Fc portion of an antibody can mediate one or more effector functions, such as cytokine induction, ADCC, phagocytosis, complement-dependent cytotoxicity (CDC), and half-life/clearance rate of antibodies and antigen-antibody complexes. In some cases, these effector functions are desirable for therapeutic antibodies, but in other cases may be unnecessary or even detrimental, depending on the therapeutic purpose. Certain human IgG isotypes, particularly IgG1 and IgG3, mediate ADCC and CDC via binding to FcγR and complement C1q, respectively. In another embodiment, at least one amino acid residue is substituted in the constant region of the antibody, e.g., the Fc region of the antibody, thereby altering the effector function of the antibody. The dimerization of two identical heavy chains of an immunoglobulin is mediated by dimerization of CH3 domains and stabilized by a disulfide bond in the hinge region connecting the CH1 constant domain to the Fc constant domains (e.g., CH2 and CH3). The anti-inflammatory activity of IgG relies on the sialylation of N-linked glycan of the IgG Fc fragment. The precise glycan requirements for anti-inflammatory activity have been determined, such that an appropriate IgG1 Fc fragment can be created, thereby generating a fully recombinant, sialylated IgG1 Fc with greatly enhanced potency (Anthony et al., Science, 320:373-376 (2008)). Unless otherwise stated, the numbering of amino acid residues in the Fc region or constant region is based on the EU numbering system, which is also known as the EU index.

The term "variable region" or "variable domain" refers to a domain of a heavy chain or light chain of an antibody involved in the binding of the antibody to an antigen. Variable domains of heavy and light chains of natural antibodies typically have similar structures, wherein each domain comprises four conservative Framework regions (FRs) and three complementarity determining regions (CDRs) (see, e.g., Kindt et al., Kuby Immunology, 6th ed., W. H. Freeman and Co., page 91 (2007)). A single VH or VL domain is sufficient to confer antigen binding specificity.

The "complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). The CDRs are primarily responsible for binding to antigenic epitopes. The CDRs of the heavy chain are generally referred to as CDR1, CDR2, and CDR3, and are numbered sequentially from the N-terminus. In a given amino acid sequence of a heavy chain variable region, the exact amino acid sequence boundary of each CDR may be determined using any one or a combination of many well-known antibody CDR assignment systems including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (http://imgt.cines.fr/), and North CDR definition based on the affinity propagation clustering using a large number of crystal structures.

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32...34 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the boundaries of the CDRs of the antibodies of the present disclosure can be determined by those skilled in the art according to any scheme (e.g., different assignment systems or combinations) in the art, and the term "CDR" or "CDR sequence" encompasses CDR sequences determined in any one of the ways described above.

CDRs may also be determined based on having the same Kabat numbering positions as a reference CDR sequence (e.g., any one of the exemplary CDRs of the present disclosure). In one embodiment, the positions of CDRs of the antibody of the present disclosure are determined according to the Kabat numbering scheme.

It should be noted that boundaries of CDRs of variable regions of an antibody based on different assignment systems may differ. That is, CDR sequences of the variable regions of the same antibody defined by different assignment schemes are different. Thus, when it comes to defining an antibody with a particular CDR sequence defined in the present disclosure, the scope of the antibody also encompasses antibodies whose variable region sequences comprise the particular CDR sequence but whose claimed CDR boundaries differ from the particular CDR boundaries defined in the present disclosure due to the application of different schemes (e.g., different assignment systems or combinations).

Unless otherwise stated, residue positions of antibody variable regions and CDRs (including heavy chain variable region residues) are numbered according to the Kabat numbering system in the present disclosure.

Antibodies with different specificities (i.e., different binding sites for different antigens) have different CDRs. However, although CDRs vary from antibody to antibody, only a limited number of amino acid positions within the CDRs are directly involved in antigen binding. The smallest overlapping region can be determined using at least two of the Kabat, Chothia, AbM, Contact, and North methods, thereby providing a "smallest binding unit" for antigen binding. The smallest binding unit may be a sub-portion of the CDR. As will be clear to those skilled in the art, the residues in the remainder of the CDR sequences may be determined by the antibody structure and protein folding. Thus, variants of any of the CDRs presented herein are also contemplated by the present disclosure. For example, in a variant of one CDR, the amino acid residues of the smallest binding unit may remain unchanged, while the remaining CDR residues defined according to Kabat or Chothia may be substituted with conservative amino acid residues.

The term "multispecific" refers to the binding properties of an antibody/fusion protein/binding molecule having at least two antigen-binding sites, each of which binds to a different epitope/binding site of the same target molecule/antigen or a different epitope/binding site of a different target molecule/antigen. In one embodiment, the present disclosure provides a multispecific antibody/fusion protein/binding molecule having binding specificities for a first target molecule/antigen and a second target molecule/antigen, also referred to as a "bispecific antibody/fusion protein/binding molecule". In some embodiments, the bispecific antibody/fusion protein/binding molecule of the present disclosure comprises four binding sites and is typically a tetravalent binding protein. In some embodiments, the bispecific antibody/fusion protein/binding molecule of the present disclosure comprises six binding sites and is typically a hexavalent binding protein.

The term "agonize" refers to increasing certain parameters (e.g., activity) of a given molecule (e.g., a co-stimulatory molecule). For example, this term includes substances that increase the activity of a given molecule by at least 5%, 10%, 20%, 30%, 40%, or more. Thus, agonism does not have to be 100%.

Herein, the antibody having "activating/activation activity" or "internalization-promoting activity" for BDCA2 refers to the function where the antibody binds to BDCA2 to promote its binding to FcεRIγ receptors for internalization and thereby facilitate the transmission of its downstream related signals. Assays that can be used to determine such activating activity may be conventional assays known in the art.

As used herein, the antibody having "neutralizing activity" or "blocking activity" for BAFF/APRIL refers to the function of the antibody to block the signaling pathway induced by the binding of BAFF/APRIL to its natural receptor. Assays that can be used to determine the activity may be conventional assays known in the art, such as FACS assay, or reporter gene-based signaling pathway blocking assay.

The sequence identity between sequences is calculated as follows.

To determine the percent identity of two amino acid sequences or two nucleic acid sequences, the sequences are aligned for optimal comparison purposes (e.g., for optimal alignment, gaps may be introduced in one or both of the first and second amino acid sequences or nucleic acid sequences, or non-homologous sequences may be discarded for comparison purposes). In one preferred embodiment, the length of a reference sequence aligned for comparison purposes is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percent identity between the sequences. In one preferred embodiment, the percent identity between two amino acid sequences is determined with the Needlema and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In yet another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

Additionally or alternatively, the nucleic acid sequences and protein sequences described herein may be further used as "query sequences" to perform searches against public databases to, e.g., identify other family member sequences or related sequences.

As used herein, the term "kₒₙ" (also referred to as "Kon" or "kon") refers to the association rate constant for the binding of a binding protein (e.g., an antibody) to an antigen to form an association complex (e.g., an antibody/antigen complex) as is known in the art. "kₒₙ" is also known as the terms "association rate constant" or "ka", as used interchangeably herein. This value represents the binding rate of an antibody to its target antigen or the rate of complex formation between an antibody and an antigen, as shown in the following formula:

antibody ("Ab") + antigen ("Ag") →Ab-Ag.

As used herein, the term "k_{off}" (also referred to as "Koff" or "koff") refers to the rate constant for the dissociation of a binding protein (e.g., an antibody) from an association complex (e.g., an antibody/antigen complex) or "dissociation rate constant", as is known in the art. This value represents the dissociation rate of an antibody from its target antigen, or the rate of separation of the Ab-Ag complex over time into free antibody and antigen, as shown in the following formula:

Ab + Ag←Ab-Ag.

As used herein, the term "K_{D}" (also referred to as "K_{d}") is intended to mean the "equilibrium dissociation constant" and refers to a value obtained in a titration measurement at equilibrium or a value obtained by dividing the dissociation rate constant (k_{off}) by the association rate constant (kₒₙ). The association rate constant (kₒₙ), the dissociation rate constant (k_{off}), and equilibrium dissociation constant (K_{D}) are used to represent the binding affinity of an antibody for an antigen. Methods of determining the association and dissociation rate constants are well known in the art. Fluorescence-based techniques may be used to offer high sensitivity and the ability to examine samples in physiological buffers at equilibrium. Other experimental approaches and instruments such as a BIAcore^{®} (biomolecular interaction analysis) assay may be used (e.g., instrument available from BIAcore International AB, a GE Healthcare company, Uppsala, Sweden). Biolayer interferometry (BLI) using, e.g., the Octet^{®} RED96 system (PallFortéBio LLC), is another affinity assay technique. Additionally, a KinExA^{®} (kinetic exclusion assay) assay, available from Sapidyne Instruments (Boise, Idaho), may also be used.

The term "isolated nucleic acid" refers to a polynucleotide (e.g., of genomic, cDNA, or synthetic origin, or some combinations thereof) that, by human intervention, is separated from all or a portion of the polynucleotides with which it is associated in nature, is operably linked to a polynucleotide that it is not linked to in nature, or is present as part of a larger sequence that does not occur in nature. As used herein, the term "vector" refers to a nucleic acid molecule capable of transporting another nucleic acid to which it is linked. One type of vector is a "plasmid", which refers to a circular doublestranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. In addition, certain vectors are capable of directing the expression of genes to which they are operably linked. Such vectors are referred to herein as "recombinant expression vectors" (or "expression vectors" for short). In general, expression vectors useful in recombinant DNA techniques are often in the form of plasmids. In this description, "plasmid" and "vector" are used interchangeably as the plasmid is the most commonly used form of vector. However, the present disclosure is intended to include other forms of expression vectors, such as viral vectors (e.g., replication-deficient retroviruses, adenoviruses, and adeno-associated viruses), which serve equivalent functions.

"Transformation" as used herein refers to any process by which exogenous DNA enters a host cell. Transformation can be performed under natural or artificial conditions using various methods well known in the art. Transformation may rely on any known method for the insertion of exogenous nucleic acid sequences into prokaryotic or eukaryotic host cells. The method is selected based on the host cell to be transformed and may include, but is not limited to, transfection, viral infection, electroporation, lipofection, and particle bombardment. Such "transformed" cells include stably transformed cells in which the inserted DNA is capable of replication either as an autonomously replicating plasmid or as part of the host chromosome. Also included are cells that transiently express the inserted DNA or RNA for a limited time.

The term "recombinant host cell" (or "host cell" for short) refers to a cell into which exogenous DNA has been introduced. In one embodiment, the host cell comprises two or more (e.g., a plurality of) nucleic acids encoding an antibody, e.g., a host cell described in U.S. Patent No. 7,262,028. These terms are intended to refer not only to a particular subject cell but to the progeny of such a cell. Because certain modifications may occur in the progeny due to either mutation or environmental influences, such progeny may not, in fact, be identical to the parent cell, but are still included within the scope of the term "host cell" as used herein. In one embodiment, the host cell includes prokaryotic and eukaryotic cells selected from any kingdom of life. In another embodiment, eukaryotic cells include protist, fungal, plant, and animal cells. In another embodiment, host cells include, but are not limited to, the prokaryotic cell line *Escherichia coli*; the mammalian cell lines CHO, HEK 293, Jurkat, COS, NS0, SP2, and PER.C6; the insect cell line Sf9; and the fungal cell *Saccharomyces cerevisiae*. The term "treatment" (or "treat" or "treating") refers to slowing, interrupting, arresting, ameliorating, stopping, lowering, or reversing the progression or severity of an existing symptom, disorder, condition, or disease. Desired therapeutic effects include, but are not limited to, preventing the occurrence or recurrence of diseases, alleviating symptoms, reducing any direct or indirect pathological outcomes of diseases, preventing metastasis, delaying disease progression, ameliorating or alleviating disease conditions, and ameliorating or improving prognosis. In some embodiments, the antibody of the present disclosure is used to delay the progression of a disease or to slow the progression of a disease.

The term "prevention" (or "prevent" or "preventing") includes the inhibition of the development or progression of a disease or disorder, or symptoms of a specific disease or disorder. In some embodiments, subjects with a family history of cancer are candidates for preventive regimens. Generally, in the context of cancer, the term "prevention" refers to the administration of a drug prior to the appearance of signs or symptoms of cancer, particularly in subjects at risk of cancer.

The term "effective amount" refers to an amount or dose of the antibody or composition of the present disclosure which generates expected effects in a patient in need of treatment or prevention after administration to the patient in a single dose or multiple doses. The effective amount can be easily determined by an attending physician as a person skilled in the art by considering a variety of factors as follows: species such as mammals; weight, age, and general health condition; the specific disease involved; the extent or severity of the disease; response in an individual patient; specific antibody administered; mode of administration; bioavailability characteristics of the administered formulation; selected administration regimen; and use of any concomitant therapy.

The term "therapeutically effective amount" refers to an amount effective to achieve a desired therapeutic result at a necessary dose for a necessary period of time. The therapeutically effective amount of the antibody or antibody fragment or the composition may vary depending on a variety of factors such as disease state, age, sex, and weight of an individual, and the ability of the antibody or antibody portion to elicit a desired response in the individual. The therapeutically effective amount is also such an amount that any toxic or undesired effect of the antibody or antibody fragment or the composition is inferior to the therapeutically beneficial effect. "Therapeutically effective amount" preferably inhibits a measurable parameter (e.g., tumor growth rate, tumor volume, etc.) by at least about 20%, more preferably at least about 40%, even more preferably at least about 50%, 60%, or 70%, and still more preferably at least about 80% or 90%, relative to an untreated subject. The ability of a compound to inhibit a measurable parameter (e.g., cancer) can be evaluated in an animal model system that predicts efficacy in human tumors.

The term "prophylactically effective amount" refers to an amount effective to achieve a desired prophylactic result at a necessary dose for a necessary period of time. Generally, since a prophylactic dose is administered to a subject before or at an earlier stage of a disease, a prophylactically effective amount will be less than a therapeutically effective amount.

The term "pharmaceutical composition" refers to a composition that is present in a form allowing the effective biological activity of an active ingredient contained therein and does not contain additional ingredients having unacceptable toxicity to a subject to which the composition is administered.

The terms "linker", "linker peptide", "peptide linker", and the like are used interchangeably in the present application and refer to a peptide comprising one or more contiguous amino acids, wherein the amino acids are, for example, small amino acid residues or hydrophilic amino acid residues (e.g., glycine, serine, threonine, proline, aspartic acid, asparagine, etc.). A linker peptide typically comprises 5-50 amino acids in length, e.g., 10, 15, 20, 25, or 30 amino acids in length. It will be appreciated by those skilled in the art that many commonly used linkers may be used in the embodiments of the present disclosure.

The terms "individual" and "subject" are used interchangeably and include mammals. The mammals include, but are not limited to, domesticated animals (e.g., cows, sheep, cats, dogs, and horses), primates (e.g., human and non-human primates such as monkeys), rabbits, and rodents (e.g., mice and rats). Particularly, the individual or subject is a human.

### II. Anti-BDCA2 Antibody

In one aspect, the present disclosure provides an antibody or an antigen-binding fragment thereof specifically binding to BDCA2. The term "anti-BDCA2 antibody", "anti-BDCA2", "BDCA2 antibody", or "antibody that binds to BDCA2" refers to an antibody that is capable of binding to a BDCA2 protein or a fragment thereof with sufficient affinity such that the antibody can be used as a diagnostic and/or therapeutic agent targeting BDCA2.

In some embodiments, the antibody of the present disclosure binds to a human or cynomolgus monkey BDCA2 protein. In some embodiments, the antibody of the present disclosure binds to HEK293/hBDCA2/FcεRIγ cells and HEK293/Cyno BDCA2/FcεRIγ cells. In some embodiments, the antibody of the present disclosure inhibits the stimulation of human PBMC cells by the TLR9 agonist to release the cytokine IFNα. In some embodiments, the antibody of the present disclosure exhibits a strong effector function.

Any suitable method for producing antibodies may be employed to produce the antibody of the present disclosure. BDCA2 in any suitable form may be used as an immunogen (antigen) for antibody production. By way of example and without limitation, any BDCA2 variant or a fragment thereof may be used as an immunogen. In some embodiments, hybridoma cells that produce murine monoclonal anti-BDCA2 antibodies can be produced by methods well known in the art.

Antibodies derived from rodents (e.g., mice) may induce undesired immunogenicity of the antibodies when used as therapeutic agents *in vivo*. Repeated use of these antibodies induces immune responses in the human body against therapeutic antibodies. Such immune responses result in at least a loss of therapeutic efficacy and, in severe cases, a potentially lethal allergic reaction. One method for reducing the immunogenicity of rodent antibodies comprises producing chimeric antibodies, in which the mouse variable region is fused with the human constant region (Liu et al., (1987) Proc. Natl. Acad. Sci. USA 84:3439-43). However, the preservation of intact rodent variable regions in a chimeric antibody can still induce deleterious immunogenicity in patients. Grafting of complementarity determining region (CDR) loops of the rodent variable domain onto a human framework (i.e., humanization) has been used to further minimize rodent sequences (Jones et al., (1986) Nature 321:522; Verhoeyen et al., (1988) Science 239:1534). In some embodiments, the antibody of the present disclosure is a chimeric antibody. In some preferred embodiments, the antibody of the present disclosure is a humanized antibody. In some preferred embodiments of the present disclosure, the affinity of the murine antibody, the chimeric antibody, and/or the human antibody of the present disclosure for the antigen human BDCA2 or cynomolgus monkey BDCA2 is embodied as a K_{D} value of 1.0 × 10⁻⁶ M or less, a K_{D} value of 5.0 × 10⁻⁷ M or less, a K_{D} value of 2.5 × 10⁻⁷ M or less, a K_{D} value of 1.0 × 10⁻⁷ M or less, a K_{D} value of 5.0 × 10⁻⁸ M or less, a K_{D} value of 2.5 × 10⁻⁸ M or less, a K_{D} value of 1.0 × 10⁻⁸ M or less, a K_{D} value of 5.0 × 10⁻⁹ M or less, a K_{D} value of 2.5 × 10⁻⁹ M or less, a K_{D} value of 1.0 × 10⁻⁹ M or less, a K_{D} value of 5.0 × 10⁻¹⁰ M or less, a K_{D} value of 2.5 × 10⁻¹⁰M or less, a K_{D} value of 1.0 × 10⁻¹⁰ M or less, a K_{D} value of 5.0 × 10⁻¹¹ M or less, a K_{D} value of 2.5 × 10⁻¹¹ M or less, a K_{D} value of 1.0 × 10⁻¹¹ M or less, a K_{D} value of 5.0 × 10⁻¹² M or less, a K_{D} value of 2.5 × 10⁻¹² M or less, or a K_{D} value of 1.0 × 10⁻¹² M or less.

The amino acid sequences of the light and heavy chain variable regions and CDRs of the anti-BDCA2 murine antibodies of the present disclosure are shown in Table 2.

In some embodiments, the chimeric or humanized antibody of the present disclosure can be prepared on the basis of the sequence of the prepared murine monoclonal hybridoma antibody. DNA encoding an immunoglobulin having heavy and light chains can be obtained from a murine hybridoma of interest and engineered to comprise non-murine (e.g., human) immunoglobulin sequences using standard molecular biology techniques.

In some embodiments, to prepare the chimeric BDCA2 antibody described herein, the chimeric heavy chain and the chimeric light chain can be obtained by operably linking the immunoglobulin heavy chain and light chain variable regions of hybridoma origin to the human IgG constant regions, respectively, using methods known in the art (see, e.g., U.S. Pat. No. 4,816,567 to Cabilly et al.). In some embodiments, the chimeric antibody of the present disclosure comprises constant regions which can be selected from any human IgG subtype, such as IgG1, IgG2, IgG3, and IgG4, preferably IgG1.

In some embodiments, the chimeric BDCA2 antibody of the present disclosure can be obtained by "mixing and matching" a chimeric light chain expression plasmid with a chimeric heavy chain expression plasmid to transfect expression cells. The BDCA2 binding of such "mixed and matched" antibodies can be assayed using the above binding assays and other conventional binding assays (e.g., ELISA).

In the present disclosure, the expression plasmids for the various chimeric heavy and light chains were mixed and paired to transfect expression cells, thus obtaining anti-BDCA2 chimeric antibodies. For the humanized antibodies described herein, murine CDR regions can be inserted into human germline framework regions using methods known in the art. See U.S. Pat. No. 5,225,539 to Winter et al., and U.S. Pat. Nos. 5,530,101; 5,585,089; 5,693,762 and 6,180,370 to Queen et al.

In some embodiments, the amino acid change includes an amino acid deletion, insertion, or substitution. In some embodiments, the anti-BDCA2 antibody or the antigen-binding fragment thereof of the present disclosure includes those antibodies having an amino acid sequence that has been mutated by amino acid deletion, insertion, or substitution but still has at least about 90%, 91%, 92%, 93%, 94%, 95%, 96%, 97%, 98%, 99%, or 100% identity to amino acid sequences of the antibodies described above (particularly in the CDR regions set forth in the above sequences). In some embodiments, when compared to the CDR regions set forth in a particular sequence, the antibodies of the present disclosure have no more than 1, 2, 3, 4, or 5 amino acid mutations (deletions, insertions, or substitutions) in the CDR regions. In some embodiments, when compared to the framework regions set forth in a particular sequence, the antibodies of the present disclosure have no more than 1, 2, 3, 4, or 5 amino acid mutations (deletions, insertions, or substitutions) in the framework regions.

The term "percent (%) amino acid sequence identity", or simply "identity", is defined as the percentage of amino acid residues in a candidate amino acid sequence that are identical to those in a reference amino acid sequence after aligning the amino acid sequences (with gaps introduced if necessary) to achieve maximum percent sequence identity without considering any conservative substitution as part of sequence identity. Sequence alignment for the purpose of determining percent amino acid sequence identity can be performed using various methods in the art, for example, using computer software available to the public, such as the BLAST, BLAST-2, ALIGN, or MEGALIGN (DNASTAR) software. Those skilled in the art can determine suitable parameters for measuring alignment, including any algorithm required to achieve the maximum alignment over the full length of the aligned sequences.

Thus, in some embodiments, the antibody of the present disclosure comprises HCDR1, HCDR2, HCDR3, LCDR1, LCDR2, and LCDR3 as shown in any one combination in Table A below.

**Table A**

| Combination# | HCDR1, comprising or consisting of the following sequence (or a sequence differing therefrom by 1, 2, or 3 amino acids) | HCDR2, comprising or consisting of the following sequence (or a sequence differing therefrom by 1, 2, or 3 amino acids) | HCDR3, comprising or consisting of the following sequence (or a sequence differing therefrom by 1, 2, or 3 amino acids) | LCDR1, comprising or consisting of the following sequence (or a sequence differing therefrom by 1, 2, or 3 amino acids) | LCDR2, comprising or consisting of the following sequence (or a sequence differing therefrom by 1, 2, or 3 amino acids) | LCDR3, comprising or consisting of the following sequence (or a sequence differing therefrom by 1, 2, or 3 amino acids) |
|---|---|---|---|---|---|---|
| 1 | SEQ ID NO:33 | SEQ ID NO:34 | SEQ ID NO:35 | SEQ ID NO:36 | SEQ ID NO:37 | SEQ ID NO:38 |
| 2 | SEQ ID NO:39 | SEQ ID NO: 40 or 49 | SEQ ID NO:41 | SEQ ID NO: 42 or 48 | SEQ ID NO:43 | SEQ ID NO:44 |

In some embodiments, the antibody of the present disclosure comprises VH and VL as shown in any one combination in Table B below.

**Table B**

| Combination# | VH, comprising or consisting of the following sequence (or a sequence having at least 95% identity thereto) | VL, comprising or consisting of the following sequence (or a sequence having at least 95% identity thereto) |
|---|---|---|
| 1 | SEQ ID NO:1 | SEQ ID NO:2 |
| 2 | SEQ ID NO:3 | SEQ ID NO:4 |
| 3 | SEQ ID NO:13 | SEQ ID NO:15 |
| 4 | SEQ ID NO:14 | SEQ ID NO:15 |

In some embodiments, the BDCA2 antibody is an IgG antibody, e.g., an IgG1, IgG2, IgG3, or IgG4 antibody, or a modified form thereof, as described in the section below.

In some embodiments, one or more amino acid modifications may be introduced into an Fc region of the antibody provided herein, thus producing an Fc region variant. The Fc region variant may comprise a human Fc region sequence (such as the human IgG1, IgG2, IgG3, or IgG4 Fc region) comprising an amino acid modification (such as substitution) at one or more amino acid positions. For modifications to the constant domain/Fc region, see also the detailed description of the modifications below in this description.

In some embodiments, the anti-BDCA2 antibody or the antigen-binding fragment thereof of the present disclosure has one or more of the following properties:
(i) showing the same or similar binding affinity and/or specificity for BDCA2 as the antibody of the present disclosure;
(ii) inhibiting (e.g., competitively inhibiting) the binding of the antibody of the present disclosure to BDCA2;
(iii) binding to the same or overlapping epitope as the antibody of the present disclosure;
(iv) competing with the antibody of the present disclosure for binding to BDCA2; and
(v) having one or more biological properties of the antibody of the present disclosure.

In some embodiments, antibodies modified by cysteine engineering may need to be produced, such as "sulfo-MAb", wherein one or more residues of the antibodies are substituted by cysteine residues. In some embodiments, the antibodies provided herein may be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

### III. Bispecific Fusion Protein of the Present Disclosure

Components of the bispecific fusion protein of the present disclosure and the bispecific fusion protein of the present disclosure consisting of the components are described in detail below. It will be understood by those skilled in the art that any combination of any of the technical features of these components is within the contemplation of the present disclosure, unless otherwise expressly stated to the contrary. Furthermore, it will be understood by those skilled in the art that the bispecific fusion protein of the present disclosure may comprise any such combination of features, unless otherwise expressly stated to the contrary.

The similar terms "bispecific fusion protein specifically binding to BDCA2 and BAFF/APRIL", "bispecific antibody against BDCA2 and BAFF/APRIL", "anti-BDCA2×BAFF/APRIL bispecific fusion protein", "BDCA2×BAFF/APRIL bispecific antibody", and the like described herein refer to a bispecific fusion protein that is capable of binding to both the targets BDCA2 and BAFF/APRIL with sufficient affinity.

In one aspect, the present disclosure provides a bispecific fusion protein specifically binding to BDCA2 and BAFF/APRIL. In some embodiments, the bispecific fusion protein comprises a first target-binding region and a second target-binding region, wherein the first target-binding region specifically binds to BDCA2, and the second target-binding region specifically binds to BAFF and APRIL. In some embodiments, the bispecific fusion protein comprises an antibody (anti-BDCA2) or a fragment thereof specifically binding to BDCA2 and soluble TACI or a fragment thereof specifically binding to BAFF/APRIL.

As used herein, unless otherwise specified, when referring to "TACI" that forms a part of a bispecific fusion protein, it may refer to any form of the wild-type natural TACI molecule that retains the ability to specifically binding to BAFF and APRIL, such as full-length molecule, soluble fragments, domains, variants, and fusion proteins comprising the same.

In some embodiments, the first target-binding region and the second target-binding region are connected via a linker or directly connected without a linker.

In some specific embodiments of the bispecific fusion protein of the present disclosure, the anti-BDCA2 and TACI moieties may be in a tandem form comprised in one polypeptide chain (e.g., linked in tandem on one polypeptide chain via a linker and/or an Fc region). In some specific embodiments, the bispecific fusion protein according to the present disclosure further comprises an Fc region. In the bispecific fusion protein according to the present disclosure, the anti-BDCA2 and TACI moieties may be linked to the Fc region in a tandem form comprising both anti-BDCA2 and TACI, optionally further linked to a linker fragment.

In some preferred embodiments of the bispecific fusion protein of the present disclosure, the first target-binding region is an anti-BDCA2 antibody or an antigen-binding fragment thereof as follows: (i) the anti-BDCA2 antibody or the antigen-binding fragment thereof of the present disclosure; or (ii) exhibiting exactly the same binding specificity as the anti-BDCA2 antibody or the antigen-binding fragment thereof of the present disclosure.

In some embodiments of the bispecific fusion protein of the present disclosure, the second target-binding region comprises a BAFF/APRIL receptor or a functional fragment thereof specifically binding to BAFF and APRIL; preferably, the BAFF/APRIL receptor is TACI.

In some specific embodiments of the bispecific fusion protein of the present disclosure, the second target-binding region is TACI, which (i) comprises or consists of an amino acid sequence having at least 85%, 90%, 91%, 92%, 93%, 94%, 95%, 96%, 97%, 98%, or 99% identity to the amino acid sequence of SEQ ID NO: 20; (ii) comprises or consists of the amino acid sequence of SEQ ID NO: 20; or (iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 20.

In some specific embodiments of the bispecific fusion protein of the present disclosure, the second target-binding region is a fragment of a full-length TACI molecule. Preferably, the fragment is an extracellular region of a full-length TACI molecule or a functional variant thereof; more preferably, the fragment is a CRD2 of a full-length TACI molecule, e.g., a CRD2 of a wild-type TACI molecule, e.g., a CRD2 of a wild-type TACI molecule, or a variant CRD2 comprising a modification relative to the wild-type CRD2.

In some specific embodiments of the bispecific fusion protein of the present disclosure, the second target-binding region is a CRD2 of a TACI molecule, which (i) comprises or consists of an amino acid sequence having at least 85%, 90%, 91%, 92%, 93%, 94%, 95%, 96%, 97%, 98%, or 99% identity to the amino acid sequence of SEQ ID NO: 21; (ii) comprises or consists of the amino acid sequence of SEQ ID NO: 21; (iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 21.

In some embodiments, the bispecific fusion protein of the present disclosure comprises (i) a chain A: an anti-BDCA2 antibody light chain; and (ii) a chain B: an anti-BDCA2 antibody heavy chain linked, at the N-terminus or C-terminus, to TACI or a functional fragment thereof with or without a linker. In some more specific embodiments, the fusion protein molecule consists of 2 chains A and 2 chains B; preferably, the fusion protein molecule consists of 2 identical chains A and 2 identical chains B, i.e., the fusion protein molecule is a homodimer.

### Immunoglobulin Constant Domain

The bispecific fusion protein according to the present disclosure preferably comprises an immunoglobulin constant domain, e.g., an Fc region consisting of immunoglobulin constant regions CH2 and CH3. In some embodiments of the present disclosure, the present disclosure thus provides a bispecific fusion protein comprising an Fc region polypeptide chain consisting of immunoglobulin heavy chain constant regions CH2-CH3. The immunoglobulin constant domains comprised in the bispecific fusion protein according to the present disclosure, including the CH2 and CH3 constant domains in the Fc region, may be, independently of each other, immunoglobulin constant domains from human IgG, e.g., constant domains of human IgG1, IgG2, IgG3, or IgG4, preferably constant domains of human IgG1. In addition, the immunoglobulin constant domains comprised in the bispecific fusion protein according to the present disclosure, including the constant regions CH2 and CH3, may be, independently of each other, a natural sequence constant domain (e.g., a human natural sequence constant domain) or an amino acid sequence variant thereof, preferably a natural sequence constant domain.

In one preferred embodiment, the Fc region comprised in the bispecific fusion protein according to the present disclosure is an Fc region sequence from human IgG1, an amino acid sequence having at least 90%, 95%, or 99% identity to the sequence, or an amino acid sequence having no more than 1-10 (preferably 1-5) amino acid residue modifications compared to the sequence.

In some specific embodiments, the bispecific fusion protein according to the present disclosure may comprise polypeptide chains having two Fc regions, wherein, for example, the Fc regions of two homologous polypeptide chains are capable of pairing and dimerization. In such an embodiment, a first Fc region polypeptide and a second Fc region polypeptide constituting the dimerized Fc region of the antibody may both be Fc domains from IgG, e.g., human IgG1, IgG2, IgG3, or IgG4, preferably Fc domains of human IgG 1, and may be, independently of each other, a natural sequence Fc region and a variant Fc region as defined herein, provided that the first Fc region polypeptide and the second Fc region polypeptide can be paired and dimerized. Preferably, the first Fc region and the second Fc region are natural sequence Fc regions, particularly natural sequence Fc regions of human IgG1.

In some embodiments, the constant domain/Fc region of the present disclosure has one or more amino acid substitutions compared to a natural human constant domain/Fc region sequence; preferably, the one or more amino acid substitutions result in enhanced ADCC and/or CDC effect and/or prolonged *in vivo* half-life of the antibody and/or increased affinity for an Fc receptor (e.g., FcRn). In one embodiment, the constant domain/Fc region of the present disclosure has amino acid substitutions M252Y/S254T/T256E compared to a natural human constant domain/Fc region sequence (amino acid positions are numbered according to the EU numbering scheme).

In some embodiments, the constant domain/Fc region of the present disclosure is low-fucosylated or defucosylated, or is an antibody or a fragment thereof or a multispecific binding molecule with increased proportion of bisecting GlcNac structure.

In some embodiments, the constant domain/Fc region of the present disclosure has altered glycosylation and/or altered amino acid sequences, e.g., is low-fucosylated or defucosylated and has a combination of amino acid substitutions of M252Y/S254T/T256E.

The details regarding the modifications to the constant domain/Fc region of the bispecific fusion protein are also provided in the detailed description of the modifications below in this description.

### Linker

In the bispecific fusion protein according to the present disclosure, different target-binding regions may be linked using a linker. The linker that can be used in the bispecific fusion protein of the present disclosure is not particularly limited. Those skilled in the art can readily determine available linker sequences based on the components to be linked and the linking positions.

In an embodiment of the present disclosure, those skilled in the art may select different linkers depending on the linking position of different target-binding regions, i.e., linking to the N-terminus or C-terminus of the Fc region. In one embodiment, an Fc region polypeptide chain is linked, at the N-terminus, to a single target-binding region or two different target-binding regions linked in tandem by a linker, wherein the linker preferably comprises (one or two) cysteine residues, such that (one pair of or two pairs of) disulfide bonds can be formed when two polypeptide chains comprising the Fc region are paired to stabilize the structure of the bispecific fusion protein formed thereby. Thus, in some embodiments, the Fc region polypeptide chain comprised in the bispecific fusion protein of the present disclosure carries, at the N-terminus, a linker sequence comprising "CPPC". In other embodiments, the Fc region polypeptide chain comprised in the bispecific fusion protein of the present disclosure carries, at the N-terminus, a hinge region derived from an immunoglobulin, e.g., a hinge region amino acid sequence comprising "CPPC", such as the amino acid sequence "EPKSCDKTHTCPPCP" or "EPKSSDKTHTCPPCP", more preferably, the amino acid sequence "EPKSCDKTHTCPPCP".

In a preferred embodiment of the present disclosure, the linker is a flexible linker peptide having a length of preferably 5-50 amino acids, and more preferably comprises glycine (G) and/or serine (S) and/or threonine (T) residues. In some specific embodiments, the linker has a length of 5-50 amino acids, such as 10, 15, 20, 25, or 30 amino acids, or has an amino acid length falling between any two integers. In some embodiments, the linker is (GGGGS)n, (GSSSS)n, (GGGSG)n, (GGSGG)n, (GSGGG)n, (SGGGG)n, (GGTGS)n, (GTSPGG)n, (GNGGGS)n, (GGG)n, (DGGGS)n, (TGEKP)n, (GGRR)n, (EGKSSGSGSESKVD)n, (KESGSVSSEQLAQFRSLD)n, (GGRRGGGS)n, (LRQRDGERP)n, (LRQKDGGGSERP)n, (GSTSGSGKPGSGEGSTKG)n, and/or G₄S-GGSGG-G₄S-SGGGG, etc., wherein n is an integer equal to or greater than 1; for example, n is an integer of 1, 2, 3, 4, 5, 6, 7, 8, or 9. In a specific embodiment, the linker is (G₄S)n or (GS₄)n, wherein n is an integer equal to or greater than 1; for example, n is an integer of 1, 2, 3, 4, 5, 6, 7, 8, or 9. In some most preferred embodiments, the linker is GS₄, G₄S, (GS₄)₂, (G₄S)₂, (GS₄)₃, (G₄S)₃, (GS₄)₄, or (G₄S)₄.

Embodiments of Bispecific Fusion Protein of the Present Disclosure

In some embodiments, the bispecific fusion protein of the present disclosure comprises (i) a chain A: an anti-BDCA2 antibody light chain; and (ii) a chain B: an anti-BDCA2 antibody heavy chain linked, at the N-terminus or C-terminus, to TACI or a functional fragment thereof with or without a linker.

In some more specific embodiments, the chain B comprises, from N-terminus to C-terminus, the following elements in sequence:
(i) an anti-BDCA2 heavy chain, a linker, and TACI; or
(ii) TACI, a linker, and an anti-BDCA2 heavy chain; or
(iii) an anti-BDCA2 heavy chain, a linker, TACI, and a polyserine residue; or
(iv) TACI, a polyserine residue sequence, a linker, and an anti-BDCA2 heavy chain;

wherein TACI is a full-length TACI molecule or a fragment thereof;
the linker is preferably GS₄, G₄S, (GS₄)₂, (G₄S)₂, (GS₄)₃, (G₄S)₃, (GS₄)₄, or (G₄S)₄; the polyserine residue is SSSS, SSSSS, SSSSSS, SSSSSSS, SSSSSSSS, SSSSSSSSS, or SSSSSSSSSS,
and optionally, the linker may be absent.

In some more specific embodiments, the chain B comprises, from N-terminus to C-terminus, the following elements in sequence:
(i) an anti-BDCA2 heavy chain, a linker, and a CRD2 domain; or
(ii) a CRD2 domain, a linker, and an anti-BDCA2 heavy chain; or
(iii) an anti-BDCA2 heavy chain, a linker, a CRD2 domain, and a polyserine residue; or
(iv) a CRD2 domain, a polyserine residue sequence, a linker, and an anti-BDCA2 heavy chain;

the linker is preferably GS₄, G₄S, (GS₄)₂, (G₄S)₂, (GS₄)₃, (G₄S)₃, (GS₄)₄, or (G₄S)₄; the polyserine residue is SSSS, SSSSS, SSSSSS, SSSSSSS, SSSSSSSS, SSSSSSSSS, or SSSSSSSSSS,
and optionally, the linker may be absent.

In some more specific embodiments, the chain A comprises or consists of the amino acid sequence set forth in SEQ ID NO: 18 or an amino acid sequence having at least 85%, 90%, 91%, 92%, 93%, 94%, 95%, 96%, 97%, 98%, or 99% identity to the amino acid sequence. In some more specific embodiments, the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence.

In some more specific embodiments, the chain A comprises or consists of the amino acid sequence set forth in SEQ ID NO: 18 or an amino acid sequence having at least 85%, 90%, 91%, 92%, 93%, 94%, 95%, 96%, 97%, 98%, or 99% identity to the amino acid sequence; and the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence.

In some more specific embodiments, the chain A comprises or consists of the amino acid sequence set forth in SEQ ID NO: 18, and the chain B comprises or consists of the amino acid sequence set forth in any one of SEQ ID NOs: 22-32, 50-51, and 53.

In some embodiments, the bispecific fusion protein of the present disclosure may comprise two bispecific antigen-binding polypeptides comprising an Fc region (e.g., chain B) arranged in tandem, wherein the two bispecific antigen-binding polypeptides can associate to form a dimer through, e.g., the dimerization association of the Fc regions; preferably, the two bispecific antigen-binding polypeptides are identical.

In some preferred embodiments, the bispecific fusion protein of the present disclosure may comprise two pairs of polypeptides (e.g., chain A and chain B), wherein two bispecific antigen-binding polypeptides comprising an Fc region (e.g., chain B) are included, wherein the two bispecific antigen-binding polypeptides can associate to form a dimer through, for example, the dimerization of the Fc regions; preferably, the two bispecific antigen-binding polypeptides are identical; more preferably, the two pairs of bispecific antigen-binding polypeptides are identical.

In one embodiment, the bispecific fusion protein of the present disclosure comprises an amino acid modification, such as an amino acid substitution, addition, or deletion, preferably an amino acid substitution, and more preferably a conservative amino acid substitution.

In one embodiment, the two identical bispecific antigen-binding polypeptides of the bispecific fusion protein of the present disclosure are non-covalently linked or covalently linked. In one embodiment, the covalent linkage is a disulfide bond.

For modifications, see the detailed description below in this description.

In one embodiment, the BDCA2×BAFF/APRIL bispecific fusion protein or the target-binding fragment thereof of the present disclosure is capable of inhibiting the overactivation of plasmacytoid dendritic cells and/or eliminating plasmacytoid dendritic cells, inhibiting IFNα release, inhibiting B cell proliferation, treating or ameliorating inflammatory disorders/diseases and/or autoimmune disorders/diseases, such as systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, ulcerative colitis, neuromyelitis optica, neuromyelitis optica spectrum disorder, primary Sjögren's syndrome, myasthenia gravis, and various combinations thereof.

### IV. Modifications for Modifying Anti-BDCA2 Antibody or Fragment Thereof or Multispecific Binding Molecule

The molecule of the present disclosure (e.g., the anti-BDCA2 antibody molecule or the fragment thereof or the multispecific binding molecule) may be mutated to obtain desired properties.

For example, the FR regions contained in the variable regions of the molecule of the present disclosure may be modified to reduce their immunogenicity or remove T cell epitopes.

In one embodiment, the amino acid modification described herein occurs in a region outside the CDRs (e.g., in FRs). In one embodiment, the substitution is a conservative substitution. The conservative substitution refers to a substitution of an amino acid with another amino acid of the same class, for example, a substitution of an acidic amino acid with another acidic amino acid, a substitution of a basic amino acid with another basic amino acid, or a substitution of a neutral amino acid with another neutral amino acid.

In certain embodiments, the substitutions occur in the CDRs of the antibody. Generally, the obtained variant has modifications (e.g., improvements) in certain biological properties (e.g., increased affinity) relative to the parent antibody and/or will substantially retain certain biological properties of the parent antibody. Exemplary substituted variants are affinity-matured antibodies.

In certain embodiments, one or more amino acid modifications may be introduced into the Fc region of the antibody provided herein, thus producing an Fc region variant. The Fc region variant may comprise a human Fc region sequence (e.g., a human IgGl, IgG2, IgG3, or IgG4 Fc region) comprising an amino acid modification (e.g., a substitution) at one or more amino acid positions.

In certain embodiments, antibodies modified by cysteine engineering may need to be produced, such as "sulfo mAb", wherein one or more residues of the antibodies are substituted with cysteine residues. In one embodiment, cysteine residues may be increased or decreased, for example, to facilitate assembly of different chains of the molecule or to increase or decrease the stability of the molecule. The molecule of the present disclosure may also be engineered to introduce one or more modifications into the constant region, e.g., the heavy chain constant region, e.g., the Fc region, to alter one or more functional properties of the antibody, such as serum half-life, complement fixation, Fc receptor binding, and/or antigen-dependent cellular cytotoxicity. Furthermore, the molecule of the present disclosure may be chemically modified (e.g., one or more chemical moieties may be attached to the antibody) or be modified to alter its glycosylation, thereby altering one or more functional properties of the molecule again.

In some embodiments, the heavy chain constant region or Fc region contained in the molecule of the present disclosure may be modified to adjust one or more properties of the antibody, for example, to alter its effector functions, such as ADCC and/or CDC activity, and/or to alter its *in vivo* half-life, such as to enhance ADCC activity, enhance CDC activity, and/or enhance ADCC and CDC activity, or to prolong the *in vivo* half-life of the antibody, and/or to increase affinity for Fc receptors (e.g., FcRn). In some embodiments, the modification is one or more amino acid substitutions.

In some preferred embodiments, the one or more amino acid substitutions occur at one or more of positions 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 328, 329, 330, 331, 333, 334, 335, 337, 338, 340, 360, 373, 376, 378, 382, 388, 389, 398, 414, 416, 419, 430, 434, 435, 437, 438, and 439 of the heavy chain constant region sequence according to the EU numbering system. In some preferred embodiments, the one or more amino acid substitutions occur at one or more of positions L234, L235, G236, S239, F243, T256, D265, H268, D270, K290, R292, S298, Y300, V305, K326, A330, I332, E333, K334, A339, and P396 of the heavy chain constant region sequence according to the EU numbering system. In some preferred embodiments, the one or more amino acid substitutions are one or more substitutions selected from G236A, S239D, F243L, T256A, K290A, R292P, S298A, Y300L, V305I, A330L, I332E, E333A, K334A, A339T, and P396L according to the EU numbering system. In some preferred embodiments, the one or more amino acid substitutions are one or more substitutions selected from an N297A substitution, an N297Q substitution, an L235A substitution together with an L237A substitution, an L234A substitution together with an L235A substitution, an E233P substitution, an L234V substitution, an L235A substitution, a C236 deletion, a P238A substitution, a D265A substitution, an A327Q substitution, and a P329A substitution according to the EU numbering system. In some preferred embodiments, the one or more amino acid substitutions occur at one or more of positions 235, 239, 243, 292, 300, 330, 332, and 396 of the heavy chain constant region sequence according to the EU numbering system. In some preferred embodiments, the one or more amino acid substitutions are at least one selected from S239D, L235V, F243L, R292P, Y300L, A330L, I332E, and P396L according to the EU numbering system.

In some preferred embodiments, the heavy chain constant region has one or more sets of mutations that occur simultaneously at combinations of positions selected from the following according to the EU numbering system: (1) L235/F243/R292/Y300/P396, (2) F243/R292/Y300/V305/P396, (3) D270/K326/A330/K334, (4) S239/A330/I332, (5) S298/E333/K334, (6) L234/L235/G236/S239/H268/D270/S298, (7) M252/S254/T256, (8) L234/L235/D265, (9) G236/S239/I332, and (10) S239/I332.

In some preferred embodiments, the heavy chain constant region has one or more sets of mutations selected from the following combinations of mutations according to the EU numbering system: (1) L235V/F243L/R292P/Y300L/P396L, (2) F243L/R292P/Y300L/V305I/P396L, (3) D270E/K326D/A330M/K334E, (4) S239D/A330L/I332E, (5) S298A/E333A/K334A, (6) L234Y/L235Q/G236W/S239M/H268D/D270E/S298A, (7) M252Y/S254T/T256E, (8) L234A/L235A/D265A, (9) L234F/L235E/D265A, (10) G236A/S239D/I332E, and (11) S239D/I332E.

In some embodiments, the molecule of the present disclosure may be subjected to post-translational modification to alter the effector function of the antibody, for example, to alter the glycosylation type of the molecule of the present disclosure.

In some embodiments, the glycosylation of the molecule of the present disclosure may be modified. For example, an aglycosylated antibody or binding molecule may be prepared (i.e., the antibody or binding molecule lacks glycosylation). Glycosylation may be altered to, for example, increase the affinity of the molecule of the present disclosure for an antigen. Such carbohydrate modifications may be achieved by, for example, altering one or more glycosylation sites within the antibody sequence. For example, one or more amino acid substitutions may be performed to eliminate one or more variable region framework glycosylation sites, thereby eliminating glycosylation at the site(s). Such aglycosylation may increase the affinity of the antibody for the antigen. See, e.g., U.S. Patent Nos. 5,714,350 and 6,350,861.

Additionally or optionally, an antibody or binding molecule having an altered type of glycosylation may be prepared, such as a low-fucosylated antibody having reduced amounts of fucosyl residues or an antibody or fusion protein having an increased proportion of a bisecting GlcNac structure. Such altered glycosylation has been demonstrated to increase the ADCC ability of the antibody or binding molecule. Such carbohydrate modifications may be achieved by, for example, expressing the antibody or binding molecule in a host cell with an altered glycosylation mechanism. Cells with an altered glycosylation mechanism have been described in the art and can be used as host cells for expressing the molecule of the present disclosure to produce antibodies or binding molecules with altered glycosylation. For example, cell lines Ms704, Ms705, and Ms709 lack the fucosyltransferase gene FUT8 (α(1,6)-fucosyltransferase), and thus antibodies or binding molecules expressed in the Ms704, Ms705, and Ms709 cell lines lack fucose on their carbohydrates. The Ms704, Ms705, and Ms709 FUT8^{-/-} cell lines were produced by the targeted disruption of the FUT8 gene in CHO/DG44 cells using two substitution-type vectors (see U.S. Patent Publication No. 20040110704 and Yamane-Ohnuki et al. (2004) Biotechnol Bioeng 87:614-22). As another example, EP 1,176,195 describes a cell line with a functionally disrupted FUT8 gene, which encodes a fucosyltransferase, such that antibodies expressed in such a cell line exhibit low fucosylation by reducing or eliminating the α-1,6 bond-related enzyme. EP 1,176,195 also describes cell lines with low or no enzymatic activity for adding fucose to the N-acetylglucosamine that binds to the Fc region of the antibody, such as the rat myeloma cell line YB2/0 (ATCC CRL 1662). PCT Publication WO 03/035835 describes a variant CHO cell line, Lec13 cells, with reduced ability to link fucose to Asn(297)-linked carbohydrates, thereby also causing low fucosylation of antibodies expressed in the host cell (see also Shields et al. (2002) J. Biol. Chem. 277:26733-26740). Antibodies or binding molecules with modified glycosylation profiles can also be produced in chicken eggs, as described in PCT Publication WO 06/089231. Optionally, antibodies or binding molecules with modified glycosylation profiles can be produced in plant cells such as duckweed. Methods for producing antibodies in a plant system are disclosed in U.S. Patent Application corresponding to Alston & Bird LLP Attorney Docket No. 040989/314911, filed on 11 August, 2006. PCT Publication WO 99/54342 describes cell lines engineered to express glycoprotein-modifying glycosyltransferases (e.g., β(1,4)-N-acetylglucosaminyltransferase III (GnTIII)) such that antibodies or binding molecules expressed in the engineered cell lines exhibit the increased proportion of a bisecting GlcNac structure, which results in increased ADCC activity of the antibodies or binding molecules (see also Umana et al. (1999) Nat. Biotech. 17:176-180). Optionally, fucosidase can be used to cleave off fucosyl residues of the antibody; for example, fucosidase, α-L-fucosidase, removes fucosyl residues from the antibody or binding molecule (Tarentino et al. (1975) Biochem. 14:5516-23).

Another modification contemplated by the present disclosure for the molecule herein is pegylation. An antibody or binding molecule can be pegylated to, for example, extend the biological (e.g., serum) half-life of the antibody or binding molecule. To pegylate an antibody or binding molecule, the antibody or binding molecule is typically reacted with polyethylene glycol (PEG), such as a reactive ester or aldehyde derivative of PEG, under conditions in which one or more PEG groups are linked to the antibody or binding molecule. Preferably, pegylation is performed via an acylation reaction or an alkylation reaction with a reactive PEG molecule (or an analogous reactive water-soluble polymer). The term "polyethylene glycol" as used herein is intended to encompass any one of the forms of PEG that have been used to derivatize other proteins, such as mono(C1-C10)alkoxy polyethylene glycol, aryloxy polyethylene glycol, or polyethylene glycol-maleimide. In certain embodiments, the antibody or binding molecule to be pegylated is an aglycosylated antibody or binding molecule. Methods for pegylating proteins are known in the art and can be applied to the molecule of the present disclosure. See, for example, EPO 154 316 and EP 0 401 384.

Accordingly, the present disclosure relates to an antibody or a fragment thereof or a multispecific binding molecule featuring enhanced ADCC and/or prolonged half-life and/or increased affinity for an Fc receptor (e.g., FcRn). In some embodiments, the antibody or the fragment thereof or the multispecific binding molecule featuring enhanced ADCC and/or prolonged half-life and/or increased affinity for an Fc receptor (e.g., FcRn) is an antibody or a fragment thereof or a multispecific binding molecule having an altered glycosylation type, e.g., a low-fucosylated or defucosylated antibody or a fragment thereof or a multispecific binding molecule, or an antibody or a fragment thereof or a multispecific binding molecule having an increased proportion of a bisecting GlcNac structure. In some embodiments, the antibody featuring enhanced ADCC and/or prolonged half-life and/or increased affinity for an Fc receptor (e.g., FcRn) carries an amino acid substitution combination of M252Y/S254T/T256E. In some embodiments, the antibody featuring enhanced ADCC and/or prolonged half-life and/or increased affinity for an Fc receptor (e.g., FcRn) has altered glycosylation and/or altered amino acid sequences, e.g., is low-fucosylated or defucosylated and carries an amino acid substitution combination of M252Y/S254T/T256E in the Fc region.

As described herein, the antibody featuring enhanced ADCC and/or prolonged half-life and/or increased affinity for an Fc receptor (e.g., FcRn) can be obtained by modifying an antibody or a fragment thereof or a multispecific binding molecule or expressing an antibody or a fragment thereof or a multispecific binding molecule in a host cell with an altered glycosylation mechanism.

### V. Nucleic Acid of Present Disclosure and Vector and Host Cell Comprising Same

In one aspect, the present disclosure provides a nucleic acid encoding any of the above antibodies, bispecific fusion proteins, or any fragments thereof (e.g., antigen/target-binding fragments). The present disclosure also encompasses nucleic acids that hybridize to the nucleic acid described above under stringent conditions, nucleic acids that have one or more substitutions (e.g., conservative substitutions), deletions, or insertions compared to the nucleic acid described above, or nucleic acid sequences having at least 80%, at least 85%, at least 90%, 91%, 92%, 93%, 94%, 95%, 96%, 97%, 98%, 99%, or higher identity to the nucleic acid described above.

For example, the nucleic acid of the present disclosure includes a nucleic acid encoding an amino acid sequence selected from any one of SEQ ID NOs: 1-6, 9-18, 20-32, and 50-53, or a nucleic acid encoding an amino acid sequence having at least 85%, 90%, 91%, 92%, 93%, 94%, 95%, 96%, 97%, 98%, or 99% identity to the amino acid sequence selected from any one of SEQ ID NOs: 1-6, 9-18, 20-32, and 50-53.

As will be appreciated by those skilled in the art, each antibody/fusion protein or polypeptide amino acid sequence may be encoded by a variety of nucleic acid sequences because of codon degeneracy. Nucleic acid sequences encoding the molecule of the present disclosure may be generated using methods well known in the art, e.g., *de novo* solid-phase DNA synthesis, or PCR amplification.

In one aspect, the present disclosure provides a nucleic acid encoding any of the above antibodies/fusion proteins or any peptide chains/fragments thereof. When expressed in a suitable expression vector, a polypeptide encoded by the nucleic acid is capable of exhibiting a binding ability for human and/or monkey (e.g., cynomolgus monkey) BDCA2 and/or BAFF/APRIL.

In one embodiment, nucleic acids encoding each chain of the antibody, the bispecific binding molecule, the fusion protein, the fragment thereof, or the multispecific binding molecule of the present disclosure may be in the same vector or in different vectors. In yet another embodiment, nucleic acids encoding each chain of the antibody, the bispecific binding molecule, the fusion protein, the fragment thereof, or the multispecific binding molecule of the present disclosure may be introduced into the same or different host cells for expression. Thus, in some embodiments, the method for producing the antibody, the bispecific binding molecule, the fusion protein, the fragment thereof, or the multispecific binding molecule of the present disclosure comprises a step of: culturing a host cell comprising a nucleic acid encoding each chain of the molecule under conditions suitable for the expression of the chains to produce the antibody, the bispecific binding molecule, the fusion protein, the fragment thereof, or the multispecific binding molecule of the present disclosure.

In another aspect, the present disclosure provides a vector comprising the nucleic acid described above. In one preferred embodiment, the vector is an expression vector. It is well understood by those skilled in the art that vectors commonly used in the art to which the present disclosure pertains can be applied to the present disclosure.

In one embodiment, the present disclosure provides a host cell comprising the nucleic acid or the vector.

The term "host cell" refers to a cell into which an exogenous polynucleotide has been introduced, including the progeny of such cells. Host cells include "transformants" and "transformed cells", which include primary transformed cells and progenies derived therefrom, regardless of the number of passages. The progeny may not be exactly the same as parent cells in terms of the nucleic acid content, and may contain mutations. Mutant progenies having the same function or biological activity that are screened or selected from the initially transformed cells are included herein. Host cells are any type of cell system that may be used to produce the antibody molecule of the present disclosure, including eukaryotic cells, e.g., mammalian cells (e.g., CHO cells or HEK293 cells), insect cells, yeast cells, and prokaryotic cells, e.g., *Escherichia coli* cells. Host cells include cultured cells, as well as cells within a transgenic animal, a transgenic plant, or a cultured plant tissue or animal tissue. Host cells may also be cells having an altered glycosylation mechanism as described herein, e.g., cells with complete or partial knockout of the FUT8 gene or complete or partial disruption of the FUT8 gene or protein function, e.g., CHO cells with knockout of the FUT8 gene.

### VI. Composition of Present Disclosure

In some embodiments, the present disclosure provides a composition comprising any of the anti-BDCA2 antibodies, BDCA2×BAFF/APRIL bispecific fusion proteins, or the antigen/target-binding fragments thereof described herein; preferably, the composition is a pharmaceutical composition. In one embodiment, the composition further comprises a pharmaceutical supplementary material. The term "pharmaceutical supplementary material" refers to a diluent, an adjuvant, a carrier, an excipient, a stabilizer, or the like, which is administered with an active substance.

In one embodiment, the composition (e.g., the pharmaceutical composition) comprises the anti-BDCA2 antibody, the BDCA2×BAFF/APRIL bispecific fusion protein, or the antigen/target-binding fragment thereof of the present disclosure, and a combination of one or more additional therapeutic agents (e.g., an anti-inflammatory agent, a chemotherapeutic agent, a cytotoxic agent, a vaccine, an additional antibody, an anti-infective active agent, a small molecule drug, or an immunomodulatory agent).

The pharmaceutical composition of the present disclosure may further comprise one or more additional therapeutic agents, which are required for a specific indication being treated and encompass any substance that is effective in preventing or treating tumors (e.g., cancer) and inflammations (e.g., chronic inflammation, local inflammation, or systemic inflammation), preferably those therapeutic agents that do not adversely affect the activity of each other, for example, glucocorticoids. The therapeutic agents are suitably present in combination in amounts that are effective for the intended application.

### VII. Preparation of Anti-BDCA2 Antibody or BDCA2×BAFF/APRIL Bispecific Fusion Protein of Present Disclosure

In one embodiment, the present disclosure provides a method for preparing an anti-BDCA2 antibody or a BDCA2×BAFF/APRIL bispecific fusion protein, which comprises culturing a host cell comprising a nucleic acid encoding an anti-BDCA2 antibody, a BDCA2×BAFF/APRIL bispecific fusion protein, or a fragment thereof, or an expression vector comprising the nucleic acid, under conditions suitable for expression of the anti-BDCA2 antibody, the BDCA2×BAFF/APRIL bispecific fusion protein, or the fragment thereof, and optionally isolating the anti-BDCA2 antibody, the BDCA2×BAFF/APRIL bispecific fusion protein, or the fragment thereof. In a certain embodiment, the method further comprises recovering the anti-BDCA2 antibody, the BDCA2×BAFF/APRIL bispecific fusion protein, or the fragment thereof from the host cell (or the host cell medium).

To recombinantly produce the anti-BDCA2 antibody, the BDCA2×BAFF/APRIL bispecific fusion protein, or the fragment thereof of the present disclosure, a nucleic acid encoding the anti-BDCA2 antibody, BDCA2×BAFF/APRIL bispecific fusion protein, or the fragment thereof is first isolated, and the nucleic acid is inserted into a vector for further cloning and/or expression in a host cell. Such nucleic acids can be easily isolated and sequenced by using conventional procedures, for example, by using an oligonucleotide probe that is capable of specifically binding to the nucleic acid encoding the anti-BDCA2 antibody, the BDCA2×BAFF/APRIL bispecific fusion protein, or the fragment thereof of the present disclosure.

The anti-BDCA2 antibody, the BDCA2×BAFF/APRIL bispecific fusion protein, or the fragment thereof of the present disclosure prepared as described herein can be purified by known techniques such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art. The purity of the anti-BDCA2 antibody, the BDCA2×BAFF/APRIL bispecific fusion protein, or the fragment thereof of the present disclosure can be determined by any one of a variety of well-known analytical methods including size exclusion chromatography, gel electrophoresis, high-performance liquid chromatography, and the like.

### VIII. Combination Product or Kit

In some embodiments, the present disclosure also provides a combination product comprising the anti-BDCA2 antibody, the BDCA2×BAFF/APRIL bispecific fusion protein, or the fragment thereof of the present disclosure, and one or more additional therapeutic agents (e.g., a chemotherapeutic agent, an additional antibody, a cytotoxic agent, an anti-infective active agent, a small molecule drug, or an immunomodulatory agent).

In some embodiments, the combination product is used for preventing or treating a disease associated with BDCA2 and/or BAFF/APRIL and/or a disease mediated by BDCA2 and/or BAFF/APRIL. In some embodiments, the additional therapeutic agent is an existing standard therapeutic agent.

In some embodiments, two or more of the ingredients in the combination product may be administered to a subject in combination sequentially, separately, or simultaneously.

In some embodiments, the present disclosure also provides a kit comprising the anti-BDCA2 antibody, the BDCA2×BAFF/APRIL bispecific fusion protein, or the fragment thereof, the pharmaceutical composition, or the combination product of the present disclosure, and optionally a package insert directing administration.

In some embodiments, the present disclosure further provides a pharmaceutical article of manufacture comprising the anti-BDCA2 antibody, the BDCA2×BAFF/APRIL bispecific fusion protein, or the fragment thereof, the pharmaceutical composition, or the combination product of the present disclosure, wherein optionally, the pharmaceutical article of manufacture further comprises a package insert directing administration.

### IX. Use of Anti-BDCA2 Antibody and BDCA2×BAFF/APRIL Bispecific Fusion Protein of Present Disclosure and Treatment Method

The antibodies, the antigen-binding fragments thereof, and the bispecific multivalent binding proteins described herein can be used to detect BDCA2 and/or BAFF/APRIL, given their ability to bind to human BDCA2 and/or BAFF/APRIL, e.g., in a biological sample containing cells expressing one or both of those target antigens. The antibodies, the antigen-binding fragments, and the binding proteins of the present disclosure can be used in conventional immunoassays, e.g., enzyme-linked immunosorbent assay (ELISA), radioimmunoassay (RIA), or tissue immunohistochemistry. The present disclosure provides a method for detecting BDCA2 and/or BAFF/APRIL in a biological sample, which comprises contacting a biological sample with the antibody, the antigen-binding portion thereof, or the binding protein of the present disclosure, and detecting whether binding to a target antigen occurs, thereby detecting whether the target is present in the biological sample. The antibody, the antigen-binding fragment, or the binding protein may be directly or indirectly labeled with a detectable substance to facilitate detection of the bound or unbound antibody/fragment/binding protein. Suitable detectable substances include various enzymes, prosthetic groups, fluorescent materials, luminescent materials, and radioactive materials. Examples of suitable enzymes include horseradish peroxidase, alkaline phosphatase, β-galactosidase, or acetylcholinesterase. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, or phycoerythrin; examples of luminescent materials include luminol; examples of suitable radioactive substances include ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm.

In one embodiment, a therapeutically effective amount of the anti-BDCA2 antibody, the BDCA2×BAFF/APRIL bispecific fusion protein, the fragment thereof, the pharmaceutical composition, or the combination product disclosed herein prevents or treats an inflammatory disorder/disease and/or an autoimmune disorder/disease and/or a tumor, e.g., a hematopoietic tumor, inhibits overactivation of plasmacytoid dendritic cells and/or eliminates plasmacytoid dendritic cells, inhibits IFNα release, and inhibits B cell proliferation.

In some specific embodiments, a therapeutically effective amount of the anti-BDCA2 antibody, the BDCA2×BAFF/APRIL bispecific fusion protein, the fragment thereof, the pharmaceutical composition, or the combination product disclosed herein prevents or treats systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, ulcerative colitis, neuromyelitis optica, neuromyelitis optica spectrum disorder, primary Sjögren's syndrome, myasthenia gravis, or any combination thereof.

In another aspect, the present disclosure relates to a method for preventing or treating an inflammatory disorder/disease and/or an autoimmune disorder/disease and/or a tumor, e.g., a hematopoietic tumor, inhibiting the overactivation of plasmacytoid dendritic cells and/or eliminating plasmacytoid dendritic cells, inhibiting IFNα release, and inhibiting B cell proliferation, which comprises administering to the subject an effective amount of the anti-BDCA2 antibody, the BDCA2×BAFF/APRIL bispecific fusion protein, the fragment thereof, the pharmaceutical composition, or the combination product disclosed herein.

The subject may be a mammal, e.g., a primate, preferably a higher primate, such as a human (e.g., a patient suffering from or at risk of suffering from the disease described herein). In one embodiment, the subject is suffering from or at risk of suffering from the disease described herein. In certain embodiments, the subject is receiving or has received other therapies, such as standard treatment regimens. Alternatively or in combination, the subject is immunocompromised due to infection or is at risk of being immunocompromised due to infection.

In some embodiments, the prevention or treatment method described herein further comprises administering to the subject or individual the anti-BDCA2 antibody, the BDCA2×BAFF/APRIL bispecific fusion protein, the fragment thereof, the pharmaceutical composition, or the combination product disclosed herein in combination with one or more additional therapies, e.g., therapeutic modalities and/or additional therapeutic agents.

The anti-BDCA2 antibody, the BDCA2×BAFF/APRIL bispecific fusion protein, or the fragment thereof (and the pharmaceutical composition comprising the same) of the present disclosure can be administered by any suitable method, including parenteral administration, intrapulmonary administration, intranasal administration, and, if required by locoregional treatment, intralesional administration. Parenteral infusion includes intramuscular, intravenous, intraarterial, intraperitoneal, or subcutaneous administration. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat a disease, the appropriate dose of the anti-BDCA2 antibody, the BDCA2×BAFF/APRIL bispecific fusion protein, or the fragment thereof of the present disclosure (when used alone or in combination with one or more additional therapeutic agents) will depend on the type of the disease to be treated, the type of the anti-BDCA2 antibody, the BDCA2×BAFF/APRIL bispecific fusion protein, or the fragment thereof, the severity and progression of the disease, whether the anti-BDCA2 antibody, the BDCA2×BAFF/APRIL bispecific fusion protein, or the fragment thereof is administered for a prophylactic or therapeutic purpose, previous therapies, clinical histories of patients, responses to the anti-BDCA2 antibody, the BDCA2×BAFF/APRIL bispecific fusion protein, or the fragment thereof, and the discretion of an attending physician. The anti-BDCA2 antibody, the BDCA2×BAFF/APRIL bispecific fusion protein, or the fragment thereof is suitably administered to the patient in a single treatment or over a series of treatments. The dose and treatment regimen of the anti-BDCA2 antibody, the BDCA2×BAFF/APRIL bispecific fusion protein, or the fragment thereof can be determined by those skilled.

It will be appreciated that any of the prevention or treatment described above can be performed by substituting the composition or combination product of the present disclosure for the anti-BDCA2 antibody, the BDCA2×BAFF/APRIL bispecific fusion protein, or the fragment thereof.

### X. Diagnosis and Detection

In one aspect, the present disclosure further relates to methods for diagnosis and detection (e.g., for diagnostic or non-diagnostic purposes) using an anti-BDCA2 antibody or a fragment thereof or an anti-BDCA2×BAFF/APRIL bispecific fusion protein, and a composition comprising the anti-BDCA2 antibody or the fragment thereof or the anti-BDCA2×BAFF/APRIL bispecific fusion protein for diagnosis and detection.

The term "detection" used herein includes quantitative and qualitative detections, and exemplary detections may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a body fluid, such as blood, serum, or plasma.

In certain embodiments, the anti-BDCA2 antibody or the fragment thereof or the anti-BDCA2×BAFF/APRIL bispecific fusion protein provided herein can be used to detect the presence of BDCA2 in a biological sample.

In certain embodiments, the anti-BDCA2×BAFF/APRIL bispecific fusion protein provided herein can be used to detect the presence of BAFF/APRIL in a biological sample.

In certain embodiments, the method comprises contacting a biological sample with the anti-BDCA2 antibody or the fragment thereof or the anti-BDCA2×BAFF/APRIL bispecific fusion protein described herein under conditions that allow the anti-BDCA2 antibody or the fragment thereof or the anti-BDCA2×BAFF/APRIL bispecific fusion protein to bind to BDCA2 and/or BAFF/APRIL, and detecting whether a complex is formed by the anti-BDCA2 antibody or the fragment thereof or the anti-BDCA2×BAFF/APRIL bispecific fusion protein and BDCA2 and/or BAFF/APRIL. The formation of the complex indicates the presence of BDCA2. The method may be an *in vitro* or *in vivo* method.

In certain embodiments, provided is a labeled anti-BDCA2 antibody or a fragment thereof or an anti-BDCA2×BAFF/APRIL bispecific fusion protein. The label includes, but is not limited to, a label or moiety that is detected directly (such as a fluorescent label, a chromophoric label, an electron-dense label, a chemiluminescent label, and a radioactive label), and a moiety that is detected indirectly, such as an enzyme or a ligand, for example, by enzymatic reaction or molecular interaction. In some embodiments, the label is a marker such as biotin or hFc.

In some embodiments provided herein, the sample is obtained prior to treatment with the anti-BDCA2 antibody or the fragment thereof or the anti-BDCA2×BAFF/APRIL bispecific fusion protein of the present disclosure. In some embodiments, the sample is obtained prior to application of additional therapies. In some embodiments, the sample is obtained during treatment with additional therapies, or after treatment with additional therapies.

In some embodiments, BDCA2 and/or BAFF/APRIL are detected prior to treatment, e.g., prior to an initial treatment or prior to a certain treatment after a treatment interval.

The following examples are described to facilitate the understanding of the present disclosure. The examples are not intended to be and should not be construed in any way as limiting the scope of protection of the present disclosure.

### Examples

Unless otherwise specified, the experimental methods used in the following examples are conventional chemical, biochemical, organic chemistry, molecular biology, microbiology, recombinant DNA technology, genetics, immunology, and cell biology methods within the skill of the art. The materials, reagents, and the like used in the following examples are commercially available unless otherwise specified.

### Example 1: Hybridoma Screening

### 1.1 Animal immunization

### Construction of CHO-K1/hBDCA2/FcεRIγ cell line

Sequences encoding human BDCA2 (NP_569708) and FcεRIγ (UniProt Number: P30273) were cloned into a lentivirus vector, and the lentivirus was packaged. The human BDCA2 and FcεRIγ were transduced to CHO-K1 cells (ATCC, Cat. No.: CCL-61^{™}) using the lentivirus, and the cells were then screened with 10 µg/mL puromycin (Gibco, Cat. No.: A1113802) and 600 µg/mL G418 (ThermoFisher, Cat. No.: 10131027) to obtain a cell line (CHO-K1/hBDCA2/FcεRIγ) highly expressing human BDCA2 and FcεRIγ.

### Construction of HEK293/Cyno BDCA2/FcεRIγ cell line

Sequences encoding Cyno BDCA2 (UniProt Number: A0A2K5UWP4) and FcεRIγ (UniProt Number: P30273) were cloned into a lentivirus vector, and the lentivirus was packaged. The Cyno BDCA2 and FcεRIγ were transduced to HEK293 cells (ATCC, Cat. No.: CRL-1573) using the lentivirus, and the cells were then screened with 1 µg/mL puromycin (Gibco, Cat. No.: A1113802) and 600 µg/mL G418 (ThermoFisher, Cat. No.: 10131027) to obtain a cell line (HEK293/Cyno BDCA2/FcεRIγ) highly expressing human BDCA2 and FcεRIγ.

5 BALB/c mice (Zhejiang Vital River Laboratory Animal Technology Co., Ltd.) were immunized with a human BDCA2-His protein (ACRO Biosystems, Cat. No.: CLC-H5245). The mice were immunized once every two weeks. Starting from the second immunization, 7 days after each immunization, the mice were subjected to venous blood collection, flow cytometry (FACS) determination was performed on the mouse immune serum using the HEK293/hBDCA2/FcεRIγ cell line and the HEK293/Cyno BDCA2/FcεRIγ cell line, and ELISA detection was performed on the mouse immune serum using human BDCA2-His and cynomolgus monkey BDCA2-His (ACRO Biosystems, Cat. No.: CLC-C52H4). Two mice were selected for animal fusion. Four days after the last booster immunization, spleens of the mice were taken, and lymphocytes isolated from the spleens were used for fusion to produce hybridoma cells.

### 1.2 Hybridoma screening

The culture supernatant of the hybridoma cells was subjected to a primary screening assay by ELISA and FACS detection. In this assay, 20 clones that had a binding ability for the human BDCA2-His protein and were capable of binding to the HEK293/hBDCA2/FcεRIγ cell line and the HEK293/Cyno BDCA2/FcεRIγ cell line were selected for subcloning.

### 1.3 Subcloning

20 clones selected in 1.2 were subcloned to ensure monoclonality. Subcloning was performed by reseeding the parental clones for subcloning screening using a single step cloning system. The 20 subclones were transferred to a 96-well culture plate. Subclones were screened by ELISA and FACS, and subclones in which the antibodies expressed in the cell culture supernatant were capable of binding to the human BDCA2 and cynomolgus BDCA2 proteins and binding to the HEK293/hBDCA2/FcεRIγ cell line and the HEK293/cyBDCA2/FcεRIγ cell line were screened.

### 1.4 Hybridoma sequencing

The subcloned hybridoma cell lines obtained in Example 1.3 were expanded. A part of the cells was taken and cryopreserved, and RNA was extracted from a part of the cells and reverse transcribed. The resulting cDNA was sequenced. The amino acid sequences of the variable regions of the obtained antibodies are shown in Table 1 below.

**Table 1. Amino acid sequences of variable regions of antibodies**

| Antibody name | Heavy chain variable region sequence No. | Light chain variable region sequence No. |
|---|---|---|
| 37F2C6 | SEQ ID NO: 3 | SEQ ID NO: 4 |
| 26H10A11-1 | SEQ ID NO: 1 | SEQ ID NO: 2 |

### Example 2: Construction, Expression, and Purification of Anti-BDCA2 Chimeric Antibodies 2.1 Construction of anti-BDCA2 chimeric antibodies

The amino acid sequences of the variable regions of the 2 murine antibodies in Table 1 were sent to Beijing Tsingke Biotech Co., Ltd. for codon optimization and coding gene synthesis. The genes encoding the VH regions and the VL regions of the antibodies were inserted sequentially into expression vectors containing the human IgG1 heavy chain constant region (SEQ ID NO: 5) and the kappa light chain constant region (SEQ ID NO: 6) to obtain plasmids of the murine chimeric antibodies.

### 2.2 Expression and purification of anti-BDCA2 chimeric antibodies

According to the manufacturer's product instructions, the plasmids encoding the heavy and light chains of the murine chimeric anti-BDCA2 antibodies in 2.1 were co-transfected into ExpiCHO-S cells using an ExpiCHO^{™} expression system (ThermoFisher, Cat# A29133) to express the anti-BDCA2 chimeric antibodies, and similarly to express the control antibody litifilimab analogue (heavy chain: SEQ ID NO: 7, light chain: SEQ ID NO: 8). The cells were cultured for 10-12 days after transfection. When the cell viability, as determined by a cell counter, decreased to 60%-70%, the supernatant was collected, and the antibody expressed in the supernatant was purified using a MabSelect Sure protein A affinity chromatography system (GE healthcare). The purified antibodies were concentrated and subjected to sterile filtration. The purity of the antibody protein was detected by SDS-PAGE and size exclusion chromatography. The results indicated that the purity of the antibodies was greater than 90%, and the antibodies could be used in the next step.

**Table 2. Chimeric antibody names and combinations of corresponding heavy and light chains**

| Chimeric antibody name | Heavy chain | Light chain |
|---|---|---|
| 26H10A11-1 | 26H10A11VH-IgG1 (SEQ ID NO:9) | 26H10A11VL-kappa (SEQ ID NO:10) |
| 37F2C6 | 37F2C6VH-IgG1 (SEQ ID NO:11) | 37F2C6VL-kappa (SEQ ID NO:12) |

### Example 3: Binding Affinity of Anti-BDCA2 Chimeric Antibodies for Human BDCA2 Protein

The binding affinity of the anti-BDCA2 chimeric antibodies for human BDCA2 protein (Cat# BCA-HM402, Kactus Biosystems) was detected by ForteBio Octet RED96. According to the manufacturer's instructions, an AHC sensor (ForteBio, Cat# 18-5060) was placed in a running buffer (1× PBS, Hyclone, Cat# SH30256.01, containing 0.02% Tween 20, pH 7.0) and pre-equilibrated at room temperature for 10 min. In a 96-well plate, the kinetic assay was performed according to the following steps: a) equilibrating the baseline for 100 s with the running buffer; b) diluting the anti-BDCA2 chimeric antibodies with the running buffer to 5 µg/mL, immobilizing for 200 s, and stopping; c) equilibrating the baseline for 300 s with the running buffer; and d) diluting the human BDCA2 protein with the running buffer to 50 nM, binding for 200 s, and dissociating for 600 s. The experimental data were fitted and calculated using a Fortebio Data Analysis software 1:1 binding model.

The results are shown in Table 3 below.

**Table 3. Binding affinity of anti-BDCA2 chimeric antibodies for human BDCA2 protein**

| Antibody | Antigen | Ka (M⁻¹S⁻¹) | Kd (S⁻¹) | KD (M) |
|---|---|---|---|---|
| 26H10A11-1 | Human BDCA2-His | 1.23E+05 | 6.16E-05 | 5.03E-10 |
| 37F2C6 | Human BDCA2-His | 1.61E+05 | <1.0E-07 | <1.0E-12 |

### Example 4: Binding of Anti-BDCA2 Chimeric Antibodies to Cell Line Expressing Human BDCA2 Protein

### 4.1. Construction of engineered cells expressing human BDCA2 protein

The sequence encoding human BDCA2 (NP_569708) and the FcεRIγ sequence (UniProt Number: P30273) were cloned into a lentivirus vector, and the lentivirus was packaged. The human BDCA2 and FcεRIγ were transfected into HEK293 cells (ATCC, Cat. No.: CRL-1573) using the lentivirus, and the cells were then screened with 1 µg/mL puromycin (Gibco, Cat. No.: A1113802) and 600 µg/mL G418 (ThermoFisher, Cat. No.: 10131027) to obtain a cell line (HEK293/hBDCA2/FcεRIγ) highly expressing human BDCA2 and FcεRIγ.

### 4.2. Binding of BDCA2 antibodies to HEK293/hBDCA2/FcεRIγ engineered cells

The HEK293/hBDCA2/FcεRIγ cell line was subjected to enzymatic digestion to obtain a single-cell suspension. The single-cell suspension was centrifuged at room temperature at 300 g for 4 min, and then the medium was discarded. The resulting cell pellet was washed once with PBS. The cells were resuspended in a PBS gradient dilution solution (at an initial concentration of 100 nM, 5-fold gradient dilution, 8 concentration points in total) of the anti-BDCA2 chimeric antibodies, and the cells were incubated at 4 °C for 30 min. The cells were washed once with PBS, and then fluorescent secondary antibody R-PE-conjugated AffiniPure Goat Anti-Human IgG, Fcγ Fragment Specific (Jackson ImmunoResearch, Cat. No.: 109-116-098) diluted in a 1:200 ratio was added. The cells were incubated in the dark at 4 °C for 30 min. The cells were washed twice with PBS and then resuspended, and finally, the fluorescence intensity of PE was measured using a flow cytometer.

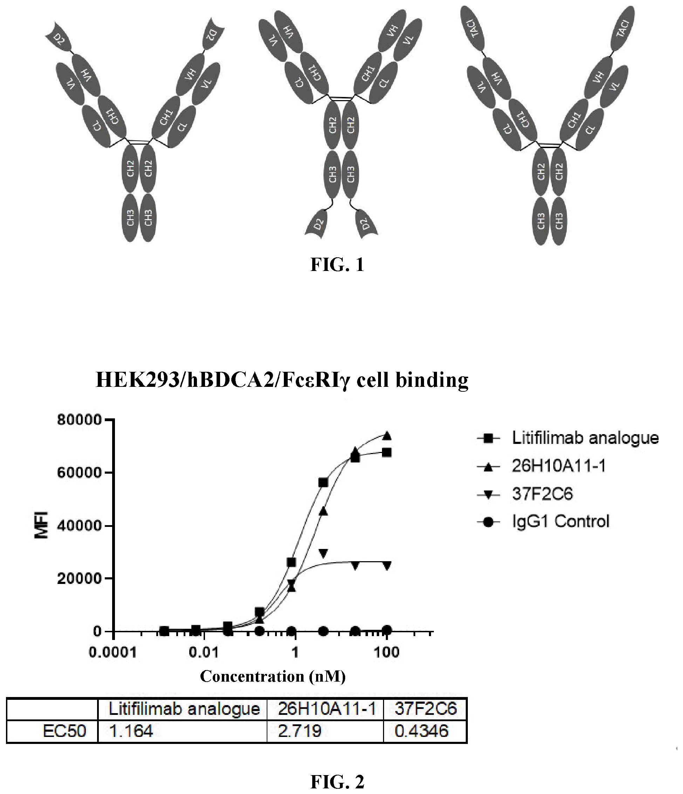

As can be seen from the results shown in FIG. 2, the anti-BDCA2 chimeric antibodies 37F2C6 and 26H10A11-1 can both bind to the cell line expressing the human BDCA2 protein.

### Example 5: Killing of Cell Line Expressing Human BDCA2 Protein by Anti-BDCA2 Chimeric Antibodies

Cryopreserved PBMCs were taken out from a liquid nitrogen tank and rapidly thawed in a water bath at 37 °C. The reconstituted cells were transferred to a 15 mL centrifuge tube, and 9 mL of an RPMI-1640 complete medium was added. The mixture was mixed well and centrifuged at 350 g for 10 min. After NK cells were sorted from the PBMCs according to the NK cell sorting kit (Stemcell, Cat. No.: 17955), the sorted NK cells were resuspended in an RPMI-1640 complete medium containing 200 IU/mL IL-2 and activated overnight.

The HEK293/hBDCA2/FcεRIγ cell line was subjected to enzymatic digestion to obtain a single-cell suspension. The single-cell suspension was centrifuged at room temperature at 300 g for 4 min, and then the medium was discarded. The resulting cell pellet was washed once with PBS. The cells were stained and labeled with the CellTrace^{™} Violet Cell Proliferation Kit (Invitrogen, Cat. No.: C34557) according to the instructions. The anti-BDCA2 chimeric antibodies were subjected to gradient dilution (at an initial concentration of 100 nM, 5-fold gradient dilution, 8 concentration points in total), and the anti-BDCA2 chimeric antibodies were mixed well with NK cells of the generation activated overnight by IL-2. Then the stained and labeled HEK293/hBDCA2/FcεRIγ cells were added to the well where the anti-BDCA2 chimeric antibodies and NK cells were well mixed in an effector-to-target ratio of 3:1. After the cells were incubated in a carbon dioxide incubator for 4 h, a 1:500 diluent of propidium iodide (Beyotime, Cat. No.: ST511) dye was added, and finally, the percentage of PI-positive cells among the stained and labeled target cells was determined using a flow cytometer.

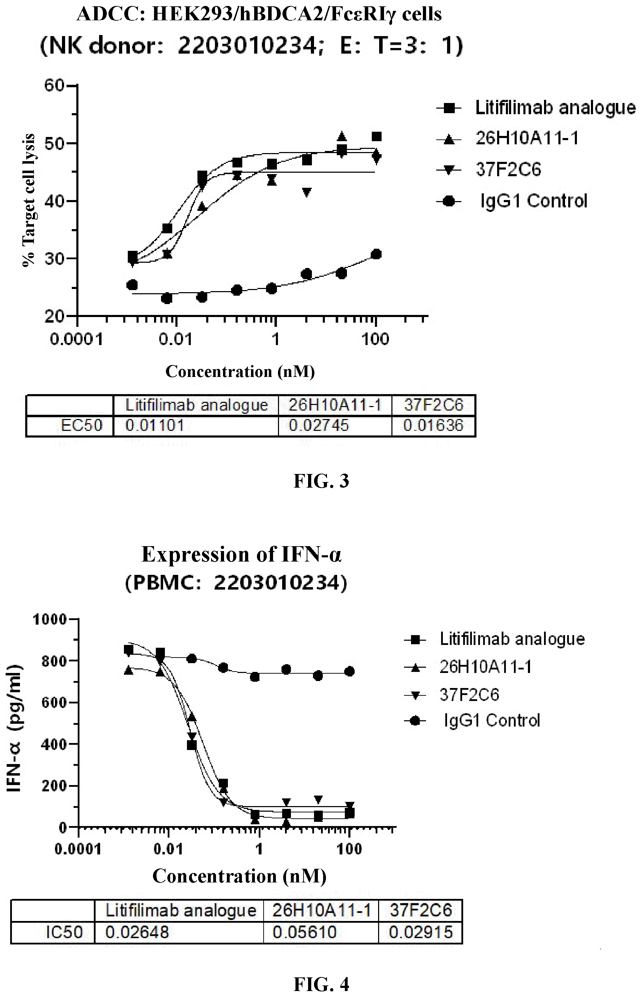

As can be seen from the results shown in FIG. 3, the anti-BDCA-2 chimeric antibodies had ADCC effects and could induce primary NK cells to kill the engineered cells expressing BDCA2. The killing activity against the target cells HEK293/hBDCA2/FcεRIγ of the chimeric antibodies 37F2C6 and 26H10A11-1 was comparable to that of the litifilimab analogue.

### Example 6: Inhibition of Production of IFN-α in TLR9 Agonist-Stimulated PBMCs by Anti-BDCA2 Chimeric Antibodies

Whether the anti-BDCA2 chimeric antibodies in the present disclosure could inhibit TLR9 agonist-induced IFN-α production in human PBMCs was determined by the IFN-α factor inhibition experiment.

Cryopreserved PBMCs were taken out from a liquid nitrogen tank and rapidly thawed in a water bath at 37 °C. The reconstituted cells were transferred to a 15 mL centrifuge tube, and 9 mL of a 1640 complete medium was added. The mixture was mixed well and centrifuged at 350 g for 10 min, and the cells were resuspended in a fresh medium and then counted. PBMCs were added to a 96-well plate at 3 × 10^5 cells/well, and the anti-BDCA2 chimeric antibodies were subjected to gradient dilution (at an initial concentration of 100 nM, 5-fold gradient dilution, 8 concentration points in total). The gradient-diluted antibody and the PBMCs were mixed well and then pre-incubated in a carbon dioxide incubator for 30 min. A TLR9 agonist (InvivoGen, Cat. No.: tlrl-2216) at a final concentration of 1 µM was added, and the mixture was incubated in the carbon dioxide incubator for 24 h. After 24 h, the supernatant was collected, and the content of IFN-α in the supernatant was measured using an IFN-α HTRF detection kit (Cisbio, Cat. No.: 62HIFNAPEG).

As can be seen from the results shown in FIG. 4, the anti-BDCA-2 chimeric antibodies were able to inhibit the production of IFN-α by human PBMCs induced by TLR9 agonists, and the inhibitory effects on IFN-α of the chimeric antibodies 37F2C6 and 26H10A11-1 clones were comparable to that of the litifilimab analogue.

### Example 7: Humanization and Expression of Murine Anti-BDCA2 Antibody 7.1 Humanization of murine anti-BDCA2 antibody

The murine antibody 37F2C6 was humanized using CDR grafting technology. The sequences of 37F2C6 were searched and aligned in the IMGT database, separately, thereby obtaining a human germline gene sequence IGHV1-3*04 with high homology to the 37F2C6 heavy chain variable region for use as a humanized framework of the heavy chain variable region, and selecting a human germline gene sequence IGKV3-11*01 with high homology to the light chain variable region for use as a humanized framework of the light chain variable region. The CDRs of the 37F2C6 heavy and light chain variable regions were grafted into the corresponding humanized frameworks to form humanized 37F2C6 antibody variable regions. In order to maintain the affinity of antibody 37F2C6, the humanized antibody variable regions obtained were back-mutated.

Therefore, the sequences of the humanized heavy chain variable region and the humanized light chain variable region were obtained. The amino acid sequences of the variable regions of the 37F2C6 humanized antibodies are listed in Table 4. The variable region amino acid sequences were sent to Beijing Tsingke Biotech Co., Ltd. for codon optimization and gene synthesis. The genes encoding the VH regions and the VL regions of the antibodies were inserted into an expression vector comprising a coding gene of a human IgG1 heavy chain constant region (SEQ ID NO: 5) and a coding gene of a kappa light chain constant region (SEQ ID NO: 6), separately, to obtain plasmids expressing the anti-BDCA2 humanized antibody full-length heavy chains and plasmids encoding the full-length light chains. Names, variable region sequences, and heavy and light chain sequences of each antibody are listed in Table 4.

**Table 4. Humanized antibody names and corresponding sequences**

| Antibody name | Heavy chain | Light chain | Heavy chain variable region | Light chain variable region |
|---|---|---|---|---|
| Hu37F2C6-8 | SEQ ID NO:16 | SEQ ID NO:18 | SEQ ID NO:13 | SEQ ID NO:15 |
| Hu37F2C6-9 | SEQ ID NO:17 | SEQ ID NO:18 | SEQ ID NO:14 | SEQ ID NO:15 |

### 7.2 Expression and purification of humanized anti-BDCA2 antibodies

The plasmids encoding the heavy and light chains were co-transfected into ExpiCHO-S cells in combinations as described in Table 4 to express anti-BDCA2 humanized antibodies, and the fucose inhibitor (Sigma, Cat. No.: 344827-10 mg) was added or not added during transfection to obtain wild-type or ADCC-enhanced antibodies. The antibodies obtained with the addition of the fucose inhibitor were designated as: Hu37F2C6-8-A and Hu37F2C6-9-A, respectively, and were purified accordingly as described in Example 2.2. The purified antibodies were concentrated, and sterile filtration was performed. The purity of the antibodies was measured by SDS-PAGE and size exclusion chromatography (SEC).

### 7.3 Physicochemical analysis of humanized anti-BDCA2 antibodies

For the humanized anti-BDCA2 antibodies obtained, the purity of the antibodies was determined by size exclusion chromatography. Specifically, 20 µg of the antibody sample was injected onto a TSK G3000SWXL column using 100 mM sodium phosphate + 100 mM Na₂SO₄ (pH 7.0) as a running buffer for 30 min. The collected effluent was measured using the Agilent 1220 HPLC system, and the data were analyzed using OpenLAB software. The results indicated that the purity of the antibodies was more than 90%, and the antibodies could be used in the next step.

### Example 8: Binding Affinity of Anti-BDCA2 Humanized Antibodies for Human BDCA2 Protein

In this experiment, according to the manufacturer's instructions, the binding affinity of antibodies 37F2C6, Hu37F2C6-8, and Hu37F2C6-9 for the human BDCA2 protein (KACTUS, Cat. No.: BCA-HM402) was measured using ForteBio Octet RED96e.

Briefly, an AHC sensor (ForteBio, Cat. No.: 18-5060) was pre-equilibrated in a running buffer (1× PBS, XiGene, Cat. No.: XG3650, containing 0.02% Tween 20, 0.1% BSA, pH 7.0) at room temperature for 10 min. In a 96-well plate, the BDCA2 kinetic assay was performed according to the following steps: a) equilibrating the baseline with the running buffer for 180 s; b) adding the antibodies diluted with the running buffer at a final concentration of 5 µg/mL, and immobilizing for 200 s; c) equilibrating the baseline with the running buffer for 180 s; d) adding the human BDCA2 protein diluted with the running buffer at the following concentration of 100 nM to each well, binding for 200 s, and dissociating for 180 s; and e) applying a regeneration solution (0.01 M Gly-HCl, pH 1.5) for 30 s. The experimental data were fitted and calculated using a 1:1 binding model in the Fortebio Data Analysis software. The results are shown in the table below.

**Table 5. Binding affinity of anti-BDCA2 humanized antibodies for human BDCA2 protein**

| Antibody | Antigen | KD (M) | Kon (1/Ms) | Kdis (1/s) |
|---|---|---|---|---|
| 37F2C6 | Human BDCA2 protein | <1.0E-12 | 1.41E+05 | <1.0E-07 |
| Hu37F2C6-8 | | <1.0E-12 | 1.13E+05 | <1.0E-07 |
| Hu37F2C6-9 | | <1.0E-12 | 1.21E+05 | <1.0E-07 |

Table 5 shows that the antibodies 37F2C6, Hu37F2C6-8, and Hu37F2C6-9 of the present disclosure bound to the human BDCA2 protein with high affinity.

### Example 9: Killing of Cells Expressing Human BDCA2 Protein by Anti-BDCA2 Humanized Antibodies

Two experiments, namely killing of HEK293/hBDCA2/FcεRIγ cells by primary NK cells and killing of pDCs by autologous NK cells in a PBMC system, were performed to determine whether the anti-BDCA2 humanized antibodies in the present disclosure can kill HEK293/hBDCA2/FcεRIγ cells and pDCs.

### 9.1 Experimental system for killing HEK293/hBDCA2/FcεRIγ cells by primary NK cells

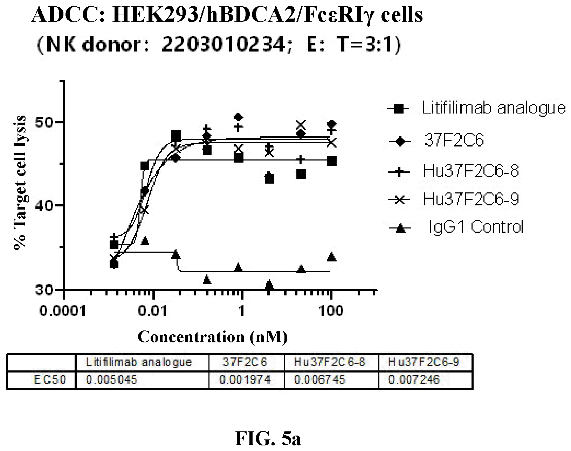

The experimental procedures were the same as those in Example 5. As can be seen from the results shown in FIG. 5a, the killing activity against the target cells HEK293/hBDCA2/FcεRIγ of the chimeric antibody 37F2C6 and its humanized antibodies Hu37F2C6-8/Hu37F2C6-9 was comparable to that of the litifilimab analogue.

### 9.2 PBMC ADCC experimental system

Cryopreserved PBMCs were taken out from a liquid nitrogen tank and rapidly thawed in a water bath at 37 °C. The reconstituted cells were transferred to a 15 mL centrifuge tube, and 9 mL of a 1640 complete medium was added. The mixture was mixed well and centrifuged at 350 g for 10 min, and the cells were resuspended in a 1640 complete medium containing 200 IU/mL IL-2 and activated overnight. PBMC cells activated by IL-2 overnight were collected, resuspended, and counted. The cells were added to a 96-well plate at 3 × 10^5 cells/well, and the BDCA2 antibody was subjected to gradient dilution (at an initial concentration of 100 nM, 5-fold gradient dilution, 8 concentration points in total). The gradient-diluted antibody and the PBMCs were mixed well, and the mixture was incubated in a carbon dioxide incubator for 48 h. After 48 h, the cells were collected, and the number of pDCs in PBMCs was measured by using a flow cytometer. The flow cytometry for pDCs was performed as follows: The Zombie Violet^{™} Fixable Viability Kit (Biolegend, Cat. No.: 423114) was used to label live PBMC cells with a live/dead dye, and the cells were labeled with APC anti-human Lineage Cocktail (CD3, CD14, CD19, CD20, CD56) (Biolegend, Cat. No.: 348703) to exclude T cells, NK cells, monocytes, and B cells. Then, in the HLA-DR+ (Biolegend, Cat. No.: 307644)/CD11c- (Biolegend, Cat. No.: 301608) cell population, CD123+ (Biolegend, Cat. No.: 306014)/ILT7+ (Biolegend, Cat. No.: 326408) cells were considered as pDCs.

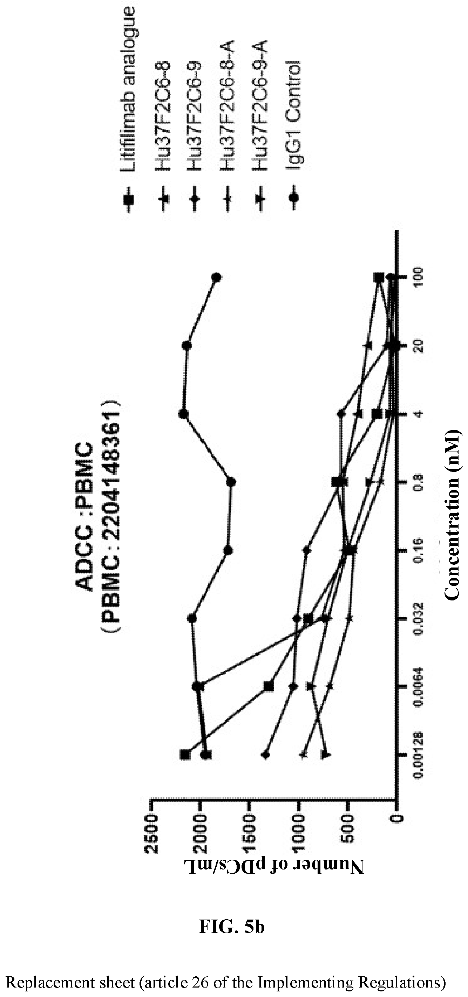

As can be seen from the results shown in FIG. 5b, the killing activity against pDCs of the humanized antibodies Hu37F2C6-8/Hu37F2C6-9 was comparable to that of the litifilimab analogue, and the killing activity against pDCs of Hu37F2C6-8-A/Hu37F2C6-9-A was superior to that of the control antibody litifilimab analogue.

### Example 10: Inhibition of Production of IFN-α in TLR9 Agonist-Stimulated PBMCs by Anti-BDCA2 Humanized Antibodies

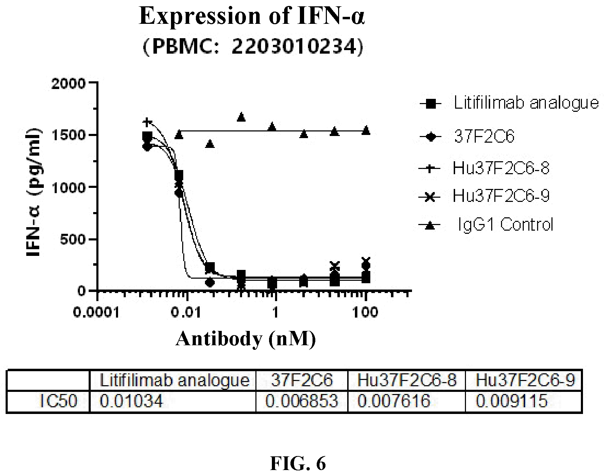

The experimental procedures were the same as those in Example 6. As can be seen from the results shown in FIG. 6, the inhibitory effects on IFN-α of the chimeric antibody 37F2C6 clone and its humanized antibodies Hu37F2C6-8/Hu37F2C6-9 were comparable to that of the litifilimab analogue.

### Example 11: Effect of Anti-BDCA2 Humanized Antibodies in Eliminating pDCs in huHSC-NCG Mouse Model

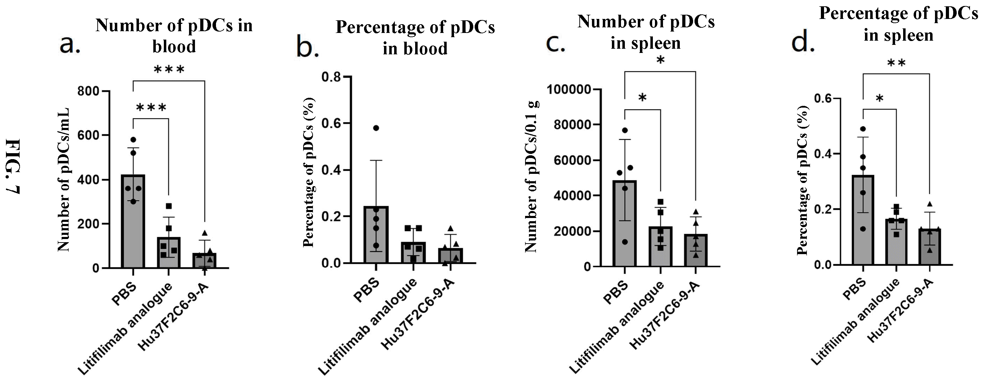

The *in vivo* efficacy of the BDCA2 humanized antibody in eliminating pDCs was studied in a huHSC-NCG mouse model. The huHSC-NCG mice were purchased from Gempharmatech Co., Ltd. After a one-week adaptation period, 15 huHSC-NCG mice were randomly divided into 3 experimental groups, 5 mice per group. On the day of grouping (day 0), the blank control group (PBS) (G1), litifilimab analogue (30 mg/kg) (G2), and Hu37F2C6-9-A (30 mg/kg) (G3) were administered intraperitoneally to the mice, respectively. On day 3, peripheral blood and spleens of the mice in each group were collected for pDCs detection. As shown in FIG. 7, the anti-BDCA2 humanized antibody group can exhibit an extremely significant reduction in the proportion of pDCs in the peripheral blood and an extremely significant reduction in the proportion and number of pDCs in the spleen, as compared to the PBS group.

### Example 12: Anti-BDCA2/D2 and Anti-BDCA2/TACI Bispecific Fusion Proteins

### 12.1 Construction of bispecific fusion proteins

In the present disclosure, three bispecific fusion proteins with different symmetric structures were constructed as shown in FIG. 1. The anti-BDCA2 portion of each bispecific fusion protein was derived from the humanized 37F2C6-9 antibody described above. The TACI portion (TACI for short) is a fragment of full-length TACI, which is the same as the truncated portion of the telitacicept antibody, i.e., the underlined portion of the corresponding sequence. The CRD2 portion (D2 for short) is a further truncated segment on the basis of the amino acid sequence of TACI, i.e., the underlined, bolded, and italicized sequence as follows. The amino acid sequence of the control antibody telitacicept is as follows:

SRVDQEERFPQGLWTGVAMRSCPEEQYWDPLLGTCMSCKTICNHQSQRTCAAFCRSLSCR
KEQGKFYDHLLRDCISCASICGQHPKQCAYFCENKLRSPVNLPPELDKTHTCPPCPAPEAE
GAPSVFLFPPKPKDTLMISRTPEVTCVVVDVSHEDPEVKFNWYVDGVEVHNAKTKPREEQ

YNSTYRVVSVLTVLHQDWLNGKEYKCKVSNKALPSSIEKTISKAKGQPREPQVYTLPPSRD
ELTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO:19)

The amino acid sequences of CRD2 and TACI were sent to Nanjing Tsingke Biotech Co., Ltd. for codon optimization and gene synthesis to obtain nucleic acids containing the coding sequences of CRD2 and TACI for use as templates for subsequent bispecific antibody construction.

In the present application, 3 types of bispecific fusion proteins targeting BDCA2/CRD2 or BDCA2/TACI with the structures shown in FIG. 1 were obtained using standard construction methods, and their amino acid sequence numbers are listed in Table 6 below. The heavy chain of the bispecific fusion protein was fused with a CRD2 sequence at the N-terminus or C-terminus of the humanized 37F2C6-9 antibody by the overlapping PCR technique, or fused with a TACI sequence at the N-terminus of the humanized 37F2C6-9 antibody by the overlapping PCR technique.

The heavy chains of each bispecific fusion protein molecule were constructed as follows:
37F2C6-HuVH3-D2 (SEQ ID NO: 22): With the synthesized CRD2 as a template, a nucleotide sequence encoding the CRD2 was amplified, and the sequence was spliced with the C-terminus of the nucleotide sequence encoding the complete Hu37F2C6-9 using the overlapping PCR technique (the linker peptide between the two sequences was: (G₄S)₃). The spliced nucleotide sequence was cloned into an expression vector for expression to obtain the 37F2C6-HuVH3-D2 molecule.

37F2C6-HuVH3-3N-D2 (SEQ ID NO: 23): With the synthesized CRD2 as a template, a nucleotide sequence encoding the CRD2 was amplified, and the sequence was spliced with the N-terminus of the nucleotide sequence encoding the complete Hu37F2C6-9 using the overlapping PCR technique (the linker peptide between the two sequences was: (G₄S)₃). The spliced nucleotide sequence was cloned into an expression vector for expression to obtain the 37F2C6-HuVH3-3N-D2 molecule.

37F2C6-HuVH3-3N-TACI (SEQ ID NO: 24): With the synthesized TACI as a template, a nucleotide sequence encoding the TACI was amplified, and the sequence was spliced with the N-terminus of the nucleotide sequence encoding the complete Hu37F2C6-9 using the overlapping PCR technique (the linker peptide between the two sequences was: (G₄S)₃). The spliced nucleotide sequence was cloned into an expression vector for expression to obtain the 37F2C6-HuVH3-3N-TACI molecule. 37F2C6-HuVH3-CD2-DE (SEQ ID NO: 50): With the synthesized CRD2 as a template, a nucleotide sequence encoding the CRD2 was amplified (the N-terminus of the amino acid sequence of CRD2 was truncated, and SLS was removed), and the sequence was spliced with the C-terminus of the nucleotide sequence encoding the Hu37F2C6-9 using the overlapping PCR technique (the linker peptide between the two sequences was: (G₄S)₃). The spliced nucleotide sequence was cloned into an expression vector for expression to obtain the 37F2C6-HuVH3-CD2-DE molecule.

37F2C6-HuVH3-ND2-DE (SEQ ID NO: 51): With the synthesized CRD2 as a template, a nucleotide sequence encoding the CRD2 was amplified (the C-terminus of the amino acid sequence of CRD2 was truncated, and EN was removed), and the sequence was spliced with the N-terminus of the nucleotide sequence encoding the complete Hu37F2C6-9 using the overlapping PCR technique (the linker peptide between the two sequences was: (G₄S)₃). The spliced nucleotide sequence was cloned into an expression vector for expression to obtain the 37F2C6-HuVH3-ND2-DE molecule.

### 12.2 Construction of different linker peptides (linkers) of bispecific fusion proteins

Taking the bispecific fusion protein heavy chain molecule 37F2C6-HuVH3-3N-D2 as an example, the linker peptide was changed from the original (G₄S)₃ to (GS₄)₂, (GS₄)₃, and (GS₄)₄, separately, and then co-transfected with the light chain 37F2C6-HuVL3 to obtain bispecific fusion proteins BI-9ND2-2GS4, BI-9ND2-3GS4, and BI-9ND2-4GS4, respectively.

Similarly, the linker peptide in the 37F2C6-HuVH3-D2 was changed from the original (G₄S)₃ to GS₄, (GS₄)₂, and (GS₄)₃, separately, and then co-transfected with the light chain 37F2C6-HuVL3 to obtain bispecific fusion proteins BI-9D2-1GS4, BI-9D2-2GS4, and BI-9D2-3GS4, respectively. Bispecific fusion proteins BI-9D2-3G4S4S and BI-9D2-3G4S-8S were obtained by adding SSSS and SSSSSSSS, respectively, at the C-terminus of CRD2 of 37F2C6-HuVH3-D2 while keeping the linker peptide therein unchanged (linker peptide: (G₄S)₃) and then performing co-transfection together with the light chain 37F2C6-HuVL3.

### 12.3 Construction of bispecific fusion protein having YTE mutations in Fc region

37F2C6-HuVH3-ND2-YTE (SEQ ID NO: 53): The constructed 37F2C6-HuVH3-3N-D2 was used as a template, point mutation was performed, and mutations were performed at M252Y, S254T, and T256E (according to the EU numbering) using the overlapping PCR technique. The spliced nucleotide sequence was cloned into an expression vector for expression to obtain the 37F2C6-HuVH3-ND2-YTE molecule.

The bispecific fusion proteins in FIG. 1 were obtained by accordingly combining the expression vectors described above according to the specific compositions shown in Table 6 and expressing them under appropriate conditions.

The construction, expression, purification, and preliminary analysis steps of the bispecific fusion proteins were the same as those in Example 7, and the antibodies obtained with the addition of the fucose inhibitor were designated as: BI-9D2-A, BI-9ND2-A, BI-9NTACI-A, BI-9CD2-DE-A, BI-9ND2-DE-A, and BI-9ND2-A-YTE, respectively.

**Table 6. Expression of humanized bispecific fusion proteins**

| Name of bispecific fusion protein | Heavy chain | Light chain |
|---|---|---|
| BI-9D2, BI-9D2-A | SEQ ID NO: 22 | SEQ ID NO: 18 |
| BI-9ND2, BI-9ND2-A | SEQ ID NO: 23 | SEQ ID NO: 18 |
| BI-9NTACI, BI-9NTACI-A | SEQ ID NO: 24 | SEQ ID NO: 18 |
| BI-9ND2-2GS4 | SEQ ID NO: 25 | SEQ ID NO: 18 |
| BI-9ND2-3GS4 | SEQ ID NO: 26 | SEQ ID NO: 18 |
| BI-9ND2-4GS4 | SEQ ID NO: 27 | SEQ ID NO: 18 |
| BI-9D2-1GS4 | SEQ ID NO: 28 | SEQ ID NO: 18 |
| BI-9D2-2GS4 | SEQ ID NO: 29 | SEQ ID NO: 18 |
| BI-9D2-3GS4 | SEQ ID NO: 30 | SEQ ID NO: 18 |
| BI-9D2- 3G4S -4S | SEQ ID NO: 31 | SEQ ID NO: 18 |
| BI-9D2- 3G4S -8S | SEQ ID NO: 32 | SEQ ID NO: 18 |
| BI-9CD2-DE, BI-9CD2-DE-A | SEQ ID NO: 50 | SEQ ID NO: 52 |
| BI-9ND2-DE, BI-9ND2-DE-A | SEQ ID NO: 51 | SEQ ID NO: 52 |
| BI-9ND2-A-YTE | SEQ ID NO: 53 | SEQ ID NO: 18 |

### Example 13: Binding Affinity of Bispecific Fusion Proteins, Bispecific Fusion Proteins Having Different Linkers, and Bispecific Fusion Protein Having YTE Mutations in Fc Region for Human BDCA2 Protein

13.1 In this experiment, according to the manufacturer's instructions, the binding affinity of the bispecific fusion proteins for human BDCA-2 protein (KACTUS, Cat. No.: BCA-HM402) was measured using ForteBio Octet RED96e.

Briefly, an AHC sensor (ForteBio, Cat. No.: 18-5060) was pre-equilibrated in a running buffer (1× PBS, XiGene, Cat. No.: XG3650, containing 0.02% Tween 20, 0.1% BSA, pH 7.0) at room temperature for 10 min. In a 96-well plate, the BDCA2 kinetic assay was performed according to the following steps: a) equilibrating the baseline with the running buffer for 180 s; b) adding each bispecific fusion protein diluted with the running buffer at a final concentration of 5 µg/mL, and immobilizing for 200 s; c) equilibrating the baseline with the running buffer for 180 s; d) adding the human BDCA2 protein diluted with the running buffer at the following concentrations to each well: 100 nM, 50 nM, 25 nM, 12.5 nM, 6.25 nM, 3.13 nM, and 1.56 nM, binding for 200 s, and dissociating for 180 s; and e) applying a regeneration solution (0.01 M Gly-HCl, pH 1.5) for 30 s. The experimental data were fitted and calculated using a 1:1 binding model in the Fortebio Data Analysis software. The results are shown in the table below.

**Table 7. Binding affinity of bispecific fusion proteins and control for human BDCA2 protein**

| Antigen | Antibody | KD (M) | Kon (1/Ms) | kdis (1/s) |
|---|---|---|---|---|
| Human BDCA2 | BI-9D2-A | <1.0E-12 | 8.88E+04 | <1.0E-07 |
| | BI-9ND2-A | <1.0E-12 | 8.66E+04 | <1.0E-07 |
| | BI-9NTACI-A | <1.0E-12 | 8.54E+04 | <1.0E-07 |

Table 7 shows that the bispecific fusion proteins of the present disclosure bound to the human BDCA2 protein with high affinity.

13.2 The binding affinity of the bispecific fusion proteins having different linker peptides for the human BDCA2 protein was measured using ForteBio Octet RED96e. The procedures were the same as those in Example 8. The results are shown in the table below.

**Table 8. Binding affinity of bispecific fusion proteins having different linker peptides for human BDCA2 protein**

| Antibody | Antigen | Response(n m) | KD (M) | Kon (1/Ms) | Kdis (1/s) |
|---|---|---|---|---|---|
| BI-9ND2-2GS4 | Human BDCA2 protein | 0.4662 | <1.0E-12 | 1.15E+05 | <1.0E-07 |
| BI-9ND2-3GS4 | | 0.4734 | <1.0E-12 | 1.17E+05 | <1.0E-07 |
| BI-9ND2-4GS4 | | 0.4842 | <1.0E-12 | 1.25E+05 | <1.0E-07 |
| BI-9D2-1GS4 | | 0.5912 | <1.0E-12 | 1.49E+05 | <1.0E-07 |
| BI-9D2-2GS4 | | 0.5599 | <1.0E-12 | 1.51E+05 | <1.0E-07 |
| BI-9D2-3GS4 | | 0.5512 | 2.34E-10 | 1.53E+05 | 3.58E-05 |
| BI-9D2- 3G4S -4S | | 0.4569 | <1.0E-12 | 1.55E+05 | <1.0E-07 |
| BI-9D2- 3G4S -8S | | 0.4756 | <1.0E-12 | 1.54E+05 | <1.0E-07 |
| BI-9D2-A | | 0.481 | <1.0E-12 | 1.54E+05 | <1.0E-07 |
| BI-9ND2-A | | 0.4488 | <1.0E-12 | 1.12E+05 | <1.0E-07 |
| Litifilimab analogue | | 0.4444 | <1.0E-12 | 1.72E+05 | <1.0E-07 |
| Telitacicept | | -0.0175 | 3.74E+2 0 | 9.56E+04 | 3.57E+25 |

Table 8 shows that the bispecific fusion proteins having different linker peptides of the present disclosure bound to the human BDCA2 protein with high affinity.

13.3 The binding affinity of the bispecific fusion protein having YTE mutations in the Fc region for the human BDCA-2 protein was measured using ForteBio Octet RED96e. The procedures were the same as those in Example 13.1.

**Table 9. Binding affinity of bispecific fusion protein having YTE mutations in Fc region for human BDCA2 protein**

| Antigen | Antibody | KD (M) | Kon (1/Ms) | kdis (1/s) |
|---|---|---|---|---|
| Human BDCA2 protein | BI-9ND2-A-YTE | <1.0E-12 | 1.00E+05 | <1.0E-07 |

Table 9 shows that the bispecific fusion protein having YTE mutations in the Fc region of the present disclosure bound to the human BDCA2 protein with high affinity.

### Example 14: Binding Affinity of Bispecific Fusion Proteins, Bispecific Fusion Proteins Having Different Linkers, and Bispecific Fusion Protein Having YTE Mutations in Fc Region for Human BAFF/APRIL Protein

14.1 In this experiment, according to the manufacturer's instructions, the binding affinity of the bispecific fusion proteins for the human BAFF protein (ACRO Biosystems, Cat. No.: BAF-H52D4) and the human APRIL protein (ACRO Biosystems, Cat. No.: APL-H52D1) was measured using ForteBio Octet RED96e. The procedures were the same as those in Example 13.1. The results are shown in the table below.

**Table 10. Binding affinity of bispecific fusion proteins and control for human BAFF protein and human APRIL protein**

| Antigen | Antibody | KD (M) | Kon (1/Ms) | kdis (1/s) |
|---|---|---|---|---|
| Human BAFF | BI-9D2-A | <1.0E-12 | 5.71E+05 | <1.0E-07 |
| | BI-9ND2-A | 1.47E-10 | 4.92E+05 | 7.23E-05 |
| | BI-9NTACI-A | 9.51E-10 | 3.60E+05 | 3.42E-04 |
| | Telitacicept | 1.47E-09 | 3.23E+05 | 4.75E-04 |
| Human APRIL | BI-9D2-A | 6.79E-10 | 8.68E+05 | 5.90E-04 |
| | BI-9ND2-A | 4.15E-10 | 7.74E+05 | 3.21E-04 |
| | BI-9NTACI-A | 6.19E-10 | 7.72E+05 | 4.78E-04 |
| | Telitacicept | 2.05E-09 | 6.78E+05 | 1.39E-03 |

Table 10 shows that the bispecific fusion proteins of the present disclosure were capable of specifically binding to the human BAFF protein and the human APRIL protein.

14.2 The binding affinity of the bispecific fusion proteins having different linker peptides for the human BAFF and APRIL proteins was measured using ForteBio Octet RED96e. The procedures were the same as those in Example 8. The results are shown in the table below.

**Table 11. Binding affinity of bispecific fusion proteins having different linker peptides for human BAFF protein**

| Antigen | Antibody | Response( nm) | K_{D} (M) | Kon (1/Ms) | Kdis (1/s) |
|---|---|---|---|---|---|
| | BI-9ND2-2GS4 | 0.4922 | <1.0E-12 | 4.56E+05 | <1.0E-07 |
| | BI-9ND2-3GS4 | 0.4874 | <1.0E-12 | 4.89E+05 | <1.0E-07 |
| | BI-9ND2-4GS4 | 0.468 | <1.0E-12 | 5.22E+05 | <1.0E-07 |
| | BI-9D2-1GS4 | 0.2988 | <1.0E-12 | 4.00E+05 | <1.0E-07 |
| | BI-9D2-2GS4 | 0.3018 | <1.0E-12 | 4.12E+05 | <1.0E-07 |
| | BI-9D2-3GS4 | 0.3403 | <1.0E-12 | 4.22E+05 | <1.0E-07 |
| Human BAFF | BI-9D2- 3G4S - 4S | 0.3576 | <1.0E-12 | 3.82E+05 | <1.0E-07 |
| | BI-9D2- 3G4S - 8S | 0.3939 | <1.0E-12 | 4.02E+05 | <1.0E-07 |
| | BI-9D2-A | 0.3378 | <1.0E-12 | 4.07E+05 | <1.0E-07 |
| | BI-9ND2-A | 0.4902 | <1.0E-12 | 4.38E+05 | <1.0E-07 |
| | Litifilimab analogue | 0.0284 | <1.0E-12 | 3.00E+05 | <1.0E-07 |
| | Telitacicept | 0.3191 | 1.10E-09 | 2.39E+05 | 2.62E-04 |

**Table 12. Binding affinity of bispecific fusion proteins having different linker peptides for human APRIL protein**

| Antigen | Antibody | Response( nm) | K_{D} (M) | Kon (1/Ms) | Kdis (1/s) |
|---|---|---|---|---|---|
| | BI-9ND2-2GS4 | 0.3393 | 2.75E-10 | 9.37E+05 | 2.57E-04 |
| | BI-9ND2-3GS4 | 0.3267 | 9.44E-11 | 1.06E+06 | 9.99E-05 |
| | BI-9ND2-4GS4 | 0.2983 | 6.67E-11 | 1.17E+06 | 7.79E-05 |
| | BI-9D2-1GS4 | 0.2611 | 7.24E-10 | 8.13E+05 | 5.89E-04 |
| | BI-9D2-2GS4 | 0.2425 | 3.91E-10 | 8.66E+05 | 3.39E-04 |
| | BI-9D2-3GS4 | 0.2508 | 6.16E-10 | 9.44E+05 | 5.82E-04 |
| Human APRIL | BI-9D2-3G4S - 4S | 0.285 | 5.28E-10 | 8.24E+05 | 4.35E-04 |
| | BI-9D2-3G4S - 8S | 0.2903 | 1.66E-10 | 8.08E+05 | 1.34E-04 |
| | BI-9D2-A | 0.252 | 2.46E-10 | 8.73E+05 | 2.14E-04 |
| | BI-9ND2-A | 0.397 | <1.0E-12 | 7.22E+05 | <1.0E-07 |
| | Litifilimab analogue | 0.2961 | 6.50E-09 | 9.90E+04 | 6.43E-04 |
| | Telitacicept | 0.1993 | 2.08E-09 | 6.81E+05 | 1.41E-03 |

The affinity of the bispecific fusion proteins having different linker peptides for being capable of specifically binding to the human BAFF protein and the human APRIL protein is summarized in Tables 11 and 12.

14.3 The binding affinity of the bispecific fusion protein having YTE mutations in the Fc region for human BAFF protein (BAF-H52D4) and human APRIL protein was measured using ForteBio Octet RED96e. The procedures were the same as those in Example 13.1.

**Table 13. Binding affinity of bispecific fusion protein and control for human BAFF protein and human APRIL protein**

| Antigen | Antibody | KD (M) | Kon (1/Ms) | kdis (1/s) |
|---|---|---|---|---|
| Human BAFF | BI-9ND2-A-YTE | 3.84E-11 | 5.15E+05 | 1.97E-05 |
| | Telitacicept | 1.47E-09 | 3.23E+05 | 4.75E-04 |
| Human APRIL | BI-9ND2-A-YTE | <1.0E-12 | 9.81E+05 | <1.0E-07 |
| | Telitacicept | 2.05E-09 | 6.78E+05 | 1.39E-03 |

Table 13 shows that the bispecific fusion protein having YTE mutations in the Fc region of the present disclosure was capable of specifically binding to the human BAFF protein and the human APRIL protein.

### Example 15: Sandwich ELISA for Bispecific Fusion Proteins

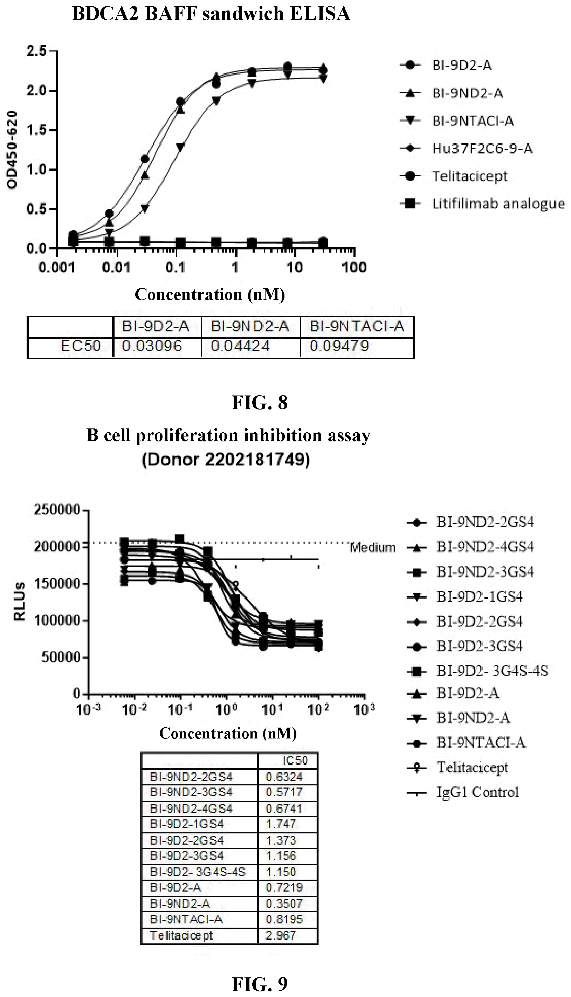

A 96-well plate was coated with 1 µg/mL human BDCA2 (ACRO Biosystems, Cat. No.: CLC-H5245) at 50 µL/well and incubated overnight at 4 °C. The plate was incubated with a blocking buffer (a PBS solution containing 1% BSA) at 37 °C and blocked for 1 h. After blocking, the plate was washed once with a PBST solution (a PBS solution containing 0.05% Tween 20). The bispecific fusion proteins were subjected to gradient dilution with a diluent and added to the plate, and the plate was incubated at 37 °C for 1 h. After the incubation was completed, the plate was washed with the PBST solution. Biotinylated human BAFF protein (KACTUS, Cat. No.: BAF-HM412B) was diluted to 1 µg/mL with a diluent and added to the plate. The plate was incubated at 37 °C for 1 h. After the incubation was completed, the plate was washed again. A secondary antibody (horseradish peroxidase HRP-labeled streptavidin, Jackson Immuno Research, Cat# 016-030-084) was diluted with a diluent and added to the plate. The plate was incubated at 37 °C for 1 h. After the incubation was completed, the plate was washed again and subjected to color development with TMB. Then the color development was stopped with 1 M H₂SO₄. Then the absorbance values at OD450 nm-OD620 nm were read on a microplate reader. The results are shown in FIG. 8.

The results show that the bispecific fusion proteins are capable of binding to both the human BDCA2 protein and the human BAFF protein by the double-antigen sandwich binding method.

### Example 16: Inhibition of B Cell Proliferation by Bispecific Fusion Proteins, Bispecific Fusion Proteins Having Different Linker Peptides, and Bispecific Fusion Protein Having YTE Mutations in Fc Region

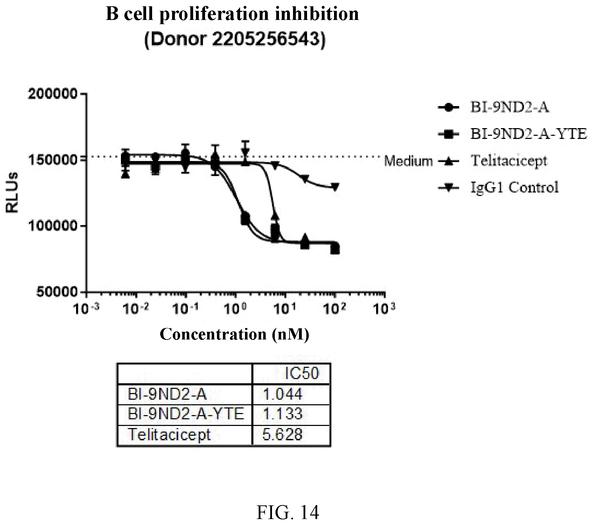

Human B cells were sorted from PBMCs (Shanghai Schbio Biotech, Donor: 2202181749) using a B cell sorting kit (Miltenyi Biotec, Cat. No.: 130-050-301). The cells were resuspended in an RPMI-1640 complete medium containing 20 µg/mL Anti-IgM (Jackson ImmunoResearch, Cat. No.: 109-007-043), 20 ng/mL IL-4 (PeproTech, Cat. No.: 200-04-100UG), 2 nM BAFF (ACRO Biosystems, Cat. No.: BAF-H52D4), and 10 nM APRIL (ACRO Biosystems, Cat. No.: APL-H52D1). The cell density was adjusted to 1E5 cells/mL, and 100 µL of the cell suspension was inoculated into a 96-well plate. The 10× antibody was prepared in an RPMI-1640 complete medium such that the final concentration was 100 nM. 4-fold gradient dilution was performed to obtain 8 points. 20 µL of the antibody was added to a 96-well plate, and each well was brought up to a total volume of 200 µL with an RPMI-1640 complete medium. The 96-well plate was incubated in an incubator at 37 °C with 5% CO₂ for 4 days and then centrifuged at 400 g for 3 min. 50 µL of the supernatant was removed, and 50 µL of Cell Counting-Lite 2.0 Luminescent Cell Viability Assay (Nanjing Vazyme Biotech Co., Ltd., Cat. No.: DD1101-02) was added. Luminescence was measured using a luminescence microplate reader (Tecan F200 Pro). The results are shown in FIGs. 9 and 14.

The results show that the anti-BDCA2/TACI bispecific fusion proteins were all able to inhibit the B cell proliferation induced by BAFF and APRIL, and the inhibitory activity of BI-9D2-A, BI-9ND2-A, and BI-9NTACI-A was better than that of the TACI fusion protein telitacicept. Linker regions of the bispecific fusion proteins were engineered. The bispecific fusion proteins having different linker peptides were also able to inhibit the B cell proliferation induced by BAFF and ARPIL, and the inhibitory activity was generally better than that of the TACI fusion protein telitacicept. The engineering of the linker regions did not affect the inhibitory effect of the bispecific proteins on the B cell proliferation. The YTE mutations were performed on the Fc region of the bispecific fusion protein. The engineered bispecific fusion protein BI-9ND2-A-YTE could also inhibit the B cell proliferation, and the inhibitory activity was better than that of the TACI fusion protein telitacicept. The YTE mutations did not affect the inhibitory effect of the bispecific fusion protein on the B cell proliferation.

### Example 17: Killing of Cells Expressing Human BDCA2 Protein by Bispecific Fusion Proteins, Bispecific Fusion Proteins Having Different Linker Peptides, and Bispecific Fusion Protein Having YTE Mutations in Fc Region

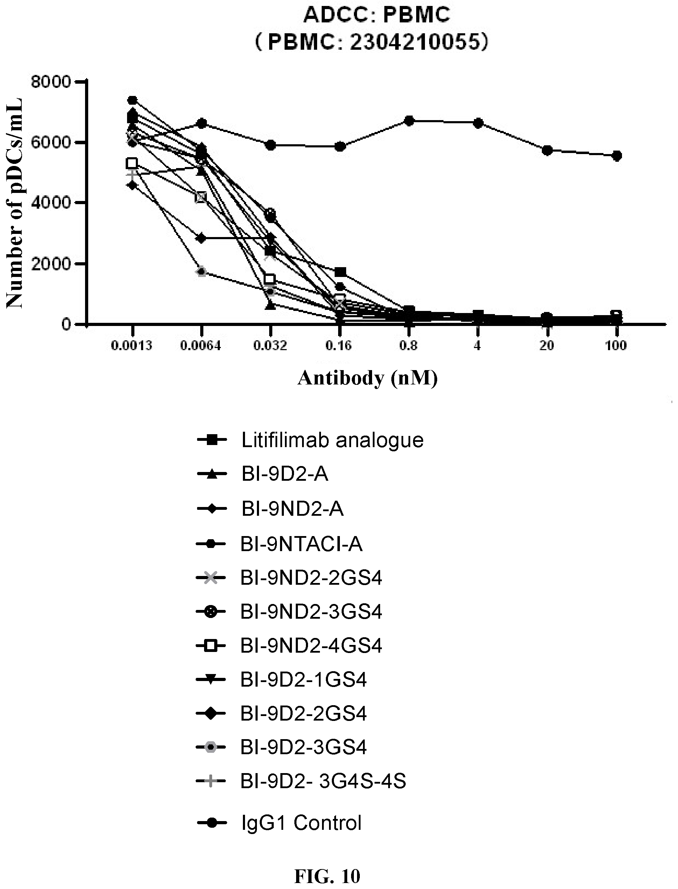

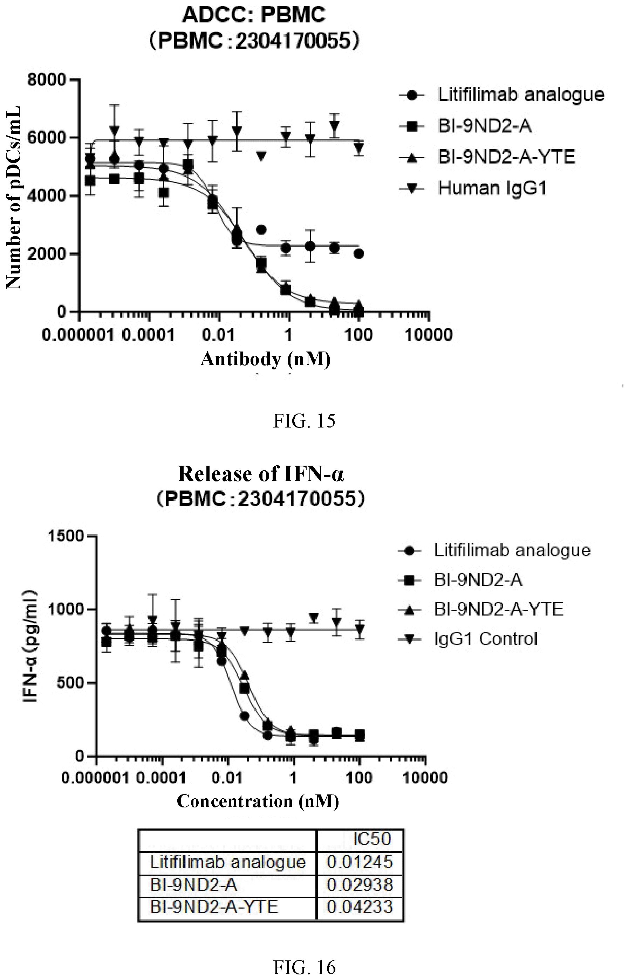

The experimental procedures were the same as those in Example 9, and the results are shown in FIGs. 10 and 15.

The results show that the bispecific fusion proteins BI-9D2-A, BI-9ND2-A, and BI-9NTACI-A were all able to eliminate the number of pDCs and had better ADCC activity than the litifilimab analogue. The linker regions of the bispecific fusion proteins were engineered, and the bispecific fusion proteins having different linker peptides were also able to eliminate the number of pDCs and had better ADCC activity than the litifilimab analogue. The YTE mutations were performed on the Fc region of the bispecific fusion protein, and the engineered bispecific fusion protein BI-9ND2-A-YTE was also able to eliminate the number of pDCs and had better ADCC activity than the litifilimab analogue. The YTE mutations did not affect the killing of cells expressing the human BDCA2 protein by the bispecific fusion protein.

### Example 18: Inhibition of Production of IFN-α in TLR9 Agonist-Stimulated PBMCs by Bispecific Fusion Proteins, Bispecific Fusion Proteins Having Different Linker Peptides, and Bispecific Fusion Protein Having YTE Mutations in Fc Region

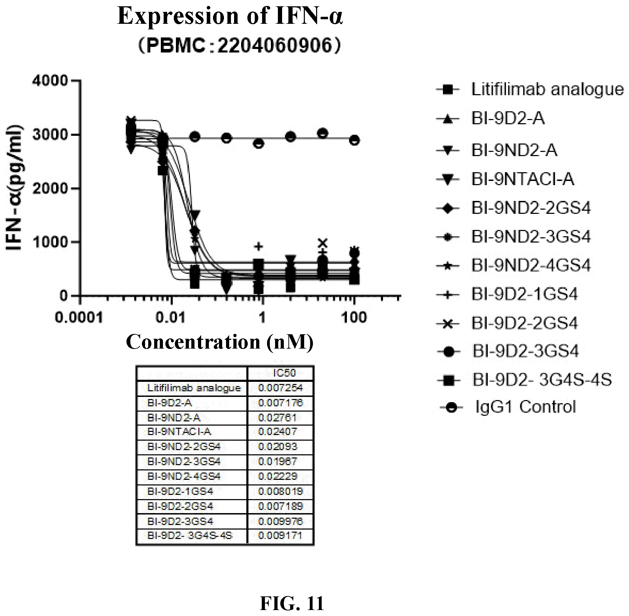

The experimental procedures were the same as those in Example 6, and the results are shown in FIGs. 11 and 16.

The results show that the TLR9 agonist could promote the release of IFN-α in PBMCs, while the bispecific fusion proteins BI-9D2-A and BI-9NTACI-A were both able to inhibit the release of IFN-α, with inhibitory effects comparable to that of the litifilimab analogue. The linker regions of the bispecific fusion proteins BI-9D2-A and BI-9ND2-A were engineered, and the bispecific fusion proteins having different linker peptides were also able to inhibit the release of IFN-α. The engineering of linkers did not affect the inhibitory effects of the bispecific proteins on IFN-α. The YTE mutations were performed on the Fc region of the bispecific fusion protein BI-9ND2-A, and the engineered bispecific fusion protein BI-9ND2-A-YTE was also able to inhibit the release of IFN-α.

### Example 19: Effect of Bispecific Fusion Proteins and Bispecific Fusion Protein Having YTE Mutations in Fc Region in Eliminating pDCs in huHSC-NCG Mouse Model

19.1 The *in vivo* efficacy of the BDCA2-TACI bispecific fusion proteins in eliminating pDCs was investigated in a huHSC-NCG mouse model.

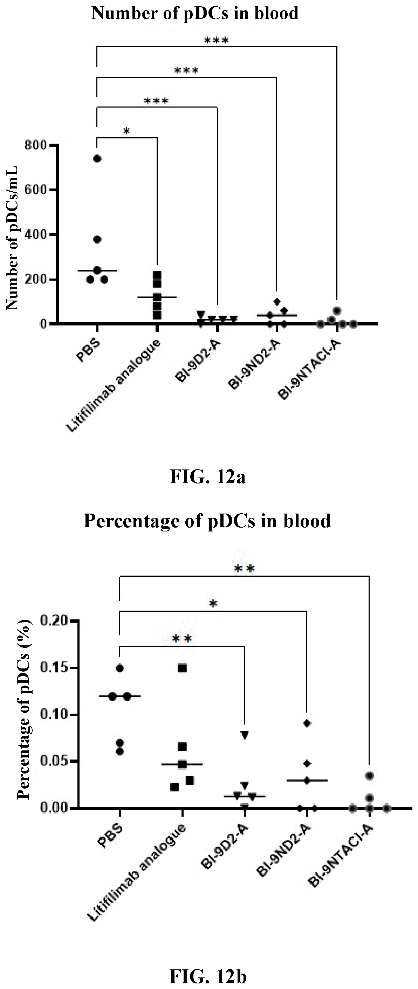

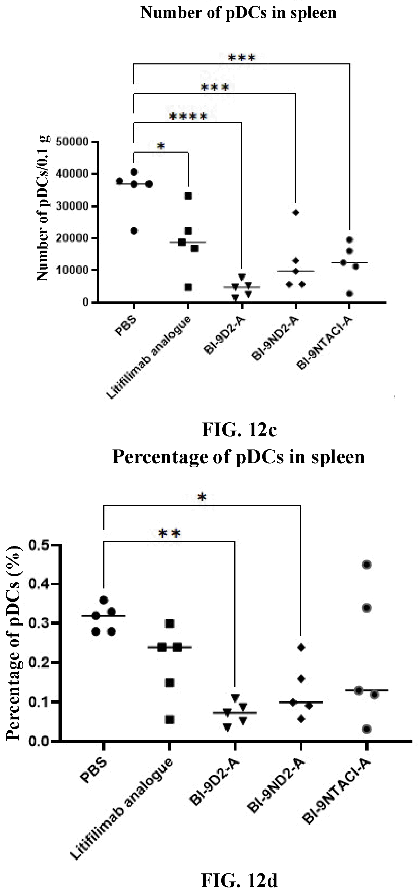

For the experiments herein, huHSC-NCG mice were purchased from Gempharmatech Co., Ltd. After a one-week adaptation period, 25 huHSC-NCG mice were randomly divided into 5 experimental groups, 5 mice per group. On day 0, the blank control group (PBS) (G1), litifilimab analogue (10 mg/kg) (G2), BI-9D2-A (10 mg/kg) (G3), BI-9ND2-A (10 mg/kg) (G4), and BI-9NTACI-A (10 mg/kg) (G5) were administered intraperitoneally to the mice, respectively. On day 3, peripheral blood and spleens of the mice in each group were collected for pDCs detection. As shown in FIG. 12, each bispecific antibody group can exhibit an extremely significant reduction in the proportions of pDCs in the peripheral blood and spleen, as compared to the PBS group. BI-9D2-A and BI-9ND2-A can significantly reduce the numbers and percentages of pDCs in the peripheral blood and spleen.

19.2 The *in vivo* efficacy of the BDCA2-TACI bispecific fusion protein with YTE mutations in the Fc region in eliminating pDCs was investigated in a huHSC-NCG mouse model.

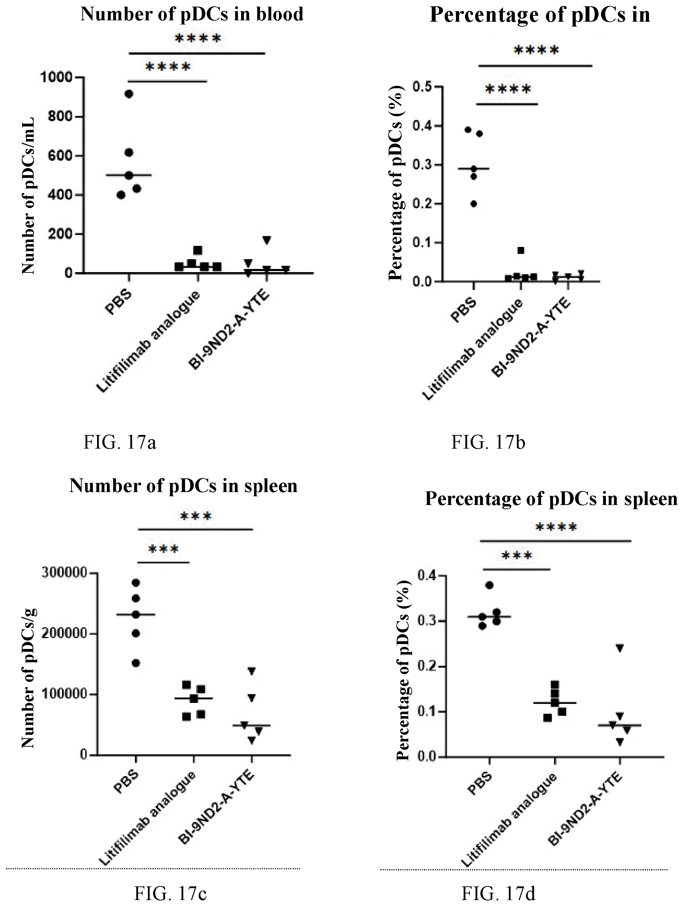

For the experiments herein, huHSC-NCG mice were purchased from Gempharmatech Co., Ltd. After a one-week adaptation period, 15 huHSC-NCG mice were randomly divided into 5 experimental groups, 5 mice per group. On day 0, the blank control group (PBS) (G1), litifilimab analogue (10 mg/kg) (G2), and BI-9ND2-A-YTE (10 mg/kg) were administered intraperitoneally to the mice, respectively. On day 3, the peripheral blood and spleen of the mice in each group were collected for pDCs detection. As shown in FIGs. 17a-17d, the bispecific antibody group can exhibit an extremely significant reduction in the numbers and percentages of pDCs in the peripheral blood and spleen, as compared to the PBS group. The YTE mutations did not affect the elimination effect on pDCs by the bispecific fusion protein.

### Example 20: Effects of Bispecific Fusion Protein and Bispecific Fusion Protein Having YTE Mutations in Fc Region in KLH-Induced Delayed-Type Hypersensitivity (DTH) Mouse Model 20.1 Effects of the bispecific fusion protein in a KLH-induced delayed-type hypersensitivity (DTH) BALB/c mouse model

The effects of the bispecific fusion protein were investigated in a KLH (Sigma, Cat. No.: SRP6195)-induced delayed-type hypersensitivity (DTH) BALB/c mouse model. The BALB/c mice were purchased from Gempharmatech Co., Ltd. After a one-week adaptation period, BALB/c mice aged 6-8 weeks were randomly divided into a healthy control group (6 mice), an induction modeling group (6 mice), and 2 antibody administration model groups (6 × 2 mice = 12 mice) according to the body weight. The day of grouping was defined as D0. The modeling group and the efficacy groups were induced with KLH (10 µg/g × mouse body weight (g)) (i.p.) on day 0 (D0) and day 12 (D12), respectively. The healthy control group was injected with normal saline (i.p.) on D4 and D11. The bispecific fusion protein was administered to the experimental group on D4 and D11 by i.p., at an administration volume of 10 µL/g × mouse body weight (g) and administration doses of 20 mpk for BI-9ND2-A and 10 mpk for telitacicept. The order of administration was kept consistent at each dose. With D20 as the endpoint, serum was collected, and the anti-KLH antibody level (IgM) was measured by ELISA (Life Diagnostics, Cat. No.: KLHM-1). Spleens were collected and weighed after the mice were euthanized. Flow cytometry assays were performed on spleen immune cells. The spleen flow cytometry assays were as follows: CD45 (Biolegend, Cat. No.: 103140), CD3 (BD, Cat. No.: 562600), CD4 (BD, Cat. No.: 560181), PD1 (Biolegend, Cat. No.: 135231), CD185 (BD, Cat. No.: 551959), B220 (Biolegend, Cat. No.: 103206), CD19 (Biolegend, Cat. No.: 115546), and CD138 (Biolegend, Cat. No.: 142506).

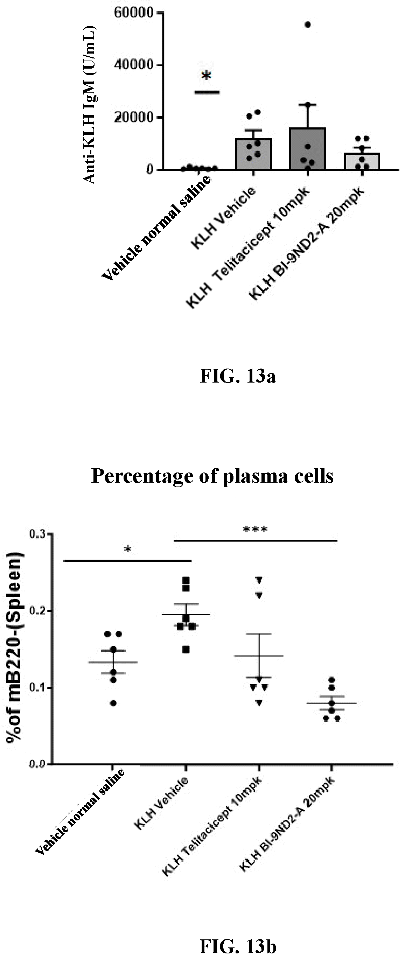

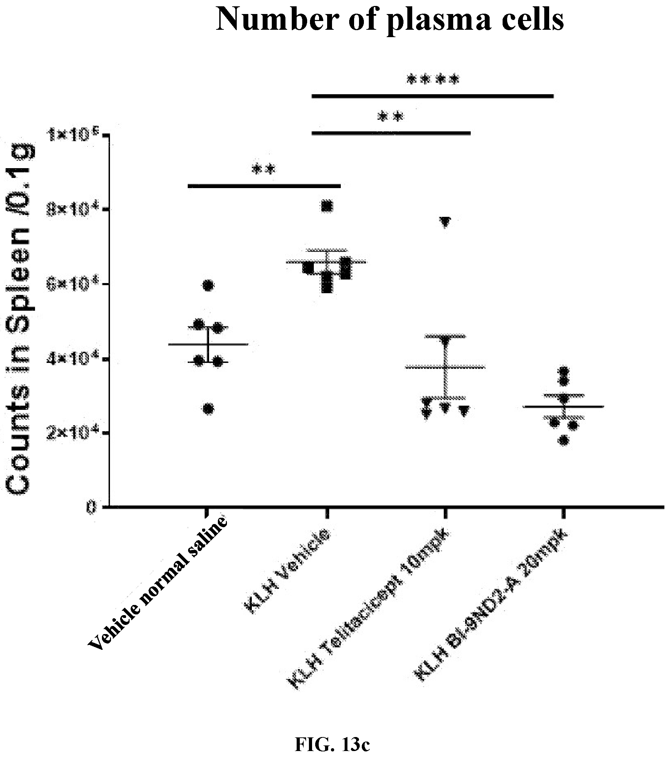

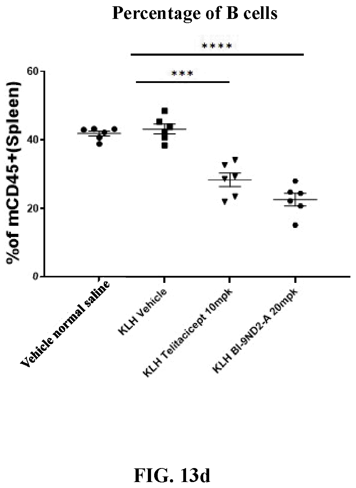

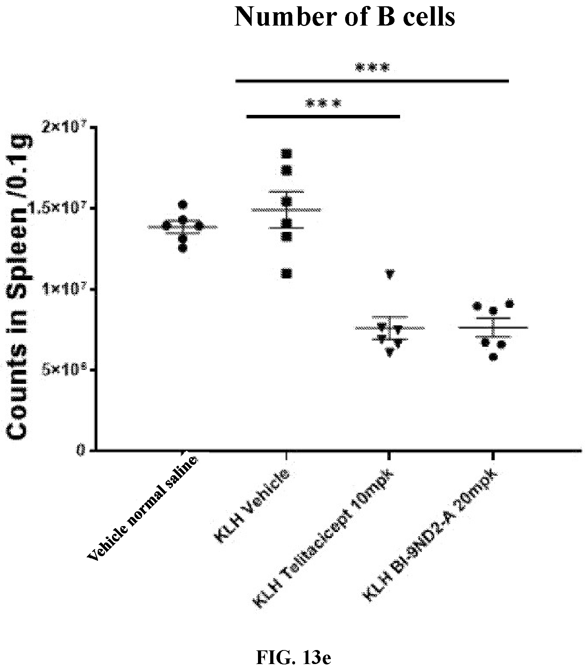

As shown in FIG. 13, the KLH IgM level in the KLH vehicle group was significantly increased compared to that in the normal saline vehicle group, and BI-9ND2-A can significantly inhibit the KLH IgM expression level. The flow cytometry results show that the bispecific fusion protein BI-9ND2-A can significantly eliminate the expression of mouse plasma cells, and the elimination effect was better than that of the control antibody telitacicept. In addition, BI-9ND2-A can also significantly inhibit the expression of mouse B cells, and the inhibitory effect was better than that of the control antibody telitacicept.

### 20.2 Effects of the bispecific fusion protein having YTE mutations in the Fc region in a KLH-induced delayed-type hypersensitivity (DTH) B-hFcRn mouse model

The effects of the bispecific fusion protein having YTE mutations in the Fc region were investigated in a KLH (Sigma, Cat. No.: SRP6195)-induced delayed-type hypersensitivity (DTH) B-hFcRn mouse model. B-hFcRn mice were purchased from Biocytogen. After a one-week adaptation period, B-hFcR mice aged 6-8 weeks were randomly divided into a healthy control group (8 mice), an induction modeling group (8 mice), and 2 antibody administration model groups (8 × 2 mice = 16 mice) according to the body weight. The day of grouping was defined as D0. The modeling group and the efficacy groups were induced with KLH (10 µg/g × mouse body weight (g)) (i.p.) on D0 and D12, respectively. The health control group was injected with normal saline (i.p.) on D4, D7, D10, D13, and D16. The bispecific fusion protein was administered on D4, D7, D10, D13, and D16 by i.p., at an administration volume of 10 µL/g × mouse body weight (g) and administration doses of 20 mpk for BI-9ND2-A-YTE and 10 mpk for telitacicept.

The order of administration was kept consistent at each dose. D20 was the endpoint. Serum was collected, and the concentration of the antigen in the mouse serum was measured.

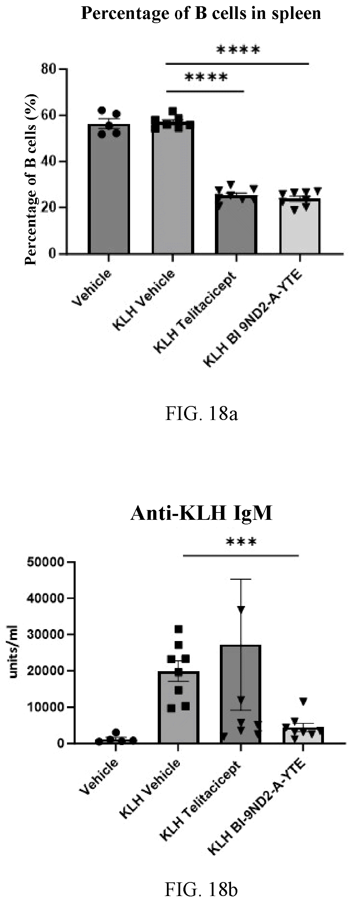

Spleens were collected and weighed after the mice were euthanized. Flow cytometry assays were performed on spleen immune cells. The spleen flow cytometry assays were as follows: CD45 (Biolegend, Cat. No.: 103140), CD3 (Biolegend, Cat. No.: 100326), CD4 (Biolegend, Cat. No.: 100526), B220 (Biolegend, Cat. No.: 103240), and CD138 (Biolegend, Cat. No.: 142519). As shown in FIG. 18a, the percentage of B cells in the spleen was measured using a flow cytometer, and BI-9ND2-A-YTE, the bispecific fusion protein having YTE mutations in the Fc region, can significantly inhibit the expression of mouse B cells.

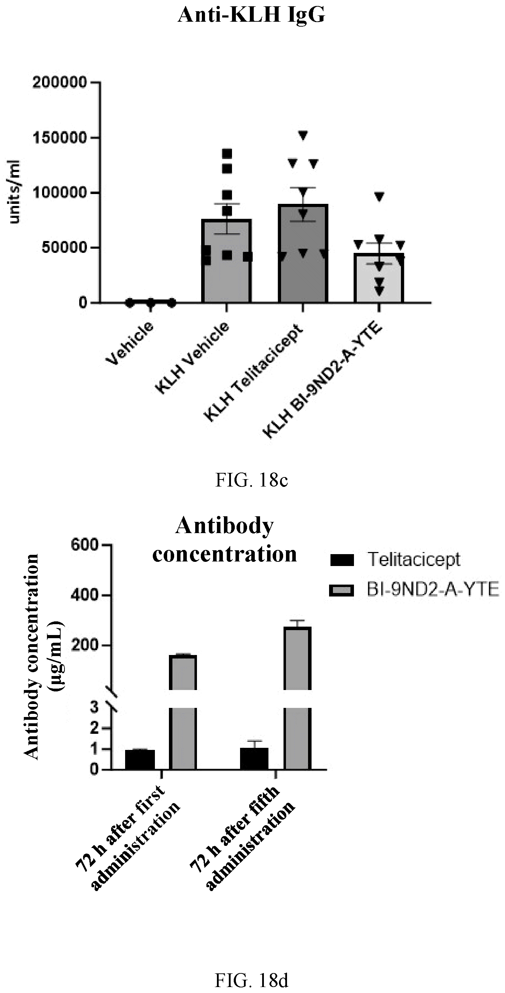

At the endpoint of the experiment, mouse serum was collected, and the anti-KLH antibody levels (IgM and IgG) were measured using an ELISA kit (Life Diagnostics, Cat. No.: KLHM-1 and KLHG-1). As shown in FIGs. 18b and 18c, the levels of anti-KLH IgM and IgG were significantly increased in the KLH vehicle group compared to those of the vehicle group, and BI-9ND2-A-YTE can significantly inhibit the expression levels of KLH IgM and IgG.

72 h after the first administration and 72 h after the fifth administration, mouse serum was collected, and the antibody concentration in the mouse serum was measured.

BI-9ND2-A-YTE was assayed by a sandwich method: A 96-well plate was coated with 1 µg/mL human BDCA2 protein (ACRObiosystems, Cat# CLC-H5245) at 50 µL/well and incubated overnight at 4 °C. The plate was incubated with a blocking buffer (a PBS solution containing 1% BSA) at 37 °C and blocked for 1 h. After blocking, the plate was washed once with a PBST solution (a PBS solution containing 0.05% Tween 20). The bispecific antibody (BI-9ND2-A-YTE) was subjected to gradient dilution with a diluent containing 2% mouse blank serum (a PBS solution containing 0.05% Tween 20 and 0.5% BSA) as a standard. The serum samples were diluted at different ratios with mouse blank serum and the diluent, and added to the plate. The plate was incubated at 37 °C for 1 h. After the incubation was completed, the plate was washed twice with the PBST solution. Biotinylated human BAFF protein (KACTUS, Cat# BAF-HM412B) was diluted to 1 µg/mL with a diluent, and added to the plate. The plate was incubated at 37 °C for 1 h. After the incubation was completed, the plate was washed with the PBST solution. A secondary antibody (horseradish peroxidase HRP-labeled streptavidin, Jackson Immuno Research, Cat# 016-030-084) was diluted with a diluent and added to the plate. The plate was incubated at 37 °C for 1 h. After the incubation was completed, the plate was washed again and subjected to color development with TMB (Thermo Scientific, Cat# 34029). Then the color development was stopped with 1 M H₂SO₄. Then the absorbance values at OD450 nm-OD620 nm were read on a microplate reader.

Detection method for telitacicept: A 96-well plate was coated with 0.5 µg/mL human BAFF protein (ACRObiosystems, Cat# BAF-H52D4) at 50 µL/well and incubated overnight at 4 °C. The plate was incubated with a blocking buffer (a PBS solution containing 1% BSA) at 37 °C and blocked for 1 h. After blocking, the plate was washed once with a PBST solution (a PBS solution containing 0.05% Tween 20). Telitacicept was subjected to gradient dilution with a diluent containing 2% mouse blank serum (a PBS solution containing 0.05% Tween 20 and 0.5% BSA) as a standard. The serum samples were diluted at different ratios with mouse blank serum and the diluent, and added to the plate. The plate was incubated at 37 °C for 1 h. After the incubation was completed, the plate was washed with the PBST solution. A secondary antibody (horseradish peroxidase HRP-labeled goat anti-human IgG, Fcγ fragment specificity, Jackson Immuno Research, Cat# 109-035-098) was diluted with a diluent and added to the plate. The plate was incubated at 37 °C for 1 h. After the incubation was completed, the plate was washed again and subjected to color development with TMB (Thermo Scientific, Cat#: 34029). Then the color development was stopped with 1 M H₂SO₄. The absorbance values at OD450 nm-OD620 nm were then read on a microplate reader.

As shown in FIG. 18d, in the KLH-induced delayed-type hypersensitivity (DTH) B-hFcRn mouse model, at the same time point, the concentration of BI-9ND2-A-YTE, the bispecific fusion protein having YTE mutations in the Fc region, was higher than that of telitacicept, suggesting that BI-9ND2-A-YTE was eliminated more slowly in mice and had better PK than telitacicept.

### Example 21: Efficacy of Bispecific Fusion Protein in MOG-Induced C57BL/6J Experimental Allergic Encephalomyelitis (EAE) Model

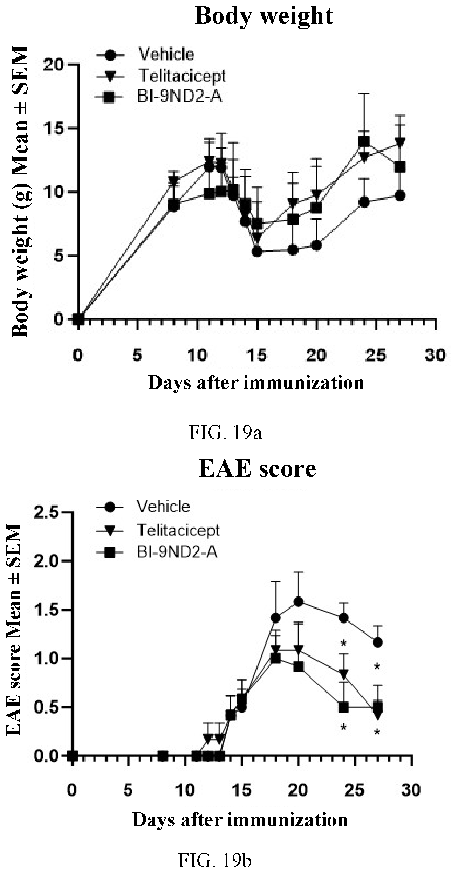

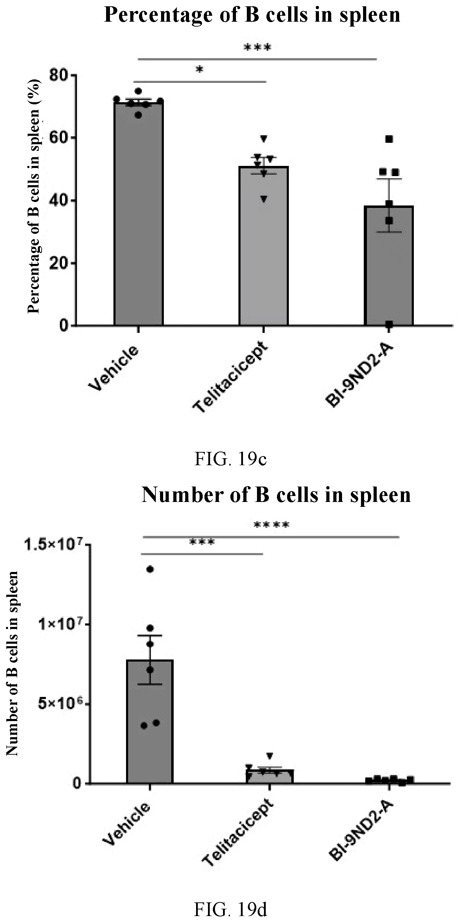

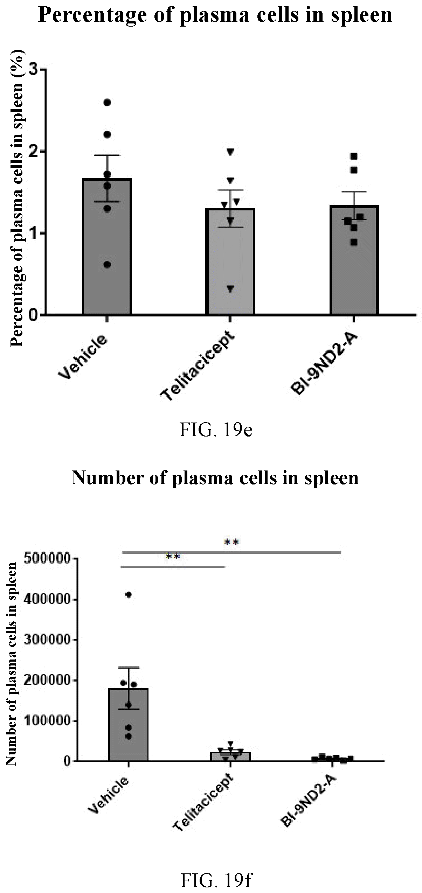

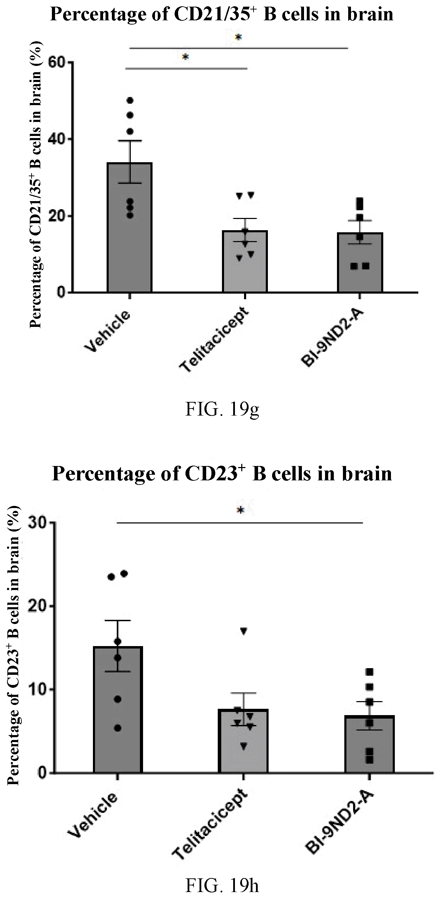

The effect of the bispecific fusion protein was investigated in a MOG₃₅₋₅₅ (Sangon Biotech)-induced C57BL/6J experimental allergic encephalomyelitis (EAE) model. For the experiments herein, C57BL/6J mice were purchased from Zhejiang Vital River Laboratory Animal Technology Co., Ltd. After a one-week adaptation period, C57BL/6J female mice aged 8-10 weeks were injected with 200 µL of a complete Freund's adjuvant (s.c.) containing 100 µg of MOG₃₅₋₅₅. Then, 2 h and 48 h after the injection, the mice were injected (i.p.) with pertussis toxin (PTX, Listlabs, Cat. No.: #180) at a dose of 200 ng per mouse. The day of induction was defined as D0. On D15, the mice were randomly divided into a model control group (6 mice) and antibody administration model groups (6 × 2 mice = 12 mice) based on their disease status and body weight. Administration was performed on D15, D18, D21, and D24 via the intraperitoneal route (i.p.), with the test article being the bispecific fusion protein described herein. The administration volume was calculated as 10 µL/g × mouse body weight (g). Administration doses: 20 mpk for BI-9ND2-A and 10 mpk for telitacicept. The order of administration was kept consistent at each dose. The body weight and clinical symptoms of the mice were determined and scored twice a week. Grade 0: no clinical symptoms; grade 1: tail weakness; grade 2: tail weakness + hind limb weakness; grade 3: tail weakness + hind limb paralysis; grade 4: complete limb paralysis; grade 5: moribund state or death. With D27 as the endpoint, the spleen and brain were collected after the mice were euthanized. The spleen and part of the brain were taken to prepare a single-cell suspension. Flow cytometry assays were performed on immune cells. The spleen flow cytometry assays were as follows: DL, CD45, CD19, and CD138. The brain flow cytometry assays were as follows: DL, CD45, CD3, CD19, CD23, and CD21/35. The results can be seen in FIGs. 19a-19h.

The results show that compared to the G1 group (vehicle), the body weight of the mice in the G2 group (telitacicept) and the G3 group (BI-9ND2-A) was increased, the clinical symptoms of the mice were significantly ameliorated, and the EAE score was significantly reduced, and the reduction was statistically significant.

After the mice were sacrificed, the spleen and brain were collected to prepare a single-cell suspension, and the proportion of each cell type was measured by a flow cytometer. As shown in FIGs. 19c-19h, the number and percentage of B cells in the spleen of mice were significantly reduced after BI-9ND2-A administration compared to the vehicle group, indicating that BI-9ND2-A can reduce B cells in the spleen of mice and had better efficacy than telitacicept. In addition, after BI-9ND2-A administration, the number and percentage of plasma cells in the spleen were also significantly reduced, indicating that BI-9ND2-A can partially eliminate plasma cells in the spleen, and the elimination effect is comparable to that of telitacicept. CD21/35 and CD23 are surface markers for activated B cells. After BI-9ND2-A treatment, the percentages of CD21/35⁺ and CD23⁺ B cells in the brain were significantly reduced, indicating that BI-9ND2-A can inhibit the activation of B cells in the brain.

### Example 22: Related Assays for Fc Regions of Bispecific Fusion Protein After YTE Mutations 22.1 Assay on affinity of bispecific fusion protein having YTE mutations in Fc region for human FcRn

The binding affinity of the BI-9ND2-A and BI-9ND2-A-YTE antibodies for the human FcRn protein (ACRO Biosystems, Cat. No.: FCM-H82W4) was measured using ForteBio Octet RED96e.

An SA sensor (ForteBio, Cat. No.: 18-5019) was pre-equilibrated in a loading buffer (1× PBS, Cytiva, Cat. No.: SH30256.01, containing 0.05% Tween 20, pH 7.0) at room temperature for 10 min. In a 96-well plate, the FcRn kinetic assay was performed according to the following steps:
a) equilibrating the baseline with a loading buffer for 180 s;
b) adding the human FcRn protein diluted with the loading buffer at a final concentration of 1 µg/mL, and stopping the immobilization until the amount reaches 0.3 nm;
c) equilibrating the baseline with a blocking solution (1× PBS, Cytiva, Cat. No.: SH30256.01, containing 0.05% Tween 20, 0.1% BSA, pH 7.0) for 180 s;
d) adding the BI-9ND2-A and BI-9ND2-A-YTE antibodies diluted with a running buffer (1× PBS, Cytiva, Cat. No.: SH30256.01, containing 0.05% Tween 20, 0.1% BSA, pH 6.0/7.4) at the following concentrations to each well: 200 nM, 100 nM, 50 nM, 25 nM, 12.5 nM, 6.25 nM, and 3.13 nM, binding for 60 s (pH 6.0), and dissociating for 60 s (pH 6.0/7.4); and
e) applying a regeneration solution (1× PBS, Cytiva, Cat. No.: SH30256.01, pH 7.7) for 30 s, and equilibrating the baseline with the blocking solution for 60 s. The experimental data were fitted and calculated using a 1:1 binding model in the Fortebio Data Analysis software.

**Table 14. Binding affinity of BI-9ND2-A and BI-9ND2-A-YTE antibodies for human FcRn protein**

| Antibody | Antigen | KD (M) | Kon (1/Ms) | Kdis (1/s) |
|---|---|---|---|---|
| BI-9ND2-A (binding at pH 6.0, dissociation at pH 6.0) | | 2.01E-08 | 1.74E+06 | 3.50E-02 |
| BI-9ND2-A (binding at pH 6.0, dissociation at pH 7.4) | Human FcRn | 3.14E-06 | 2.68E+04 | 8.42E-02 |
| BI-9ND2-A-YTE (binding at pH 6.0, dissociation at pH 6.0) | | 5.12E-09 | 1.21E+06 | 6.20E-03 |
| BI-9ND2-A-YTE (binding at pH 6.0, dissociation at pH 7.4) | | 7.27E-07 | 1.22E+05 | 8.88E-02 |

Table 14 shows that the affinity for FcRn of the BI-9ND2-A-YTE bispecific fusion protein was increased compared to that of BI-9ND2-A when the antibodies of the present disclosure were subjected to binding for 60 s (pH 6.0) and dissociation for 60 s (pH 6.0) in a running buffer, which indicates that the YTE mutation engineering of the bispecific fusion protein was successful.

### 22.2 PK assay of bispecific fusion protein having YTE mutations in Fc region in B-hFcRn mice

B-hFcRn mice (purchased from Biocytogen) were intravenously injected with 10 mg/kg of bispecific fusion protein BI-9ND2-A and BI-9ND2-A-YTE, the bispecific fusion protein having YTE mutations in the Fc region. The serum of the mice was collected before administration and at 0.167 h, 1 h, 4 h, 8 h, 24 h, 48 h, 96 h, 168 h, and 240 h after administration, and the antibody concentration in the serum of the mice was measured using a sandwich method. The sandwich method comprises the following steps: A 96-well plate was coated with 1 µg/mL human BDCA2 protein (ACRObiosystems, Cat# CLC-H5245) at 50 µL/well and incubated overnight at 4 °C. The plate was incubated with a blocking buffer (a PBS solution containing 1% BSA) at 37 °C and blocked for 1 h. After blocking, the plate was washed once with a PBST solution (a PBS solution containing 0.05% Tween 20). The bispecific antibodies (BI-9ND2-A, BI-9ND2-A-YTE) were subjected to gradient dilution with a diluent containing 2% mouse blank serum (a PBS solution containing 0.05% Tween 20 and 0.5% BSA) as standards. The serum samples were diluted at different ratios with mouse blank serum and the diluent, and added to the plate. The plate was incubated at 37 °C for 1 h. After the incubation was completed, the plate was washed twice with the PBST solution. Biotinylated human BAFF protein (KACTUS, Cat# BAF-HM412B) was diluted to 1 µg/mL with a diluent, and added to the plate. The plate was incubated at 37 °C for 1 h. After the incubation was completed, the plate was washed with the PBST solution. A secondary antibody (horseradish peroxidase HRP-labeled streptavidin, Jackson Immuno Research, Cat# 016-030-084) was diluted with a diluent and added to the plate. The plate was incubated at 37 °C for 1 h. After the incubation was completed, the plate was washed again and subjected to color development with TMB (Thermo Scientific, Cat# 34029). Then the color development was stopped with 1 M H2SO4. Then the absorbance values at OD450 nm-OD620 nm were read on a microplate reader, and finally, the pharmacokinetic parameters were calculated by WinNonlin software.

**Table 15. PK parameters of BI-9ND2-A and BI-9ND2-A-YTE bispecific fusion proteins in B-hFcRn mice**

| Antibody | AUC(0-∞) h*µg/mL | T_{1/2} h | CL mL/h/kg | Cₘₐₓ µg/mL | Vz mL/kg |
|---|---|---|---|---|---|
| BI-9ND2-A | 5610.2 | 35.1 | 1.8 | 193.4 | 90.5 |
| BI-9ND2-A-YTE | 9366.3 | 48.1 | 1.1 | 192.9 | 73.9 |

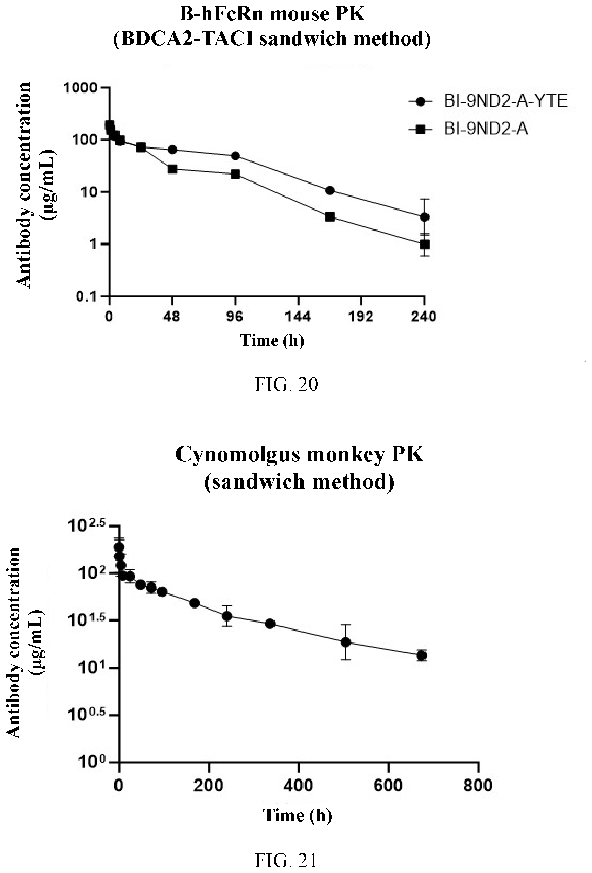

The results are shown in Table 15 and FIG. 20. Compared to the bispecific fusion protein BI-9ND2-A without Fc mutation, BI-9ND2-A-YTE showed longer T_{1/2} time (48.1 h), higher AUC(0-∞), and lower CL values, indicating that BI-9ND2-A-YTE exhibited better PK in B-hFcRn mice after YTE engineering of the Fc region.

### Example 23: PK Assay of Bispecific Fusion Protein Having YTE Mutations in Fc Region in Cynomolgus Monkeys

The cynomolgus monkeys (n = 2, Nanjing ClinBridge Biotech Co. Ltd.) were intravenously injected with BI-9ND2-A-YTE, the bispecific fusion protein having YTE mutations in the Fc region, at a single dose of 10 mg/kg, and the serum of the cynomolgus monkeys was collected before administration and at 0.017 h, 1 h, 4 h, 8 h, 24 h, 48 h, 72 h, 96 h, 168 h, 240 h, 336 h, 504 h, and 672 h after administration. The concentration of the antibody in the serum was measured by a sandwich method. The sandwich method comprises the following steps: A 96-well plate was coated with 1 µg/mL human BDCA2 protein (ACRObiosystems, Cat# CLC-H5245) at 50 µL/well and incubated overnight at 4 °C. The plate was incubated with a blocking buffer (a PBS solution containing 1% BSA) at 37 °C and blocked for 1 h. After blocking, the plate was washed once with a PBST solution (a PBS solution containing 0.05% Tween 20). The bispecific antibody (BI-9ND2-A-YTE) was subjected to gradient dilution with a diluent containing 1% cynomolgus monkey blank serum (a PBS solution containing 0.05% Tween 20 and 0.5% BSA) as a standard. The serum samples were diluted at different ratios with cynomolgus monkey blank serum and the diluent, and added to the plate. The plate was incubated at 37 °C for 1 h. After the incubation was completed, the plate was washed twice with the PBST solution. Biotinylated human BAFF protein (KACTUS, Cat# BAF-HM412B) was diluted to 1 µg/mL with a diluent, and added to the plate. The plate was incubated at 37 °C for 1 h. After the incubation was completed, the plate was washed with the PBST solution. A secondary antibody (horseradish peroxidase HRP-labeled streptavidin, Jackson Immuno Research, Cat# 016-030-084) was diluted with a diluent and added to the plate. The plate was incubated at 37 °C for 1 h. After the incubation was completed, the plate was washed again and subjected to color development with TMB (Thermo Scientific, Cat# 34029). Then the color development was stopped with 1 M H2SO4. Then the absorbance values at OD450 nm-OD620 nm were read on a microplate reader, and finally, the pharmacokinetic parameters were calculated by WinNonlin software.

**Table 16. PK parameters of bispecific fusion protein having YTE mutations in Fc region in cynomolgus monkeys**

| Antibody | AUC(0-∞) h*µg/mL | T_{1/2} h | CL mL/h/kg | Cₘₐₓ µg/mL | Vz mL/kg |
|---|---|---|---|---|---|
| BI-9ND2-A-YTE | 30676.2 | 281.8 | 0.3 | 193.3 | 135.0 |

The results are shown in Table 16 and FIG. 21. The bispecific fusion protein having YTE mutations in the Fc region had relatively good PK in cynomolgus monkeys, with a T_{1/2} of 281.8 h after administration at a single dose of 10 mg/kg.

The present application relates to the following biological sequences:

| **Sequence No.** | **Description** | **Amino acid sequence** |
|---|---|---|
| SEQ ID NO: 1 | Heavy chain variable region (VH) of antibody 26H10A11-1 | DVKLVESGGGLVKPGGSLKLSCAASGFTFSSSTMSWVRQ TPEKRLEWVATTGGGGSYTYNPDSVKGRFTISRDNAKNT LYLQMSSLRSEDTAMYYCTRDRYDGYYFSMDFWGQGT SVTVSS |
| SEQ ID NO: 2 | Light chain variable region (VL) of antibody 26H10A11-1 | DIVLTQSPASLAVSLGQRATISCKASQSVDYDGDSYMNW YQQKPGQPPKLLIYΛΛSNLESGIPΛRFSGSGSGTDFTLNIH PVEEEDAATYYCQQSNEDPRTFGGGTKLEIK |
| SEQ ID NO: 3 | Heavy chain variable region (VH) of antibody 37F2C6 | QVLLQQSGPELVRPGVSVKVSCKGSGYTFTDFAVHWVK QSQTKSLEWIGLIGTSSGSTNYNQKFKDKATMTVDKSSN TAYLELARLTSEDSAIYYCARNGYYVGYYALDYWGQGT SVTVSS |
| SEQ ID NO: 4 | Light chain variable region (VL) of antibody 37F2C6 | QTVLTQSPAIMSASPGEKVTITCSASSSVSYMYWFQQKP GTSPKLWIYSTSNLASGVPPRFSGSGSGTSYSLTISRMEAE DAATYYCQQRRNYPHTFGGGTKLEIK |
| SEQ ID NO: 5 | Human IgG1 heavy chain constant region | |
| SEQ ID NO: 6 | κ light chain constant region | |
| SEQ ID NO:7 | Heavy chain of control antibody litifilimab | DVQLVESGGGLVKPGGSLRLSCAASGFTFSTYTMSWVR QAPGKGLEWVATISPGDSFGYYYPDSVQGRFTISRDNAK NSLYLQMNSLRAEDTAVYYCTRDIYYNYGAWFAYWGQ GTLVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGK |
| SEQ ID NO:8 | Light chain of control antibody litifilimab | DIQLTQSPSSLSASVGDRVTITCKASQSVDYDGDSYMNW YQQKPGKAPKLLIYAASTLESGVPSRFSGSGSGTDFTLTIS SLQPEDFATYYCQQANEDPRTFGQGTKVEIKRTVAAPSV FIFPPSDEQLKSGTASVVCLLNNFYPREAKVQWKVDNAL QSGNSQESVTEQDSKDSTYSLSSTLTLSKADYEKHKVYA CEVTHQGLSSPVTKSFNRGEC |
| SEQ ID NO:9 | Heavy chain of antibody 26H10A11-1: 26H10A11VH-IgG1 | DVKLVESGGGLVKPGGSLKLSCAASGFTFSSSTMSWVRQ TPEKRLEWVATTGGGGSYTYNPDSVKGRFTISRDNAKNT LYLQMSSLRSEDTAMYYCTRDRYDGYYFSMDFWGQGT SVTVSSASTKGPSVFPLAPSSKSTSGGTAALGCLVKDYFP EPVTVSWNSGALTSGVHTFPAVLQSSGLYSLSSVVTVPSS SLGTQTYICNVNHKPSNTKVDKKVEPKSCDKTHTCPPCP APELLGGPSVFLFPPKPKDTLMISRTPEVTCVVVDVSHED PEVKFNWYVDGVEVHNAKTKPREEQYNSTYRVVSVLTV LHQDWLNGKEYKCKVSNKALPAPIEKTISKAKGQPREPQ VYTLPPSRDELTKNQVSLTCLVKGFYPSDIAVEWESNGQP ENNYKTTPPVLDSDGSFFLYSKLTVDKSRWQQGNVFSCS VMHEALHNHYTQKSLSLSPGK |
| SEQ ID NO:10 | Light chain of antibody 26H10A11-1: 26H10A11VL-kappa | DIVLTQSPASLAVSLGQRATISCKASQSVDYDGDSYMNW YQQKPGQPPKLLIYAASNLESGIPARFSGSGSGTDFTLNIH PVEEEDAATYYCQQSNEDPRTFGGGTKLEIKRTVAAPSV FIFPPSDEQLKSGTASVVCLLNNFYPREAKVQWKVDNAL QSGNSQESVTEQDSKDSTYSLSSTLTLSKADYEKHKVYA CEVTHQGLSSPVTKSFNRGEC |
| SEQ ID NO:11 | Heavy chain of antibody 37F2C6: 37F2C6VH-IgG1 | QVLLQQSGPELVRPGVSVKVSCKGSGYTFTDFAVHWVK QSQTKSLEWIGLIGTSSGSTNYNQKFKDKATMTVDKSSN TAYLELARLTSEDSAIYYCARNGYYVGYYALDYWGQGT SVTVSSASTKGPSVFPLAPSSKSTSGGTAALGCLVKDYFP EPVTVSWNSGALTSGVHTFPAVLQSSGLYSLSSVVTVPSS SLGTQTYICNVNHKPSNTKVDKKVEPKSCDKTHTCPPCP APELLGGPSVFLFPPKPKDTLMISRTPEVTCVVVDVSHED PEVKFNWYVDGVEVHNAKTKPREEQYNSTYRVVSVLTV LHQDWLNGKEYKCKVSNKALPAPIEKTISKAKGQPREPQ VYTLPPSRDELTKNQVSLTCLVKGFYPSDIAVEWESNGQP |
| | | ENNYKTTPPVLDSDGSFFLYSKLTVDKSRWQQGNVFSCS VMHEALHNHYTQKSLSLSPGK |
| SEQ ID NO:12 | Light chain of antibody 37F2C6: 37F2C6VL-kappa | QTVLTQSPAIMSASPGEKVTITCSASSSVSYMYWFQQKP GTSPKLWIYSTSNLASGVPPRFSGSGSGTSYSLTISRMEAE DAATYYCQQRRNYPHTFGGGTKLEIKRTVAAPSVFIFPPS DEQLKSGTASVVCLLNNFYPREAKVQWKVDNALQSGN SQESVTEQDSKDSTYSLSSTLTLSKADYEKHKVYACEVT HQGLSSPVTKSFNRGEC |
| SEQ ID NO:13 | Heavy chain variable region (VH) of humanized antibodies Hu37F2C6-8 and Hu37F2C6-8-A | QVQLVQSGAEVKKPGASVKVSCKASGYTFTDFAVHWVR QAPGQRLEWMGLIGTSSGSTNYNQKFQGRVTMTVDKSA NTAYMELSSLRSEDTAVYYCARNGYYVGYYALDYWGQ GTTVTVSS |
| SEQ ID NO:14 | Heavy chain variable region (VH) of humanized antibodies Hu37F2C6-9 and Hu37F2C6-9-A | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSS |
| SEQ ID NO:15 | Light chain variable region (VL) of humanized antibodies Hu37F2C6-8, Hu37F2C6-8-A, Hu37F2C6-9, and Hu37F2C6-9-A | ETVLTQSPATLSLSPGERATLSCRASSSVSYMYWFQQKPG QAPRLLIYSTSNLASGIPARFSGSGSGTDYTLTISSLEPEDF AVYYCQQRRNYPHTFGGGTKVEIK |
| SEQ ID NO:16 | Heavy chain of humanized antibodies Hu37F2C6-8 and Hu37F2C6-8-A: 37F2C6-HuVH2 | QVQLVQSGAEVKKPGASVKVSCKASGYTFTDFAVHWVR QAPGQRLEWMGLIGTSSGSTNYNQKFQGRVTMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGK |
| SEQ ID NO:17 | Heavy chain of humanized antibodies Hu37F2C6-9 and Hu37F2C6-9-A: 37F2C6-HuVH3 | QVQLVQSGAEVKKPGASVKVSCKASGYTFTDFAVHWVR QAPGQRLEWMGLIGTSSGSTNYNQKFQGRATMTVDKSA |
| | | NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGK |
| SEQ ID NO:18 | Light chain of humanized antibodies Hu37F2C6-8, Hu37F2C6-8-A, Hu37F2C6-9, and Hu37F2C6-9-A; | ETVLTQSPATLSLSPGERATLSCRASSSVSYMYWFQQKPG QAPRLLIYSTSNLASGIPARFSGSGSGTDYTLTISSLEPEDF AVYYCQQRRNYPHTFGGGTKVEIKRTVAAPSVFIFPPSDE QLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| | light chain of bispecific antibodies Bi-9CD2-DE-A and Bi-9ND2-DE-: 37F2C6-HuVL3 | |
| SEQ ID NO:19 | Control antibody telitacicept | SRVDQEERFPQGLWTGVAMRSCPEEQYWDPLLGTCMSC KTICNHQSQRTCAAFCRSLSCRKEQGKFYDHLLRDCISC ASICGQHPKQCAYFCENKLRSPVNLPPELDKTHTCPPCPA PEAEGAPSVFLFPPKPKDTLMISRTPEVTCVVVDVSHEDP EVKFNWYVDGVEVHNAKTKPREEQYNSTYRVVSVLTV LHQDWLNGKEYKCKVSNKALPSSIEKTISKAKGQPREPQ VYTLPPSRDELTKNQVSLTCLVKGFYPSDIAVEWESNGQP ENNYKTTPPVLDSDGSFFLYSKLTVDKSRWQQGNVFSCS VMHEALHNHYTQKSLSLSPGK |
| SEQ ID NO:20 | Fusion protein TACI | SRVDQEERFPQGLWTGVAMRSCPEEQYWDPLLGTCMSC KTICNHQSQRTCAAFCRSLSCRKEQGKFYDHLLRDCISC ASICGQHPKQCAYFCENKLRSPVNLPPEL |
| SEQ ID NO:21 | Fusion protein CRD2 | SLSCRKEQGKFYDHLLRDCISCASICGQHPKQCAYFCEN |
| SEQ ID NO:22 | Heavy chain of bispecific antibodies BI-9D2 and BI-9D2-A: 37F2C6-HuVH3-D2 | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN |
| | | GQPENNYKTTPPVLDSDGSFFLYSKLTVDKSRWQQGNVF SCSVMHEALHNHYTQKSLSLSPGKGGGGSGGGGSGGGG SSLSCRKEQGKFYDHLLRDCISCASICGQHPKQCAYFCE N |
| SEQ ID NO:23 | Heavy chain of bispecific antibody BI-9ND2-A: 37F2C6-HuVH3-3N-D2 | SLSCRKEQGKFYDHLLRDCISCASICGQHPKQCAYFCEN GGGGSGGGGSGGGGSQVQLVQSGAEVKKPGASVKVSC KASGYTFTDFAVHWVRQAPGQRLEWMGLIGTSSGSTNY NQKFQGRATMTVDKSANTAYMELSSLRSEDTAVYYCAR NGYYVGYYALDYWGQGTTVTVSSASTKGPSVFPLAPSS KSTSGGTAALGCLVKDYFPEPVTVSWNSGALTSGVHTFP AVLQSSGLYSLSSVVTVPSSSLGTQTYICNVNHKPSNTKV DKKVEPKSCDKTHTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFL YSKLTVDKSRWQQGNVFSCSVMHEALHNHYTQKSLSLS PGK |
| SEQ ID NO:24 | Heavy chain of bispecific antibody BI-9NTACI-A: 37F2C6-HuVH3-3N-TACI | SRVDQEERFPQGLWTGVAMRSCPEEQYWDPLLGTCMSC KTICNHQSQRTCAAFCRSLSCRKEQGKFYDHLLRDCISC ASICGQHPKQCAYFCENKLRSPVNLPPELGGGGSGGGGS GGGGSQVQLVQSGAEVKKPGASVKVSCKASGYTFTDFA VHWVRQAPGQRLEWMGLIGTSSGSTNYNQKFQGRATM TVDKSANTAYMELSSLRSEDTAVYYCARNGYYVGYYAL DYWGQGTTVTVSSASTKGPSVFPLAPSSKSTSGGTAALG CLVKDYFPEPVTVSWNSGALTSGVHTFPAVLQSSGLYSLS SVVTVPSSSLGTQTYICNVNHKPSNTKVDKKVEPKSCDK THTCPPCPAPELLGGPSVFLFPPKPKDTLMISRTPEVTCVV VDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPGK |
| SEQ ID NO:25 | Heavy chain of bispecific antibody BI-9ND2-2GS4: | SLSCRKEQGKFYDHLLRDCISCASICGQHPKQCAYFCEN GSSSSGSSSSQVQLVQSGAEVKKPGASVKVSCKASGYTF TDFAVHWVRQAPGQRLEWMGLIGTSSGSTNYNQKFQGR ATMTVDKSANTAYMELSSLRSEDTAVYYCARNGYYVGY YALDYWGQGTTVTVSSASTKGPSVFPLAPSSKSTSGGTA ALGCLVKDYFPEPVTVSWNSGALTSGVHTFPAVLQSSGL YSLSSVVTVPSSSLGTQTYICNVNHKPSNTKVDKKVEPK SCDKTHTCPPCPAPELLGGPSVFLFPPKPKDTLMISRTPEV TCVVVDVSHEDPEVKFNWYVDGVEVHNAKTKPREEQY NSTYRVVSVLTVLHQDWLNGKEYKCKVSNKALPAPIEK TISKAKGQPREPQVYTLPPSRDELTKNQVSLTCLVKGFYP SDIAVEWESNGQPENNYKTTPPVLDSDGSFFLYSKLTVD KSRWQQGNVFSCSVMHEALHNHYTQKSLSLSPGK |
| SEQ ID NO:26 | Heavy chain of bispecific antibody BI-9ND2-3GS4: | SLSCRKEQGKFYDHLLRDCISCASICGQHPKQCAYFCEN GSSSSGSSSSGSSSSQVQLVQSGAEVKKPGASVKVSCKA SGYTFTDFAVHWVRQAPGQRLEWMGLIGTSSGSTNYNQ |
| | | KFQGRATMTVDKSANTAYMELSSLRSEDTAVYYCARNG YYVGYYALDYWGQGTTVTVSSASTKGPSVFPLAPSSKST SGGTAALGCLVKDYFPEPVTVSWNSGALTSGVHTFPAVL QSSGLYSLSSVVTVPSSSLGTQTYICNVNHKPSNTKVDK KVEPKSCDKTHTCPPCPAPELLGGPSVFLFPPKPKDTLMI SRTPEVTCVVVDVSHEDPEVKFNWYVDGVEVHNAKTK PREEQYNSTYRVVSVLTVLHQDWLNGKEYKCKVSNKAL PAPIEKTISKAKGQPREPQVYTLPPSRDELTKNQVSLTCLV KGFYPSDIAVEWESNGQPENNYKTTPPVLDSDGSFFLYS KLTVDKSRWQQGNVFSCSVMHEALHNHYTQKSLSLSPG K |
| SEQ ID NO:27 | Heavy chain of bispecific antibody BI-9ND2-4GS4: | SLSCRKEQGKFYDHLLRDCISCASICGQHPKQCAYFCEN GSSSSGSSSSGSSSSGSSSSQVQLVQSGAEVKKPGASVKV SCKASGYTFTDFAVHWVRQAPGQRLEWMGLIGTSSGST NYNQKFQGRATMTVDKSANTAYMELSSLRSEDTAVYYC ARNGYYVGYYALDYWGQGTTVTVSSASTKGPSVFPLAP SSKSTSGGTAALGCLVKDYFPEPVTVSWNSGALTSGVHT FPAVLQSSGLYSLSSVVTVPSSSLGTQTYICNVNHKPSNT KVDKKVEPKSCDKTHTCPPCPAPELLGGPSVFLFPPKPK DTLMISRTPEVTCVVVDVSHEDPEVKFNWYVDGVEVHN AKTKPREEQYNSTYRVVSVLTVLHQDWLNGKEYKCKVS NKALPAPIEKTISKAKGQPREPQVYTLPPSRDELTKNQVS LTCLVKGFYPSDIAVEWESNGQPENNYKTTPPVLDSDGS FFLYSKLTVDKSRWQQGNVFSCSVMHEALHNHYTQKSL SLSPGK |
| SEQ ID NO:28 | Heavy chain of bispecific antibody BI-9D2-1GS4: | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGKGSSSSSLSCRKEQGK FYDHLLRDCISCASICGQHPKQCAYFCEN |
| SEQ ID NO:29 | Heavy chain of bispecific antibody BI-9D2-2GS4: | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGKGSSSSGSSSSSLSCRK |
| | | EQGKFYDHLLRDCISCASICGQHPKQCAYFCEN |
| SEQ ID NO:30 | Heavy chain of bispecific antibody BI-9D2-3GS4: | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGKGSSSSGSSSSGSSSSS LSCRKEQGKFYDHLLRDCISCASICGQHPKQCAYFCEN |
| SEQ ID NO:31 | Heavy chain of bispecific antibody BI-9D2-3G4S-4S: | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGKGGGGSGGGGSGGGG SSLSCRKEQGKFYDHLLRDCISCASICGQHPKQCAYFCE NSSSS |
| SEQ ID NO:32 | Heavy chain of bispecific antibody BI-9D2-3G4S-8S: | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSPGKGGGGSGGGGSGGGG SSLSCRKEQGKFYDHLLRDCISCASICGQHPKQCAYFCE NSSSSSSS |
| SEQ ID NO:33 | Antibody 26H10A11-1 HCDR1 (Kabat numbering) | SSTMS |
| SEQ ID NO:34 | Antibody 26H10A11-1 HCDR2 (Kabat numbering) | TTGGGGSYTYNPDSVKG |
| SEQ ID NO:35 | Antibody 26H10A11-1 HCDR3 (Kabat numbering) | DRYDGYYFSMDF |
| SEQ ID NO:36 | Antibody 26H10A11-1 LCDR1 (Kabat numbering) | KASQSVDYDGDSYMN |
| SEQ ID NO:37 | Antibody 26H10A11-1 LCDR2 (Kabat numbering) | AASNLES |
| SEQ ID NO:38 | Antibody 26H10A11-1 LCDR3 (Kabat numbering) | QQSNEDPRT |
| SEQ ID NO:39 | Antibody 37F2C6 HCDR1 (Kabat numbering) | DFAVHW |
| SEQ ID NO:40 | Antibody 37F2C6 HCDR2 (Kabat numbering) | LIGTSSGSTNYNQKFKD |
| SEQ ID NO:41 | Antibody 37F2C6 HCDR3 (Kabat numbering) | NGYYVGYYALDY |
| SEQ ID NO:42 | Antibody 37F2C6 LCDR1 (Kabat numbering) | SASSSVSYMY |
| SEQ ID NO:43 | Antibody 37F2C6 LCDR2 (Kabat numbering) | STSNLAS |
| SEQ ID NO:44 | Antibody 37F2C6 LCDR3 (Kabat numbering) | QQRRNYPHT |
| SEQ ID NO:45 | Amino acid sequence of human BDCA2 | HHHHHHNFMYSKTVKRLSKLREYQQYHPSLTCVMEGK DIEDWSCCPTPWTSFQSSCYFISTGMQSWTKSQKNCSVM GADLVVINTREEQDFIIQNLKRNSSYFLGLSDPGGRRHW QWVDQTPYNENVTFWHSGEPNNLDERCAIINFRSSEEW GWNDIHCHVPQKSICKMKKIYI |
| SEQ ID NO:46 | Amino acid sequence of rhesus monkey BDCA2 | HHHHHHNFMYSKTVKRLSKLQEYQQYYPSLTCVMEGK DMEDWSCCPTPWTSFQSSCYFISTVMQSWTKSQNNCSV MGAGLVVINTKEEQDFITQNLKINSAYFLGLSDPKGWRH WQWVDQTPYNKNVTFWHSGEPNSPDERCAIINFRSEEW GWNDVHCHVPQKSICKMKKIYI |
| SEQ ID NO:47 | Amino acid sequence of cynomolgus monkey BDCA2 | MVPEEEPQDREKGVWWFQLKVWSVAVVSILLLCVCFTV SSVASHNFMYSKTVKRLSKLQEYQQYYPSLTCVMEGKD MEDWSCCPTPWTSFQSSCYFISTVMQSWTKSQNNCSVM GADLVVINTKEEQDFITQNLKINSAYFLGLSDPKGWRHW QWVDQTPYNKNVTFWHSGEPNSPDERCAIINFRSEEWG WNDVHCHVPQKSICKMKKIYI |
| SEQ ID NO: 48 | Humanized antibodies Hu37F2C6-8, Hu37F2C6-8-A, Hu37F2C6-9, Hu37F2C6-9-A LCDR1 (Kabat numbering) | RASSSVSYMY |
| SEQ ID NO: 49 | Humanized antibodies Hu37F2C6-8, Hu37F2C6-8-A, Hu37F2C6-9, Hu37F2C6-9-A HCDR2 (Kabat numbering) | LIGTSSGSTNYNQKFQG |
| SEQ ID NO: 50 | Heavy chain of bispecific antibodies Bi-9CD2-DE-A and Bi-9CD2-DE 37F2C6-HuVH3-CD2-DE | QVQLVQSGAEVKKPGASVKVSCKASGYTFTDFAVHWVR QAPGQRLEWMGLIGTSSGSTNYNQKFQGRATMTVDKSA NTAYMELSSLRSEDTAVYYCARNGYYVGYYALDYWGQ GTTVTVSSASTKGPSVFPLAPSSKSTSGGTAALGCLVKDY FPEPVTVSWNSGALTSGVHTFPAVLQSSGLYSLSSVVTVP SSSLGTQTYICNVNHKPSNTKVDKKVEPKSCDKTHTCPP CPAPELLGGPSVFLFPPKPKDTLMISRTPEVTCVVVDVSH |
| | | EDPEVKFNWYVDGVEVHNAKTKPREEQYNSTYRVVSV LTVLHQDWLNGKEYKCKVSNKALPAPIEKTISKAKGQPR EPQVYTLPPSRDELTKNQVSLTCLVKGFYPSDIAVEWESN GQPENNYKTTPPVLDSDGSFFLYSKLTVDKSRWQQGNVF SCSVMHEALHNHYTQKSLSLSGGGGSGGGGSGGGGSCR KEQGKFYDHLLRDCISCASICGQHPKQCAYFCEN |
| SEQ ID NO: 51 | Heavy chain of bispecific antibodies Bi-9ND2-DE-A and Bi-9ND2-DE | SLSCRKEQGKFYDHLLRDCISCASICGQHPKQCAYFCGG GGSGGGGSGGGGSQVQLVQSGAEVKKPGASVKVSCKA SGYTFTDFAVHWVRQAPGQRLEWMGLIGTSSGSTNYNQ KFQGRATMTVDKSANTAYMELSSLRSEDTAVYYCARNG YYVGYYALDYWGQGTTVTVSSASTKGPSVFPLAPSSKST SGGTAALGCLVKDYFPEPVTVSWNSGALTSGVHTFPAVL QSSGLYSLSSVVTVPSSSLGTQTYICNVNHKPSNTKVDK KVEPKSCDKTHTCPPCPAPELLGGPSVFLFPPKPKDTLMI SRTPEVTCVVVDVSHEDPEVKFNWYVDGVEVHNAKTK PREEQYNSTYRVVSVLTVLHQDWLNGKEYKCKVSNKAL PAPIEKTISKAKGQPREPQVYTLPPSRDELTKNQVSLTCLV KGFYPSDIAVEWESNGQPENNYKTTPPVLDSDGSFFLYS KLTVDKSRWQQGNVFSCSVMHEALHNHYTQKSLSLSPG K |
| | 37F2C6-HuVH3-ND2-DE | |
| SEQ ID NO: 52 | Light chain of bispecific antibodies Bi-9CD2-DE-A and Bi-9ND2-DE- | ETVLTQSPATLSLSPGERATLSCRASSSVSYMYWFQQKPG QAPRLLIYSTSNLASGIPARFSGSGSGTDYTLTISSLEPEDF AVYYCQQRRNYPHTFGGGTKVEIKRTVAAPSVFIFPPSDE QLKSGTASVVCLLNNFYPREAKVQWKVDNALQSGNSQ ESVTEQDSKDSTYSLSSTLTLSKADYEKHKVYACEVTHQ GLSSPVTKSFNRGEC |
| SEQ ID NO: 53 | Heavy chain of bispecific antibody BI-9ND2-A-YTE: 37F2C6-HuVH3-ND2-YTE | SLSCRKEQGKFYDHLLRDCISCASICGQHPKQCAYFCEN GGGGSGGGGSGGGGSQVQLVQSGAEVKKPGASVKVSC KASGYTFTDFAVHWVRQAPGQRLEWMGLIGTSSGSTNY NQKFQGRATMTVDKSANTAYMELSSLRSEDTAVYYCAR NGYYVGYYALDYWGQGTTVTVSSASTKGPSVFPLAPSS KSTSGGTAALGCLVKDYFPEPVTVSWNSGALTSGVHTFP AVLQSSGLYSLSSVVTVPSSSLGTQTYICNVNHKPSNTKV DKKVEPKSCDKTHTCPPCPAPELLGGPSVFLFPPKPKDTL YITREPEVTCVVVDVSHEDPEVKFNWYVDGVEVHNAKT KPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFL YSKLTVDKSRWQQGNVFSCSVMHEALHNHYTQKSLSLS PGK |
| SEQ ID NO: 54 | Heavy chain constant region of human IgG1 carrying YTE mutation combination | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLG GPSVFLFPPKPKDTLYITREPEVTCVVVDVSHEDPEVKFN WYVDGVEVHNAKTKPREEQYNSTYRVVSVLTVLHQDW LNGKEYKCKVSNKALPAPIEKTISKAKGQPREPQVYTLP PSRDELTKNQVSLTCLVKGFYPSDIAVEWESNGQPENNY KTTPPVLDSDGSFFLYSKLTVDKSRWQQGNVFSCSVMHE ALHNHYTQKSLSLSPGK |

## Claims

1. An anti-BDCA2 antibody or an antigen-binding fragment thereof, wherein the anti-BDCA2 antibody or the antigen-binding fragment thereof comprises a heavy chain variable region VH and/or a light chain variable region VL, wherein the heavy chain variable region VH comprises 3 CDRs: HCDR1, HCDR2, and HCDR3, and the light chain variable region VL comprises 3 CDRs: LCDR1, LCDR2, and LCDR3, wherein
(i) the HCDR1, the HCDR2, and the HCDR3 are three complementarity determining regions HCDR1, HCDR2, and HCDR3 of the VH set forth in SEQ ID NO: 1; and the LCDR1, the LCDR2, and the LCDR3 are three complementarity determining regions LCDR1, LCDR2, and LCDR3 of the VL set forth in SEQ ID NO: 2;
(ii) the HCDR1, the HCDR2, and the HCDR3 are three complementarity determining regions HCDR1, HCDR2, and HCDR3 of the VH set forth in SEQ ID NO: 3; and the LCDR1, the LCDR2, and the LCDR3 are three complementarity determining regions LCDR1, LCDR2, and LCDR3 of the VL set forth in SEQ ID NO: 4; or
(iii) the HCDR1, the HCDR2, and the HCDR3 are three complementarity determining regions HCDR1, HCDR2, and HCDR3 of the VH set forth in SEQ ID NO: 13; and the LCDR1, the LCDR2, and the LCDR3 are three complementarity determining regions LCDR1, LCDR2, and LCDR3 of the VL set forth in SEQ ID NO: 15; or
(iv) the HCDR1, the HCDR2, and the HCDR3 are three complementarity determining regions HCDR1, HCDR2, and HCDR3 of the VH set forth in SEQ ID NO: 14; and the LCDR1, the LCDR2, and the LCDR3 are three complementarity determining regions LCDR1, LCDR2, and LCDR3 of the VL set forth in SEQ ID NO: 15; or
(v) relative to the sequence in any one of (i)-(iv), a sequence comprises at least one and no more than 5, 4, 3, 2, or 1 amino acid modification (preferably amino acid substitution, and preferably conservative substitution) in the three HCDRs or the three LCDRs; for example, the amino acid modification is a mutation enhancing the stability of the antibody;
preferably, the CDRs are determined by the Kabat numbering scheme.

2. The anti-BDCA2 antibody or the antigen-binding fragment thereof according to claim 1, comprising
(i) HCDR1 set forth in SEQ ID NO: 33, HCDR2 set forth in SEQ ID NO: 34, HCDR3 set forth in SEQ ID NO: 35, LCDR1 set forth in SEQ ID NO: 36, LCDR2 set forth in SEQ ID NO: 37, and LCDR3 set forth in SEQ ID NO: 38; or
(ii) HCDR1 set forth in SEQ ID NO: 39, HCDR2 set forth in SEQ ID NO: 40 or 49, HCDR3 set forth in SEQ ID NO: 41, LCDR1 set forth in SEQ ID NO: 42 or 48, LCDR2 set forth in SEQ ID NO: 43, and LCDR3 set forth in SEQ ID NO: 44; or
(iii) relative to the sequence in any one of (i) or (ii), a sequence comprising at least one and no more than 5, 4, 3, 2, or 1 amino acid modification (preferably amino acid substitution, and preferably conservative substitution) in the three HCDRs or the three LCDRs; for example, the amino acid modification is a mutation enhancing the stability of the antibody.

3. The anti-BDCA2 antibody or the antigen-binding fragment thereof according to claim 1 or 2, wherein the heavy chain variable region VH
(i) comprises or consists of an amino acid sequence having at least 90%, 91%, 92%, 93%, 94%, 95%, 96%, 97%, 98%, or 99% identity to the amino acid sequence of SEQ ID NO: 1, 3, 13, or 14; or
(ii) comprises or consists of the amino acid sequence of SEQ ID NO: 1, 3, 13, or 14.

4. The anti-BDCA2 antibody or the antigen-binding fragment thereof according to any one of claims 1-3, wherein the light chain variable region VL
(i) comprises or consists of an amino acid sequence having at least 90%, 91%, 92%, 93%, 94%, 95%, 96%, 97%, 98%, or 99% identity to the amino acid sequence of SEQ ID NO: 2, 4, or 15; or
(ii) comprises or consists of the amino acid sequence of SEQ ID NO: 2, 4, or 15.

5. The anti-BDCA2 antibody or the antigen-binding fragment thereof according to any one of claims 1-4, comprising a heavy chain variable region VH and a light chain variable region VL, wherein
(i) the heavy chain variable region comprises or consists of the amino acid sequence set forth in SEQ ID NO: 1 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 2 or an amino acid sequence having at least 90%, 91%, 92%, 93%, 94%, 95%, 96%, 97%, 98%, or 99% identity to the amino acid sequence; or
(ii) the heavy chain variable region comprises or consists of the amino acid sequence set forth in SEQ ID NO: 3 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 4 or an amino acid sequence having at least 90%, 91%, 92%, 93%, 94%, 95%, 96%, 97%, 98%, or 99% identity to the amino acid sequence; or
(iii) the heavy chain variable region comprises or consists of the amino acid sequence set forth in SEQ ID NO: 13 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15 or an amino acid sequence having at least 90%, 91%, 92%, 93%, 94%, 95%, 96%, 97%, 98%, or 99% identity to the amino acid sequence; or
(iv) the heavy chain variable region comprises or consists of the amino acid sequence set forth in SEQ ID NO: 14 or an amino acid sequence having at least 90%, 91%, 92%, 93%, 94%, 95%, 96%, 97%, 98%, or 99% identity to the amino acid sequence; and the light chain variable region comprises or consists of the amino acid sequence set forth in SEQ ID NO: 15 or an amino acid sequence having at least 90%, 91%, 92%, 93%, 94%, 95%, 96%, 97%, 98%, or 99% identity to the amino acid sequence.

6. The anti-BDCA2 antibody or the antigen-binding fragment thereof according to any one of claims 1-5, wherein the VH and the VL respectively comprise or consist of the amino acid sequences set forth in:
(i). SEQ ID NO: 1 and SEQ ID NO: 2;
(ii). SEQ ID NO: 3 and SEQ ID NO: 4;
(iii).SEQ ID NO: 13 and SEQ ID NO: 15;
(iv). SEQ ID NO: 14 and SEQ ID NO: 15.

7. The anti-BDCA2 antibody or the antigen-binding fragment thereof according to any one of claims 1-6, further comprising Fc or a heavy chain constant region CH, wherein, for example,
the Fc region is an Fc region of IgG1, IgG2, IgG3, or IgG4, preferably an Fc region of IgG1; or
the antibody heavy chain constant region CH is a heavy chain constant region of IgG1, IgG2, IgG3, or IgG4, preferably a heavy chain constant region of IgG1;
optionally, a sequence of the heavy chain constant region has one or more amino acid substitutions compared to a natural human heavy chain constant region sequence; preferably, the one or more amino acid substitutions result in an enhanced ADCC and/or CDC effect and/or prolonged *in vivo* half-life and/or increased affinity for FcRn of the antibody;
more preferably, the one or more amino acid substitutions are an amino acid substitution combination of M252Y/S254T/T256E according to the EU numbering, for example, the heavy chain constant region comprises or consists of the amino acid sequence of SEQ ID NO: 54.

8. The anti-BDCA2 antibody or the antigen-binding fragment thereof according to claim 7, wherein the heavy chain constant region CH
(i) comprises or consists of an amino acid sequence having at least 85%, 90%, 91%, 92%, 93%, 94%, 95%, 96%, 97%, 98%, or 99% identity to the amino acid sequence of SEQ ID NO: 5; or
(ii) comprises or consists of the amino acid sequence of SEQ ID NO: 5.

9. The anti-BDCA2 antibody or the antigen-binding fragment thereof according to any one of claims 1-8, further comprising a light chain constant region, wherein, for example, the light chain constant region is a lambda or kappa light chain constant region.

10. The anti-BDCA2 antibody or the antigen-binding fragment thereof according to claim 9, wherein the light chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 6; or
(ii) comprises or consists of the amino acid sequence of SEQ ID NO: 6.

11. The anti-BDCA2 antibody or the antigen-binding fragment thereof according to any one of claims 1-10, comprising a heavy chain, wherein the heavy chain comprises or consists of the amino acid sequence of SEQ ID NO: 9, 11, 16, or 17, or an amino acid sequence having at least 85%, 90%, 91%, 92%, 93%, 94%, 95%, 96%, 97%, 98%, or 99% identity to the amino acid sequence.

12. The anti-BDCA2 antibody or the antigen-binding fragment thereof according to any one of claims 1-11, comprising a light chain, wherein the light chain comprises or consists of the amino acid sequence of SEQ ID NO: 10, 12, or 18, or an amino acid sequence having at least 85%, 90%, 91%, 92%, 93%, 94%, 95%, 96%, 97%, 98%, or 99% identity to the amino acid sequence.

13. The anti-BDCA2 antibody or the antigen-binding fragment thereof according to claim 11 or 12, comprising a heavy chain and a light chain, wherein
(i) the heavy chain comprises or consists of the amino acid sequence of SEQ ID NO: 9 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 10 or an amino acid sequence having at least 85%, 90%, 91%, 92%, 93%, 94%, 95%, 96%, 97%, 98%, or 99% identity to the amino acid sequence;
(ii) the heavy chain comprises or consists of the amino acid sequence of SEQ ID NO: 11 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 12 or an amino acid sequence having at least 85%, 90%, 91%, 92%, 93%, 94%, 95%, 96%, 97%, 98%, or 99% identity to the amino acid sequence;
(iii) the heavy chain comprises or consists of the amino acid sequence of SEQ ID NO: 16 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 18 or an amino acid sequence having at least 85%, 90%, 91%, 92%, 93%, 94%, 95%, 96%, 97%, 98%, or 99% identity to the amino acid sequence; or
(iv) the heavy chain comprises or consists of the amino acid sequence of SEQ ID NO: 17 or an amino acid sequence having at least 85%, 90%, 91%, 92%, 93%, 94%, 95%, 96%, 97%, 98%, or 99% identity to the amino acid sequence; and the light chain comprises or consists of the amino acid sequence of SEQ ID NO: 18 or an amino acid sequence having at least 85%, 90%, 91%, 92%, 93%, 94%, 95%, 96%, 97%, 98%, or 99% identity to the amino acid sequence.

14. The anti-BDCA2 antibody or the antigen-binding fragment thereof according to claim 13, wherein the heavy chain and the light chain respectively comprise or consist of amino acid sequences set forth in the following SEQ ID NOs:
(i). SEQ ID NO: 9 and SEQ ID NO: 10;
(ii). SEQ ID NO: 11 and SEQ ID NO: 12;
(iii).SEQ ID NO: 16 and SEQ ID NO: 18;
(iv). SEQ ID NO: 17 and SEQ ID NO: 18.

15. The anti-BDCA2 antibody or the antigen-binding fragment thereof according to any one of claims 1-14, wherein the antibody or the antigen-binding fragment thereof features enhanced ADCC and/or prolonged half-life, for example, having an altered glycosylation type, e.g., being low-fucosylated or defucosylated or comprising an increased proportion of a bisecting GlcNac structure, and/or e.g., carrying an amino acid substitution combination of M252Y/S254T/T256E.

16. The anti-BDCA2 antibody or the antigen-binding fragment thereof according to any one of claims 1-14, wherein the anti-BDCA2 antibody or the antigen-binding fragment thereof is a humanized antibody or a chimeric antibody.

17. The anti-BDCA2 antibody or the antigen-binding fragment thereof according to any one of claims 1-16, wherein the anti-BDCA2 antibody or the antigen-binding fragment thereof is a monoclonal antibody.

18. The anti-BDCA2 antibody or the antigen-binding fragment thereof according to any one of claims 1-17, wherein the antigen-binding fragment is selected from: a Fab, a Fab', a Fab'-SH, an Fv, a single-chain antibody (e.g., scFv), an (Fab')₂, a single-domain antibody such as VHH, a domain antibody (dAb), and a linear antibody.

19. A fusion protein molecule, wherein the fusion protein molecule specifically binds to human BDCA2 and human BAFF and/or APRIL.

20. The fusion protein molecule according to claim 19, comprising a first target-binding region and a second target-binding region, wherein the first target-binding region specifically binds to BDCA2, and the second target-binding region specifically binds to BAFF and/or APRIL.

21. The fusion protein molecule according to claim 20, wherein the first target-binding region and the second target-binding region are linked via a linker, or are directly linked without a linker.

22. The fusion protein molecule according to claim 20 or 21, wherein the first target-binding region is an anti-BDCA2 antibody or an antigen-binding fragment thereof or is derived from the anti-BDCA2 antibody or the antigen-binding fragment thereof; preferably, (i) the anti-BDCA2 antibody or the antigen-binding fragment thereof is the anti-BDCA2 antibody or the antigen-binding fragment thereof according to claims 1-18; or (ii) the anti-BDCA2 antibody or the antigen-binding fragment thereof exhibits exactly the same binding specificity as the anti-BDCA2 antibody or the antigen-binding fragment thereof according to claims 1-18.

23. The fusion protein molecule according to any one of claims 19-22, wherein the second target-binding region comprises a BAFF/APRIL receptor or a functional variant or fragment thereof specifically binding to BAFF and APRIL.

24. The fusion protein molecule according to claim 23, wherein the BAFF/APRIL receptor is TACI, for example, a natural TACI molecule or a functional variant thereof, or a functional fragment/truncation of a natural TACI molecule or a functional variant thereof, for example, the functional fragment is an extracellular region, CRD1-2, CDR2, or CRD2 truncation of a TACI molecule, or a fragment consisting of amino acids at positions 30-118 of a TACI molecule; preferably, the TACI
(i) comprises or consists of an amino acid sequence having at least 85%, 90%, 91%, 92%, 93%, 94%, 95%, 96%, 97%, 98%, or 99% identity to the amino acid sequence of SEQ ID NO: 20;
(ii) comprises or consists of the amino acid sequence of SEQ ID NO: 20; or
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 20.

25. The fusion protein molecule according to claim 23 or 24, wherein the functional fragment comprises a CRD2 or is a CRD2.

26. The fusion protein molecule according to claim 25, wherein the CRD2 is a CRD2 of wild-type TACI, for example, a CRD2 of a wild-type TACI molecule, or a variant CRD2 comprising a modification relative to the wild-type CRD2.

27. The fusion protein molecule according to claim 25 or 26, wherein the CRD2 is a CRD2 of a TACI molecule, for example, the CRD2 of the TACI molecule
(i) comprises or consists of an amino acid sequence having at least 85%, 90%, 91%, 92%, 93%, 94%, 95%, 96%, 97%, 98%, or 99% identity to the amino acid sequence of SEQ ID NO: 21;
(ii) comprises or consists of the amino acid sequence of SEQ ID NO: 21;
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared to the amino acid sequence of SEQ ID NO: 21.

28. The fusion protein molecule according to any one of claims 19-27, comprising
(i) a chain A: a light chain of an anti-BDCA2 antibody;
(ii) a chain B: a heavy chain of an anti-BDCA2 antibody linked to a TACI at the N-terminus or C-terminus, with or without a linker.

29. The fusion protein molecule according to claim 28, wherein the chain B comprises, from N-terminus to C-terminus, the following elements in sequence:
(iii) an anti-BDCA2 heavy chain, a linker, and TACI; or
(iv) TACI, a linker, and an anti-BDCA2 heavy chain; or
(vi) an anti-BDCA2 heavy chain, a linker, TACI, and a polyserine residue sequence; or
(viii) TACI, a polyserine residue sequence, a linker, and an anti-BDCA2 heavy chain,
wherein TACI is a full-length TACI molecule or a fragment thereof;
wherein the polyserine residue sequence is SSSS, SSSSS, SSSSSS, SSSSSSS, SSSSSSSS, SSSSSSSSS, or SSSSSSSSSS,
and optionally, the linker may be absent.

30. The fusion protein molecule according to claim 29, wherein TACI is a CRD2 domain.

31. The fusion protein molecule according to any one of claims 28-30, wherein the linker may be any universal flexible sequence, such as a short flexible amino acid sequence; preferably, the linker comprises 10-30 amino acids in length; more preferably, the linker is (GGGGS)n, (GSSSS)n, (GGGSG)n, (GGSGG)n, (GSGGG)n, (SGGGG)n, (GGTGS)n, (GTSPGG)n, (GNGGGS)n, (GGG)n, (DGGGS)n, (TGEKP)n, (GGRR)n, (EGKSSGSGSESKVD)n, (KESGSVSSEQLAQFRSLD)n, (GGRRGGGS)n, (LRQRDGERP)n, (LRQKDGGGSERP)n, (GSTSGSGKPGSGEGSTKG)n, and/or G₄S-GGSGG-G₄S-SGGGG, etc., wherein n is an integer equal to or greater than 1, for example, n is an integer of 1, 2, 3, 4, 5, 6, 7, 8, or 9; more preferably, the linker is (G₄S)n or (GS₄)n, wherein n is an integer equal to or greater than 1, for example, n is an integer of 1, 2, 3, 4, 5, 6, 7, 8, or 9; most preferably, the linker is selected from GS₄, G₄S, (GS₄)₂, (G₄S)₂, (GS₄)₃, (G₄S)₃, (GS₄)₄, and (G₄S)₄.

32. The fusion protein molecule according to any one of claims 28-31, wherein the fusion protein molecule consists of 2 chains A and 2 chains B; preferably, the fusion protein molecule consists of 2 identical chains A and 2 identical chains B.

33. The fusion protein molecule according to any one of claims 28-32, wherein the chain A comprises or consists of the amino acid sequence set forth in SEQ ID NO: 18 or 52 or an amino acid sequence having at least 85%, 90%, 91%, 92%, 93%, 94%, 95%, 96%, 97%, 98%, or 99% identity to the amino acid sequence.

34. The fusion protein molecule according to any one of claims 28-33, wherein the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence.

35. The fusion protein molecule according to any one of claims 28-34, wherein
the chain A comprises or consists of the amino acid sequence set forth in SEQ ID NO: 18 or 52 or an amino acid sequence having at least 85%, 90%, 91%, 92%, 93%, 94%, 95%, 96%, 97%, 98%, or 99% identity to the amino acid sequence; and the chain B comprises or consists of the amino acid sequence set forth in any one of SEQ ID NOs: 22-32, 50-51, and 53 or an amino acid sequence having at least 85%, 90%, 91%, 92%, 93%, 94%, 95%, 96%, 97%, 98%, or 99% identity to the amino acid sequence; preferably, the chain A comprises or consists of the amino acid sequence set forth in SEQ ID NO: 18 or 52, and the chain B comprises or consists of the amino acid sequence set forth in any one of SEQ ID NOs: 22-32, 50-51, and 53.

36. The fusion protein molecule according to any one of claims 19-35, comprising an antibody heavy chain constant region featuring enhanced ADCC and/or prolonged half-life and/or increased affinity for FcRn, e.g., having an altered glycosylation type, e.g., being low-fucosylated or defucosylated or comprising an increased proportion of a bisecting GlcNac structure, and/or e.g., carrying an amino acid substitution combination of M252Y/S254T/T256E according to the EU numbering; for example, the heavy chain constant region comprises or consists of the amino acid sequence of SEQ ID NO: 54.

37. A nucleic acid molecule, comprising or consisting of a nucleic acid sequence encoding the anti-BDCA2 antibody or the antigen-binding fragment thereof according to any one of claims 1-18 or any one of the chains of the fusion protein molecule according to any one of claims 19-36.

38. An expression vector, comprising the nucleic acid molecule according to claim 37, wherein preferably, the expression vector is pCDNA, e.g., pCDNA3.1.

39. A host cell, comprising the nucleic acid molecule according to claim 37 or the expression vector according to claim 38, wherein preferably, the host cell is prokaryotic or eukaryotic, e.g., a 293 cell or a CHO cell, such as an HEK293 cell, a 293T cell, or a CHO-S cell.

40. A method for preparing the anti-BDCA2 antibody or the antigen-binding fragment thereof according to any one of claims 1-18 or the fusion protein molecule according to any one of claims 19-36, comprising culturing a host cell comprising the nucleic acid molecule according to claim 37 or the expression vector according to claim 38 under a condition suitable for expressing the chains of the fusion protein, and optionally recovering the antibody from the host cell (or a host cell medium).

41. A pharmaceutical composition, a medicament, or a formulation, comprising the anti-BDCA2 antibody or the antigen-binding fragment thereof according to any one of claims 1-18 or the fusion protein molecule according to any one of claims 19-36, and optionally a pharmaceutical supplementary material.

42. A pharmaceutical combination product, comprising the anti-BDCA2 antibody or the antigen-binding fragment thereof according to any one of claims 1-18 or the fusion protein molecule according to any one of claims 19-36, and one or more additional therapeutic agents (e.g., an antiinflammatory drug, an additional antibody, or a small molecule drug).

43. A method for preventing or treating a disease in a subject, comprising administering to the subject an effective amount of the anti-BDCA2 antibody or the antigen-binding fragment thereof according to any one of claims 1-18, the fusion protein molecule according to any one of claims 19-36, the pharmaceutical composition or formulation according to claim 41, or the pharmaceutical combination product according to claim 42.

44. The method according to claim 43, wherein the disease is an inflammatory disorder/disease and/or an autoimmune disorder/disease, for example, a disease/disorder selected from systemic lupus erythematosus (SLE) (e.g., moderate or severe lupus), cutaneous lupus, discoid lupus, lupus nephritis, systemic sclerosis (scleroderma), morphea, psoriasis, rheumatoid arthritis, inflammatory bowel disease (IBD), dermatomyositis, polymyositis, psoriasis, type I diabetes, asthma, Behcet's disease, CREST syndrome, Crohn's disease, dermatomyositis, juvenile dermatomyositis, diabetes, discoid lupus erythematosus, pulmonary fibrosis, autoimmune glomerulonephritis, membranous glomerulopathy, juvenile rheumatoid arthritis (juvenile chronic arthritis), mixed connective tissue disease, multiple sclerosis, nephrotic syndrome, panniculitis, pemphigoid, pemphigus, pemphigus erythematosus, pemphigus foliaceus, pemphigus vulgaris, polymyalgia rheumatica, systemic sclerosis, progressive systemic sclerosis (scleroderma), morphea (localized scleroderma), multiple sclerosis, psoriasis, psoriatic arthritis, pulmonary fibrosis, Raynaud's phenomenon/syndrome, Sjögren's syndrome, ulcerative colitis, neuromyelitis optica, neuromyelitis optica spectrum disorder, primary Sjögren's syndrome, myasthenia gravis, and any combination thereof.

45. The method according to claim 43, wherein the disease is a tumor, e.g., a hematopoietic tumor.

46. A method for inhibiting overactivation of plasmacytoid dendritic cells and/or eliminating plasmacytoid dendritic cells from a subject, comprising administering to the subject an effective amount of the anti-BDCA2 antibody or the antigen-binding fragment thereof according to any one of claims 1-18, the fusion protein molecule according to any one of claims 19-36, the pharmaceutical composition or formulation according to claim 41, or the pharmaceutical combination product according to claim 42.

47. A method for inhibiting release of IFNα in a subject, comprising administering to the subject an effective amount of the anti-BDCA2 antibody or the antigen-binding fragment thereof according to any one of claims 1-18, the fusion protein molecule according to any one of claims 19-36, the pharmaceutical composition or formulation according to claim 41, or the pharmaceutical combination product according to claim 42.

48. A method for inhibiting B cell proliferation in a subject, comprising administering to the subject an effective amount of the anti-BDCA2 antibody or the antigen-binding fragment thereof according to any one of claims 1-18, the fusion protein molecule according to any one of claims 19-36, the pharmaceutical composition or formulation according to claim 41, or the pharmaceutical combination product according to claim 42.

49. The method according to any one of claims 43-44 and 46-48, wherein the subject suffers from inflammation, and BDCA2 expression or elevated BDCA2 expression (e.g., compared to a tissue/organ of a healthy subject or compared to a healthy tissue/organ of the same patient) is present in a tissue or organ affected by inflammation of the subject.

50. The method according to claim 49, wherein a BDCA2 nucleic acid or an elevated BDCA2 nucleic acid level (e.g., compared to a tissue/organ of a healthy subject or compared to a healthy tissue/organ of the same patient) is present in a tissue or organ affected by inflammation of the subject.

51. A method for detecting the presence of BDCA2 in a biological sample, comprising
(i) contacting a biological sample with the antibody or the antigen-binding fragment thereof according to any one of claims 1-18 or the multispecific fusion protein according to any one of claims 19-35 under conditions that allow the antibody or the antigen-binding fragment thereof or the multispecific fusion protein to bind to BDCA2,
(ii) detecting whether a complex is formed by the antibody or the multispecific fusion protein and BDCA2,
wherein the formation of the complex indicates the presence of BDCA2.

52. A method for detecting the presence of BAFF and/or APRIL in a biological sample, comprising
(i) contacting a biological sample with the multispecific fusion protein according to any one of claims 19-36 under conditions that allow the multispecific fusion protein to bind to BAFF and/or APRIL,
(ii) detecting whether a complex is formed by the multispecific fusion protein and BAFF and/or APRIL,
wherein the formation of the complex indicates the presence of BAFF and/or APRIL.
